# EUROPEAN PATENT SPECIFICATION

(11) **EP 0 915 869 B1**
(45) Date of publication and mention of the grant of the patent: **14.05.2003**
(21) Application number: 97934269.8
(22) Date of filing: 29.07.1997
(51) Int. Cl.: C07D 401/04, C07D 401/14, C07D 405/14, C07D 405/12, C07D 401/12, A61K 31/445, A61K 31/435

(54) **NOVEL TRICYCLIC N-CYANOIMINES USEFUL AS INHIBITORS OF FARNESYL-PROTEIN TRANSFERASE**
TRICYCLISCHE N-CYANOIMINE ALS INHIBITOREN DER FARNESYL-PROTEIN TRANSFERASE
NOUVELLES N-CYANO-IMINES TRICYCLIQUES UTILES EN TANT QU'INHIBITEURS DE LA FARNESYLE TRANSFERASE

(30) Priority: 31.07.1996 US 690522
(43) Date of publication of application: 19.05.1999
(73) Proprietor: SCHERING CORPORATION, Kenilworth New Jersey 07033 (US)
(72) Inventor: COOPER, Alan, B., West Caldwell, NJ 07006 (US); WANG, James, J.-S., Westfield, NJ 07090 (US); LOVEY, Raymond, G., West Caldwell, NJ 07006 (US); DESAI, Jagdish, A., Spotswood, NJ 08884 (US); SAKSENA, Anil, K., Upper Montclair, NJ 07043 (US); GIRIJAVALLABHAN, Viyyoor, M., Parsippany, NJ 07054 (US); DOLL, Ronald, J., Maplewood, NJ 07040 (US)
(74) Representative: Ritter, Stephen David
(86) International application number: US9712923
(87) International publication number: WO98004545

(56) References cited:
- EP-A- 0 270 818
- EP-A- 0 341 860
- WO-A-95/10515
- WO-A-95/10516

## Description

### BACKGROUND

Patent application WO 95/00497 published 5 January 1995 under the Patent Cooperation Treaty (PCT) describes compounds which inhibit the enzyme, famesyl-protein transferase (FTase) and the famesylation of the oncogene protein Ras. Oncogenes frequently encode protein components of signal transduction pathways which lead to stimulation of cell growth and mitogenesis. Oncogene expression in cultured cells leads to cellular transformation, characterized by the ability of cells to grow in soft agar and the growth of cells as dense foci lacking the contact inhibition exhibited by non-transformed cells. Mutation and/or overexpression of certain oncogenes is frequently associated with human cancer.

To acquire transforming potential, the precursor of the Ras oncoprotein must undergo famesylation of the cysteine residue located in a carboxyl-terminal tetrapeptide. Inhibitors of the enzyme that catalyzes this modification, farnesyl protein transferase, have therefore been suggested as anticancer agents for tumors in which Ras contributes to transformation. Mutated, oncogenic forms of Ras are frequently found in many human cancers, most notably in more than 50% of colon and pancreatic carcinomas (Kohl et al., Science, Vol. 260, 1834 to 1837, 1993).

In view of the current interest in inhibitors of farnesyl protein transferase, a welcome contribution to the art would be additional compounds useful for the inhibition of famesyl protein transferase. Such a contribution is provided by this invention.

WO 95/10516 discloses tricyclic amide and urea compounds useful for the inhibition of G-protein function and for treatment of proliferative diseases. WO 95/10515 disclose tricyclic carbamate compounds useful for inhibition of G-protein function and for treatment of proliferative diseases.

In view of the current interest in inhibitors of farnesyl protein transferase, a welcome contribution to the art would be additional compounds useful for the inhibition of farnesyl protein transferase. Such a contribution is provided by this invention.

### SUMMARY OF THE INVENTION

Inhibition of famesyl protein transferase by tricyclic compounds of this invention has not been reported previously. Thus, this invention provides a method for inhibiting famesyl protein transferase using tricyclic compounds of this invention which: (i) potently inhibit famesyl protein transferase, but not geranylgeranyl protein transferase I, in vitro; (ii) block the phenotypic change induced by a form of transforming Ras which is a tarnesyl acceptor but not by a form of transforming Ras engineered to be a geranylgeranyl acceptor; (iii) block intracellular processing of Ras which is a famesyl acceptor but not of Ras engineered to be a geranylgeranyl acceptor; and (iv) block abnormal cell growth in culture induced by transforming Ras. Several compounds of this invention have been demonstrated to have anti-tumor activity in animal models.

This invention provides a method for inhibiting the abnormal growth of cells, including transformed cells, by administering an effective amount of a compound of this invention. Abnormal growth of cells refers to cell growth independent of normal regulatory mechanisms (e.g., loss of contact inhibition). This includes the abnormal growth of: (1) tumor cells (tumors) expressing an activated Ras oncogene; (2) tumor cells in which the Ras protein is activated as a result of oncogenic mutation in another gene; and (3) benign and malignant cells of other proliferative diseases in which aberrant Ras activation occurs.

Compounds useful in the claimed methods are represented by Formula 1.0: 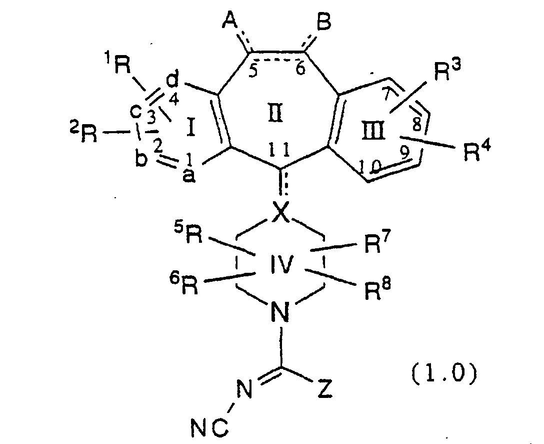 or a pharmaceutically acceptable salt or solvate thereof, wherein:
one of a, b, c and d represents N or NR⁹ wherein R⁹ is O⁻, -CH₃ or -(CH₂)ₙCO₂H wherein n is 1 to 3, and the remaining a, b, c and d groups represent CR¹ or CR²; or
each of a, b, c, and d are independently selected from CR¹ or CR²; each R¹ and each R² is independently selected from H, halo, -CF₃, -OR¹⁰ (e.g., -OCH₃), -COR¹⁰, -SR¹⁰ (e.g., -SCH₃ and -SCH₂C₆H₅), -S(O)ₜR¹¹ (wherein t is 0, 1 or 2, e.g., -SOCH₃ and -SO₂CH₃), -SON, -N(R¹⁰)₂, -NR¹⁰R¹¹, -NO₂, -OC(O)R¹⁰, -CO₂R¹⁰, -OCO₂R¹¹, -CN, -NHC(O)R¹⁰, -NHSO₂R¹⁰, -CONHR¹⁰, -CONHCH₂CH₂OH, -NR¹⁰COOR¹¹, -SR¹¹C(O)OR¹¹ (e.g., -SCH₂CO₂CH₃), -SR¹¹N(R⁷⁵)₂ wherein each R⁷⁵ is independently selected from H and -C(O)OR¹¹ (e.g., -S(CH₂)₂NHC(O)O-t-butyl and -S(CH₂)₂NH₂), benzotriazol-1-yloxy, tetrazol-5-ylthio, or substituted tetrazol-5-ylthio (e.g., alkyl substituted tetrazol5-ylthio such as 1-methyl-tetrazol-5-ylthio), alkynyl, alkenyl or alkyl, said alkyl or alkenyl group optionally being substituted with halo, -OR¹⁰ or -CO₂R¹⁰;
R³ and R⁴ are the same or different and each independently represents H, any of the substituents of R¹ and R², or R³ and R⁴ taken together represent a saturated or unsaturated C₅-C₇ fused ring to the benzene ring (Ring III);
R⁵, R⁶, R⁷ and R⁸ each independently represents H, -CF₃, -COR¹⁰, alkyl or aryl, said alkyl or aryl optionally being substituted with -OR¹⁰, -SR¹⁰, -S(O)ₜR¹¹, -NR¹⁰COOR¹¹, -N(R¹⁰)₂, -NO₂, -COR¹⁰, -OCOR¹⁰, -OCO₂R¹¹, -CO₂R¹⁰, OPO₃R¹⁰ or one of R⁵, R⁶, R⁷ and R⁸ can be taken in combination with R⁴⁰ as defined below to represent -(CH₂)ᵣ- wherein r is 1 to 4 which can be substituted with lower alkyl of one to six carbon atoms, lower alkoxy of one to six carbon atoms, -CF₃ or aryl, or R⁵ is combined with R⁶ to represent =O or =S and/or R⁷ is combined with R⁸ to represent =O or =S;
R¹⁰ and R¹² independently represent H, alkyl, cycloalkyl, cycloalkylalkyl, heteroaryl, aryl, aralkyl or -NR⁴⁰R⁴² wherein R⁴⁰ and R⁴² independently represent H, aryl, alkyl, aralkyl, heteroaryl, heteroarylalkyl, heterocycloalkyl, heterocycloalkylalkyl, heteroalkyl, cycloalkyl, cycloalkylalkyl, alkenyl and alkynyl;
R¹¹ represents alkyl or aryl;
X represents N, CH or C, such that when X is N or CH, there is a single bond to carbon atom 11 as represented by the solid line; or when X is C, there is a double bond to carbon atom 11, as represented by the solid and dotted lines;
the dotted line between carbon atoms 5 and 6 represents an optional double bond, such that when a double bond is present, A and B independently represent -NO₂, -R¹⁰, halo, -OR¹¹, -OCO₂R¹¹ or - OC(O)R¹⁰, and when no double bond is present between carbon atoms 5 and 6, A and B each independently represent H₂, -(OR¹¹)₂, H and halo, dihalo, alkyl and H, (alkyl)₂, -H and -OC(O)R¹⁰, H and -OR¹⁰, oxy, aryl and H, =NOR¹⁰ or -O-(CH₂)ₚ-O- wherein p is 2, 3 or 4; and
Z represents alkyl, aryl, aralkyl, heteroalkyl, heteroaryl, heteroarylalkyl, heterocycloalkyl, heterocycloalkylalkyl, -OR⁴⁰, -SR⁴⁰, -CR⁴⁰R⁴² or -NR⁴⁰R⁴² wherein R⁴⁰ and R⁴² are defined hereinbefore;
wherein the terms alkyl, alkoxy, alkenyl, alkynyl, aryl, aralkyl, cycloalkyl, cycloalkylalkyl, halo, heteroalkyl, heteroaryl, heteroarylalkyl, heterocycloalkyl, and heterocycloalkylalkyl are as defined hereinafter

Preferably in compound (1.0), there is a single bond at carbon atom 11, X is carbon, positions 3, 8 and 10 are substituted on the ring, preferably with halo; and Z is -NHR⁴⁰, preferably where R⁴⁰ is heteroarylalkyl, more preferably 3 or 4-methyl pyridyl N-oxide.

In another embodiment, the present invention is directed toward a pharmaceutical composition for inhibiting the abnormal growth of cells comprising an effective amount of compound (1.0) in combination with a pharmaceutically acceptable carrier.

In another embodiment, the present invention is directed toward the use of a compound of formula 1.0 for the manufacture of a medicament for inhibiting the abnormal growth of cells, including transformed cells, comprising administering an effective amount of compound (1.0) to a mammal (e.g., a human) in need of such treatment. Abnormal growth of cells refers to cell growth independent of normal regulatory mechanisms (e.g., loss of contact inhibition). This includes the abnormal growth of: (1) tumor cells (tumors) expressing an activated Ras oncogene; (2) tumor cells in which the Ras protein is activated as a result of oncogenic mutation in another gene; (3) benign and malignant cells of other proliferative diseases in which aberrant Ras activation occurs, and (4) benign or malignant cells that are activated by mechanisms other than the Ras protein. Without wishing to be bound by theory, it is believed that these compounds may function either through the inhibition of G-protein function, such as ras p21, by blocking G-protein isoprenylation, thus making them useful in the treatment of proliferative diseases such as tumor growth and cancer, or through inhibition of ras farnesyl protein transferase, thus making them useful for their antiproliferative activity against ras transformed cells.

The cells to be inhibited can be tumor cells expressing an activated ras oncogene. For example, the types of cells that may be inhibited include pancreatic tumor cells, lung cancer cells, myeloid leukemia tumor cells, thyroid follicular tumor cells, myelodysplastic tumor cells, epidermal carcinoma tumor cells, bladder carcinoma tumor cells or colon tumors cells. Also, the inhibition of the abnormal growth of cells by the treatment with compound (1.0) may be by inhibiting ras famesyl protein transferase. The inhibition may be of tumor cells wherein the Ras protein is activated as a result of oncogenic mutation in genes other than the Ras gene. Alternatively, compounds (1.0) may inhibit tumor cells activated by a protein other than the Ras protein.

This invention also provides the use of a compound of Formula 1.0 for the manufacture of a medicament for inhibiting tumor growth by administering an effective amount of compound (1.0) to a mammal (e.g., a human) in need of such treatment. In particular this invention provides the use of a compound of Formula 1.0 for the manufacture of a medicament for inhibiting the growth of tumors expressing an activated Ras oncogene by the administration of an effective amount of the above described compounds. Examples of tumors which may be inhibited include, but are not limited to, lung cancer (e.g., lung adenocarcinoma), pancreatic cancers (e.g., pancreatic carcinoma such as, for example, exocrine pancreatic carcinoma), colon cancers (e.g., colorectal carcinomas, such as, for example, colon adenocarcinoma and colon adenoma), myeloid leukemias (for example, acute myelogenous leukemia (AML)), thyroid follicular cancer, myelodysplastic syndrome (MDS), bladder carcinoma and epidermal carcinoma.

It is believed that this invention also provides the use of a compound of Formula 1.0 for the manufacture of a medicament for inhibiting proliferative diseases, both benign and malignant, wherein Ras proteins are aberrantly activated as a result of oncogenic mutation in other genes--i.e., the Ras gene itself is not activated by mutation to an oncogenic form--with said inhibition being accomplished by the administration of an effective amount of the carbonyl piperazinyl and piperidinyl compounds (1.0) described herein, to a mammal (e.g., a human) in need of such treatment. For example, the benign proliferative disorder neurofibromatosis, or tumors in which Ras is activated due to mutation or overexpression of tyrosine kinase oncogenes (e.g., neu, src, abl, Ick, and fyn), may be inhibited by the carbonyl piperazinyl and piperidinyl compounds (1.0) described herein.

In another embodiment, the present invention is directed toward the use of a compound of Formula 1.0 for the manufacture of a medicament for inhibiting ras famesyl protein transferase and the farnesylation of the oncogene protein Ras by administering an effective amount of compound (1.0) to mammals, especially humans. The administration of the compounds of this invention to patients, to inhibit famesyl protein transferase, is useful in the treatment of the cancers described above.

### DETAILED DESCRIPTION OF THE INVENTION

As used herein, the following terms are used as defined below unless otherwise indicated:
M⁺ -represents the molecular ion of the molecule in the mass spectrum;
MH⁺ -represents the molecular ion plus hydrogen of the molecule in the mass spectrum;
Bu-represents butyl;
Et-represents ethyl;
Me-represents methyl;
Ph-represents phenyl;
benzotriazol-1-yloxy represents 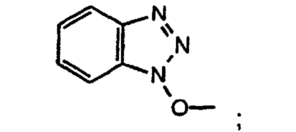
1-methyl-tetrazol-5-ylthio represents 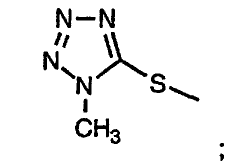
alkyl-(including the alkyl portions of alkoxy, alkylamino and dialkylamino)-represents straight and branched carbon chains and contains from one to twenty carbon atoms, preferably one to six carbon atoms; for example methyl, ethyl, propyl, iso-propyl, n-butyl, t-butyl, n-pentyl, isopentyl, hexyl and the like; wherein said alkyl group may be optionally and independently substituted with one, two, three or more of the following: halo, alkyl, aryl, alkoxy, amino, alkylamino, cyano, -CF₃, dialkylamino, hydroxy, oxy (=O), phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰;
alkoxy-an alkyl moiety of one to 20 carbon atoms covalently bonded to an adjacent structural element through an oxygen atom, for example, methoxy, ethoxy, propoxy, butoxy, pentoxy, hexoxy and the like; wherein said alkoxy group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰;
alkenyl-represents straight and branched carbon chains having at least one carbon to carbon double bond and containing from 2 to 12 carbon atoms, preferably from 2 to 6 carbon atoms and most preferably from 3 to 6 carbon atoms; wherein said alkenyl group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰;
alkynyl-represents straight and branched carbon chains having at least one carbon to carbon triple bond and containing from 2 to 12 carbon atoms, preferably from 2 to 6 carbon atoms; wherein said alkynyl group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰;
aryl (including the aryl portion of aralkyl)-represents a carbocyclic group containing from 6 to 15 carbon atoms and having at least one aromatic ring (e.g., aryl is phenyl), wherein said aryl group optionally can be fused with aryl, cycloalkyl, heteroaryl or heterocycloalkyl rings; and wherein any of the available substitutable carbon and nitrogen atoms in said aryl group and/or said fused ring(s) may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰;
aralkyl - represents an alkyl group, as defined above, wherein one or more hydrogen atoms of the alkyl moiety have been substituted with one or more aryl groups; wherein said aralkyl group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰; Representative aralkyl groups include benzyl and diphenylmethyl;
cycloalkyl-represents saturated carbocyclic rings branched or unbranched of from 3 to 20 carbon atoms, preferably 3 to 7 carbon atoms; wherein said cycloalkyl group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰;
cycloalkylalkyl - represents an alkyl group, as defined above, wherein one or more hydrogen atoms of the alkyl moiety have been substituted with one or more cycloalkyl groups; wherein said cycloalkylalkyl group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰;
halo-represents fluoro, chloro, bromo and iodo;
heteroalkyl-represents straight and branched carbon chains containing from one to twenty carbon atoms, preferably one to six carbon atoms interrupted by 1 to 3 heteroatoms selected from -O-, -S- and -N-; wherein any of the available substitutable carbon and nitrogen atoms in said heteroalkyl chain may be optionally and independendently substituted with one, two, three or more of the following: halo, C₁-C₆ alkyl, aryl, cyano, hydroxy, alkoxy, oxy, phenoxy, -CF₃, -OCF₃, amino, alkylamino, dialkylamino, heterocycloalkyl, -SO₂NH₂, -NHSO₂R¹⁰, -SO₂NHR¹⁰, -SO₂R¹⁰, -SOR¹⁰, -SR¹⁰, or -NHSO₂, -NO₂, -CONR¹⁰, -NCOR¹⁰ or -COOR¹⁰;
heteroaryl-represents cyclic groups having at least one heteroatom selected from O, S and N, said heteroatom(s) interrupting a carbocyclic ring structure and having a sufficient number of delocalized pi electrons to provide aromatic character, with the aromatic heterocyclic groups containing from 2 to 14 carbon atoms,wherein said heteroaryl group optionally can be fused with one or more aryl, cycloalkyl, heteroaryl or heterocycloalkyl rings; and wherein any of the available substitutable carbon or nitrogen atoms in said heteroaryl group and/or said fused ring(s) may be optionally and independendently substituted with one, two, three or more of the following: halo, C₁-C₆ alkyl, aryl, cyano, hydroxy, alkoxy, oxy, phenoxy, -CF₃, -OCF₃, amino, alkylamino, dialkylamino, heterocycloalkyl, -SO₂NH₂, -NHSO₂R¹⁰, -SO₂NHR¹⁰, -SO₂R¹⁰, -SOR¹⁰, -SR¹⁰, or -NHSO₂, -NO₂, -CONR¹⁰, -NCOR¹⁰ or -COOR¹⁰. Representative heteroaryl groups can include, for example, furanyl, imidazoyl, pyrimidinyl, triazolyl, 2-, 3- or 4-pyridyl or 2-, 3- or 4-pyridyl N-oxide wherein pyridyl N-oxide can be represented as: 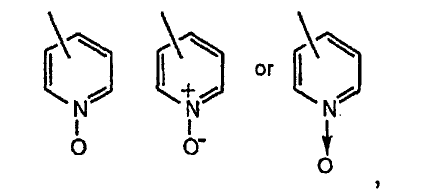
heteroarylalkyl - represents an alkyl group, as defined above, wherein one or more hydrogen atoms have been replaced by one or more heteroaryl groups; wherein said heteroarylalkyl group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰; as exemplified by 2-, 3- or 4-pyridylmethyl or 2-, 3- or 4-pyridylmethyl N-oxide;
heterocycloalkyl-represents a saturated, branched or unbranched carbocylic ring containing from 3 to 15 carbon atoms, preferably from 4 to 6 carbon atoms, which carbocyclic ring is interrupted by 1 to 3 heteroatoms selected from -O-, -S- and -N-, wherein optionally, said ring may contain one or two unsaturated bonds which do not impart aromatic character to the ring; and wherein any of the available substitutable carbon and nitrogen atoms in the ring may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR^{10,} -SR¹⁰, -NHSO₂, -NO₂, -CONR¹⁰, -NCOR¹⁰ or -COOR¹⁰. Representative heterocycloalkyl groups can include 2- or 3-tetrahydrofuranyl, 2- or 3- tetrahydrothienyl, 1-, 2-, 3- or 4-piperidinyl, 2- or 3-pyrrolidinyl, 1-, 2- or 3-piperizinyl, 2- or 4-dioxanyl, morpholinyl, 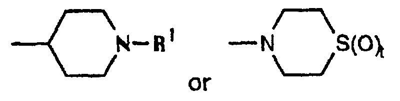 wherein R¹ is defined hereinbefore and t is 0, 1 or 2.
heterocycloalkylalkyl- represents an alkyl group, as defined above, wherein one or more hydrogen atoms have been replaced by one or more heterocycloalkyl groups; wherein optionally, said ring may contain one or two unsaturated bonds which do not impart aromatic character to the ring; and wherein said heterocycloalkylalkyl group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰.

The following solvents and reagents are referred to herein by the abbreviations indicated: tetrahydrofuran (THF); ethanol (EtOH); methanol (MeOH); acetic acid (HOAc or AcOH); ethyl acetate (EtOAc); N,N-dimethylformamide (DMF); trifluoroacetic acid (TFA); trifluoroacetic anhydride (TFAA); 1-hydroxybenzotriazole (HOBT); m-chloroperbenzoic acid (MCPBA); triethylamine (Et₃N); diethyl ether (Et₂O); ethyl chloroformate (ClCO₂Et); and 1-(3-dimethylaminopropyl)-3-ethyl carbodiimide hydrochloride (DEC).

Reference to the position of the substituents R¹, R², R³, and R⁴ is based on the numbered ring structure:

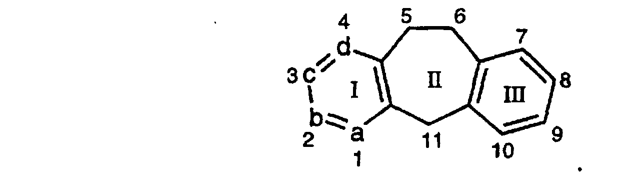

Certain compounds of the invention may exist in different stereoisomeric forms (e.g., enantiomers and diastereoisomers) . The invention contemplates all such stereoisomers both in pure form and in mixture, including racemic mixtures. For example, the carbon atom at the C-11 position can be in the S or R stereoconfiguration.

Certain tricyclic compounds will be acidic in nature, e.g. those compounds which possess a carboxyl or phenolic hydroxyl group. These compounds may form pharmaceutically acceptable salts. Examples of such salts may include sodium, potassium, calcium, aluminum, gold and silver salts. Also contemplated are salts formed with pharmaceutically acceptable amines such as ammonia, alkyl amines, hydroxyalkylamines, N-methylglucamine and the like.

Certain basic tricyclic compounds also form pharmaceutically acceptable salts, e.g., acid addition salts. For example, the pyridonitrogen atoms may form salts with strong acid, while compounds having basic substituents such as amino groups also form salts with weaker acids. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those in the art. The salts are prepared by contacting the free base form with a sufficient amount of the desired acid to produce a salt in the conventional manner. The free base forms may be regenerated by treating the salt with a suitable dilute aqueous base solution such as dilute aqueous NaOH, potassium carbonate, ammonia and sodium bicarbonate. The free base forms differ from their respective salt forms somewhat in certain physical properties, such as solubility in polar solvents, but the acid and base salts are otherwise equivalent to their respective free base forms for purposes of the invention.

All such acid and base salts are intended to be pharmaceutically acceptable salts within the scope of the invention and all acid and base salts are considered equivalent to the free forms of the corresponding compounds for purpopses of the invention.

Compounds of the invention may be made by the methods described in the examples below, and by the methods described in WO 95/10516 published April 20, 1995--see, for example, the methods for preparing compounds of Formula 400.00.

On page 57 at lines 7 to 16 of WO 95/10516 a process is disclosed for introducing substituents at the C-3 position of pyridine Ring I of Formula 1.0 by nitrating a compound of Formula 415.00. The nitro group may then be reduced to the corresponding amine using the disclosed reagents or powdered Zn and either CuCl₂ or CuBr₂ in aqueous EtOH . Compounds of the present invention can be prepared according to the following Scheme I: 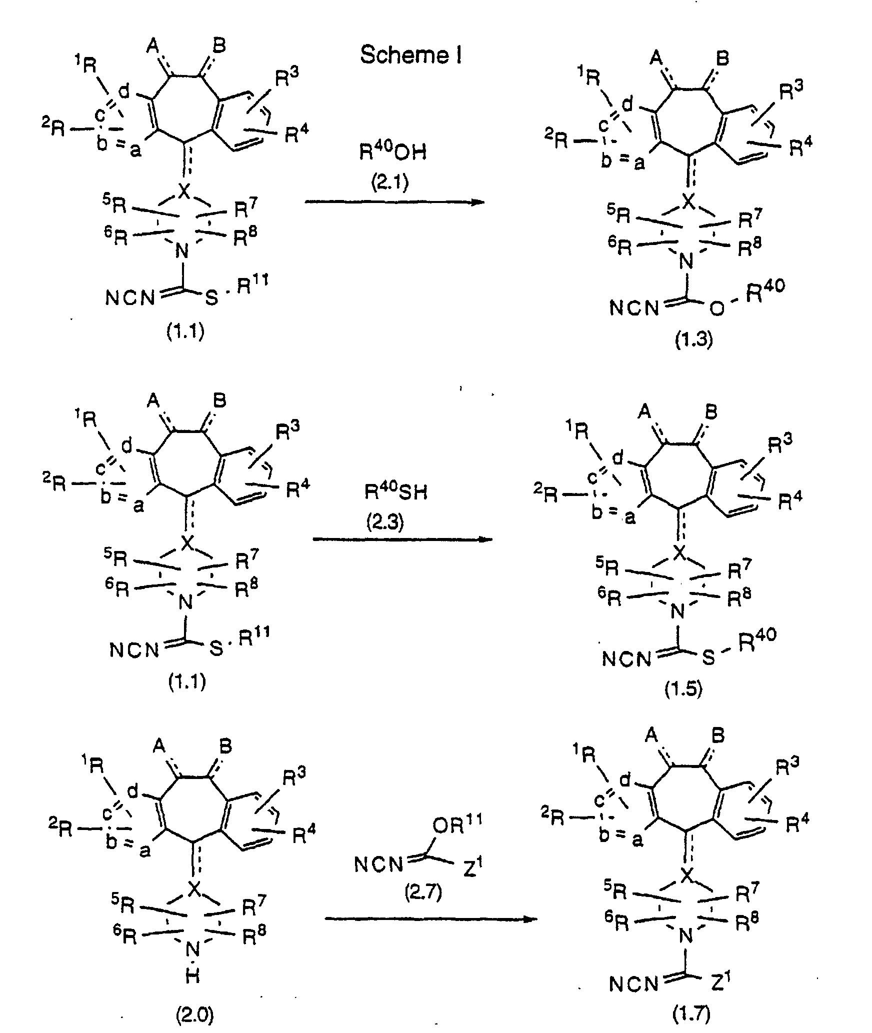 wherein Z¹ represents alkyl, aryl, aralkyl, heteroalkyl, heteroaryl, heteroarylalkyl, heterocycloalkyl, heterocycloalkylalkyl and -CR⁴⁰R⁴²; the dotted line represents a single or double bond; and a, b, c, d, A, B, R¹, R², R³, R⁴, R⁵, R⁶, R⁷, R⁸, R⁸, R¹¹, R⁴⁰ and R⁴² are as defined hereinbefore.

Referring to the Scheme I, compounds of formula (1.3) can be prepared by reacting the compounds of formula (1.1), preferably where R¹¹ is alkyl such as methyl, with alcohol (R⁴⁰OH) of formula (2.1) in the presence of a suitable base, and optional non-protic solvent, in amounts and under conditions effective to give compounds (1.3). Suitable bases include organic bases such as pyridine and triethylamine; or inorganic bases of alkali and alkaline earth metals including carbonates such as sodium, lithium, potassium and cesium carbonates, hydroxides such as sodium and potassium hydroxides; hydrides such as sodium or potassium hydride; and sodium t-butoxide, preferably sodium hydride. Suitable non-protic solvents include ethers, dimethyformamide (DMF), dimethylsulfoxide (DMSO), tetrahydrofuran (THF), dimethoxyethane (DME) and mixtures thereof, preferably DMF. Alternatively, the reaction can be carried out neat, using an excess of the alcohol. The amounts of alcohol (2.1) can range from about 1 to about 10 moles per mole of compound (1.1). Temperatures can range from 0° to 100°C, or reflux of the reaction mixture.

Compounds of formula (1.5) can be prepared by reacting the compounds of formula (1.1) with thiol (R⁴⁰SH) of formula (1.1) in the presence of a suitable base, and optional non-protic solvent to give compounds (1.5), using reaction conditions as described for preparing compounds (1.3), above.

Compounds of formula (1.7) can be prepared by reacting the compounds of formula (2.0) with N-cyanaoimidate of formula (2.7) in the presence of an optional non-protic solvent to give compounds (1.7), using reaction conditions as described for preparing compounds (1.3), above.

Compounds of the present invention can be prepared according to the following Scheme II: 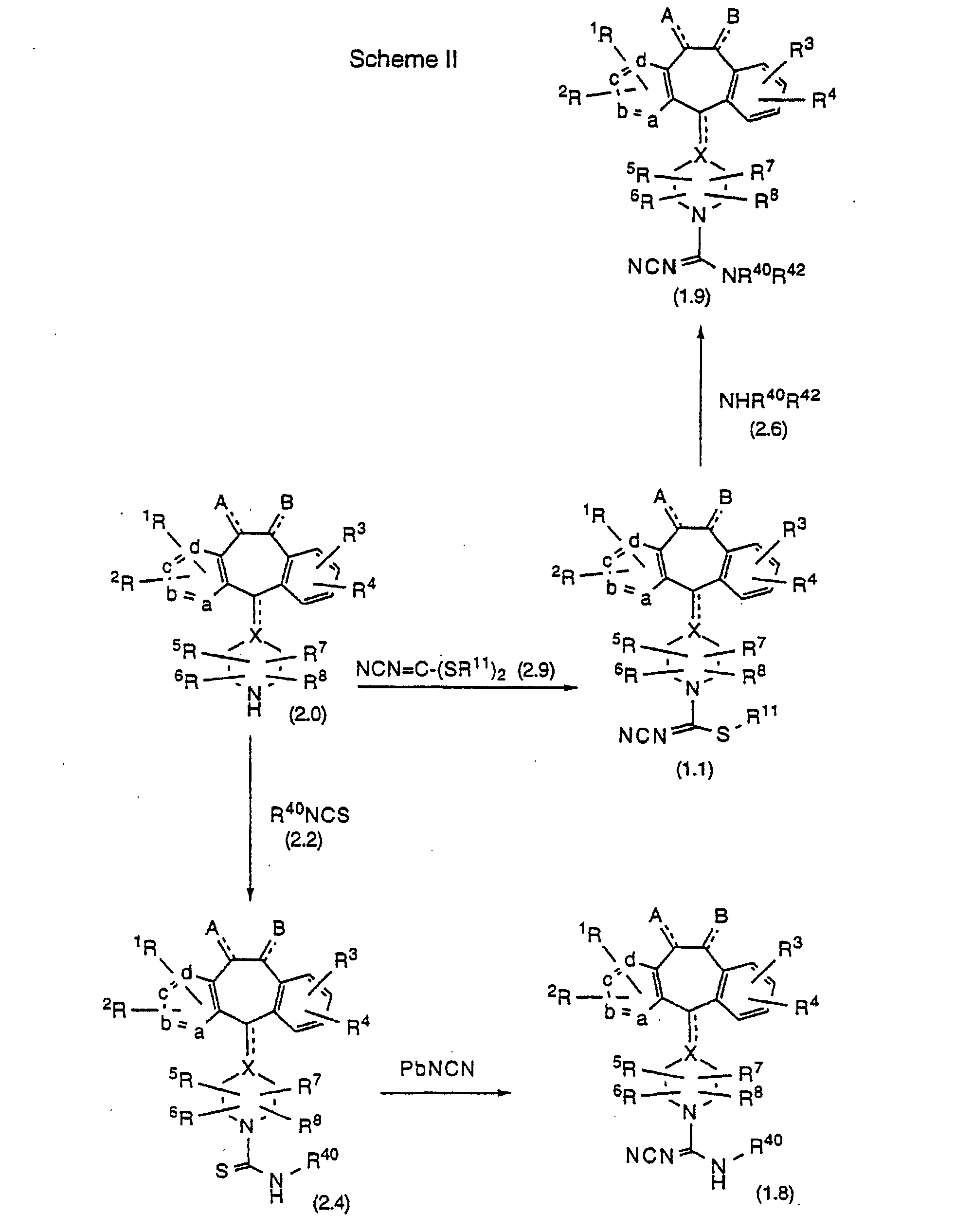 wherein a, b, c, d, A, B, R¹, R², R³, R⁴, R⁵, R⁶, R⁷, R⁸, R⁸, R¹¹, R⁴⁰ and R⁴² are as defined hereinbefore; and the dotted line represents a single or double bond..

Referring to the Scheme II, compounds of formula (1.1) can be prepared by reacting the compounds of formula (2.0) with N-cyanodithioiminocarbonate (NCN=C-(SR¹¹)₂ of formula (2.9) in the presence of a suitable protic or non-protic solvent, in amounts and under conditions effective to give compounds (1.1). Suitable protic solvents include C₁₋₁₀ alkanols, such as methanol, ethanol, propanol, hexanol, octanol, decanol and the like. Suitable non-protic solvents are described hereinbefore. The amounts of N-cyanodithioiminocarbonate (2.9) can range from about 1 to about 10 moles per mole of compound (2.0). Temperatures can range from 0° to 100°C, or reflux of the reaction mixture.

Compounds of formula (1.9) can be prepared by reacting the compounds of formula (1.1) with amine (NHR⁴⁰R⁴²) of formula (2.6) with an optional base and/or optional protic or aprotic solvent such as those described hereinbefore. In a first procedure, compound (1.1) is reacted with amine (2.6) neat, at temperatures ranging from about 50° to 80°C. In a second procedure, compound (1.1) is reacted with about equimolar amounts of amine (2.6) in the presence of a base such as sodium hydride and an aprotic solvent such as DMSO or DMF. In a third procedure, compound (1.1) is reacted with excess amine (2.6) in an protic solvent such as ethanol. In a fourth procedure, compound (1.1) is reacted with amine (2.6) neat, using catalytic amounts of base, such as sodium hydride. In a fifth procedure, compound (1.1) is reacted with greater than two equivalents of amine (2.6) in an aprotic solvent such as DMF at a temperature of about 75°C. Except as noted otherwise, temperatures can range from 0° to 100°C, or reflux of the reaction mixture and amounts of amine (2.6) can range from 1 to about 10 moles per mole of compound (1.1).

Compounds of formula (2.4) can be prepared by reacting the compounds of formula (2.0) with isothiocyanate (R⁴⁰NCS) of formula (2.2) in the presence of a suitable non-protic solvent in amounts and under conditions effective to give compounds (2.4). Suitable non-protic solvents are described hereinbefore. The amounts of isothiocyanate (2.2) can range from about 1 to about 10 moles per mole of compound (2.0). Temperatures can range from 0° to 100°C, or reflux of the reaction mixture.

Compounds of formula (1.8) can be prepared by reacting the compounds of formula (2.4) with lead cyanamine (PbNCN) in the presence of a suitable non-protic solvent in amounts and under conditions effective to give compounds (2.4). Suitable non-protic solvents are described hereinbefore, preferably DMF. The amounts of lead cyanamine can range from about 1 to about 10 moles per mole of compound (2.4). Temperatures can range from 0° to 100°C, or reflux of the reaction mixture.

Compound of fomula 1.0 can be isolated from the reaction mixture using conventional procedures, such as, for example, extraction of the reaction mixture from water with organic solvents, evaporation of the organic solvents, followed by chromatography on silica gel or other suitable chromatographic media.

Compounds of the present invention and preparative starting materials therof, are exemplified by the following examples, which should not be construed as limiting the scope of the disclosure.

### Example 1. Methyl 4-(3-bromo-8-chloro-5,6-dihydro-11H-benzo[5.6]cyclohepta[1,2-b]pyridin-11-ylidene)-B-cyano-1-piperidinecarboximidothioate

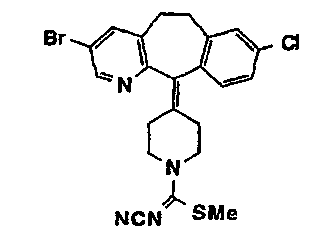

Dissolve 4-(3-bromo-8-chloro-5,6-dihydro-11H-benzo[5.6]cyclohepta[1,2-b]pyridin-11-ylidene)-1-piperidine (40 gm, 0.1 mol) in 600 mL of absolute ethanol. Add dimethyl N-cyanodithioiminocarbonate (16.5 gm, 0.11 mol) and reflux under a dry nitrogen atmosphere for two hours. Evaporate to dryness to obtain a brown foamy solid. Chromatograph on silica gel using 25% to 50% ethylacetate/hexanes as the eluent to obtain 50.8 gm of title compound. FABMS M+1= 489

### Example 2. Ethyl 4-[[[4-(3-bromo-8-chloro-5,6-dihydro-11H-benzo[5.6]cyclohepta[1,2-b]pyridin-11-ylidene)-1-piperidinyl] (cyanoimino)methyl]amino]-1-piperidinecarboxylate

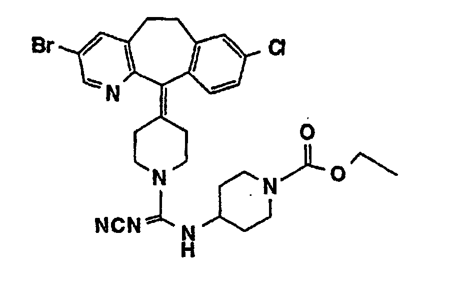

Dissolve methyl 4-(3-bromo-8-chloro-5,6-dihydro-11H-benzo[5.6]cyclohepta[1,2-b]pyridin-11-ylidene)-B-cyano-1-piperidinecarboximidothioate (0.1 gm, 0.20 mmol) in 1 ml of ethyl 4-amino-1-piperidinecarboxylate. Stir at 100 °C for 18 hours. Let cool to room temperature. Add the mixture to water and filter the resulting solids. Dissolve the solid in methylene chloride and chromatograph on silica gel using 5% methanol/methylene chloride as eluent to obtain 0.15 gm (34%) of title product. FABMS M+1= 613

### Example 3. [[4-(3-bromo-8-chloro-5,6-dihydro-11H-benzo[5.6]cyclohepta[1,2-b]pyridin-11-ylidene)-1-piperidinyl](4-pyridinylamino)methylene]cyanamide N1-oxide

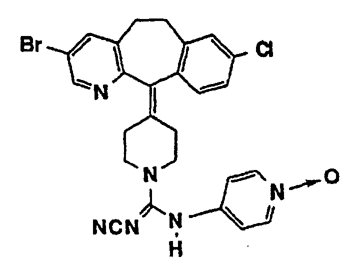

Dissolve methyl 4-(3-bromo-8-chloro-5,6-dihydro-11H-benzo[5.6]cyclohepta[1,2-b]pyridin-11-ylidene)-B-cyano-1-piperidinecarboximidothioate (0.3 g, 0.60 mmole) and 4-aminopyridyl-N-oxide (0.07 gm, 0.60 mmol) in 5 mL of dimethylsulfoxide under a dry nitrogen atmosphere at ambient temperature. Add sodium hydride as a 60% oil dispersion (24 mg,0.6 mmol) portionwise while stirring. After stirring 2 hours, add the reaction mixture to brine and extract with 20 mL of methylene chloride three times. Combine the extracts, dry over magnesium sulfate, filter and evaporate to an oil. Chromatograph the oil on a silica gel coloumn using 2% to 10% methanol in methylene chloride to obtain 0.15 g (47%) of title compound as a solid. FABMS M+1=549.1

### Example 4. [[4-(3-bromo-8-chloro-5,6-dihydro-11H-benzo[5,6]cyclohepta[1,2-b]pyridin-11-ylidene)-1-piperidinyl][cyclopropylamino]methylene] cyanamide

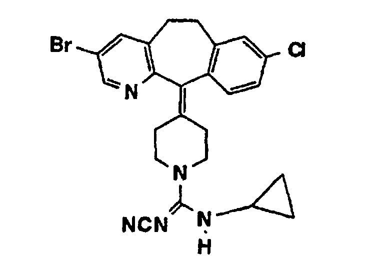

Dissolve methyl 4-(3-bromo-8-chloro-5,6-dihydro-11H-benzo[5.6]cyclohepta[1,2-b]pyridin-11-ylidene)-B-cyano-1-piperidinecarboximidothioate (0.15 gm, 0.31 mmol) in 3 mL of absolute ethanol. Add cyclopropylamine (0.3 mL, 4.30 mmol) and stir at ambient temperature. After 24 hours , add the reaction mixture to brine and extract with 20 mL of methylene chloride three times. Combine the extracts, dry over magnesium sulfate, filter and evaporate to an oil. Chromatograph the oil on a silica gel coloumn using 2% to 10% methanol in methylene chloride to obtain 0.133 gm (86%) of the title compound as a solid.
FABMS M+1=498.

### Example 5. [[4-(3-bromo-8-chloro-5,6-dihydro-11H-benzo[5.6]cyclohepta[1,2-b]pyridin-11-ylidene)-1-piperidinyl[[(4-methoxyphenyl)methyl]amino] methylene] cyanamide

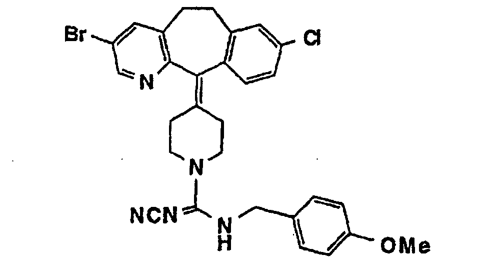

Dissolve methyl 4-(3-bromo-8-chloro-5,6-dihydro-11H-benzo[5,6]cyclohepta[1,2-b]pyridin-11-ylidene)-B-cyano-1-piperidinecarboximidothioate (0.5 gm, 1.02 mmol) in 5 mL of DMF. Add 4-methoxybenzylamine (0.4 mL, 2.9 mmol) and stir at 75 °C for 24 hours. Add to brine and extract with ethyl acetate three times. Dry the extract over magnesium sulfate, filter, and evaporate to dryness. Chromatograph on silica gel using 5% methanol/methylene chloride as eluent to obtain 0.4 gm, (68%) of title compound. FABMS M+1= 578

### Example 6. [[4-(3-bromo-8-chloro-5,6-dihydro-11H-benzo[5.6]cyclohepta[1,2-b]pyridin-11-ylidene)-1-piperidinyl] [(4-fluorophenyl)amino]methylene]cyanamide

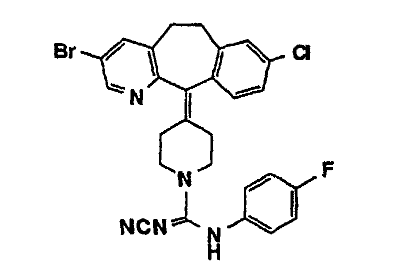

Dissolve methyl 4-(3-bromo-8-chloro-5,6-dihydro-11H-benzo[5.6]cyclohepta[1,2-b]pyridin-11-ylidene)-B-cyano-1-piperidinecarboximidothioate (0.25 gm, 0.51 mmol) in 2.5 mL of 4-fluoroanaline. While sitrring under a dry nitrogen atmosphere add approximately 10 mg of sodium hydride and stir at 100 °C for 1 hr. Let cool to room temperature. Add to brine and extract with ethyl acetate three times. Dry the extract over magnesium sulfate, filter, and evaporate to dryness and chromatograph on silica gel using 5% methanol/methylene chloride as eluent to obtain 0.155 gm (55%) of title compound. FABMS M+1=552.

### Example 7. [[(4H-1,3-benzodioxin-6-yl) amino] [4-(3-bromo-8-chloro-5,6-dihydro-11H-benzo[5,6]cyclohepta[1,2-b]pyridin-11-ylidene)-1-piperidinyl] methylene] cyanamide

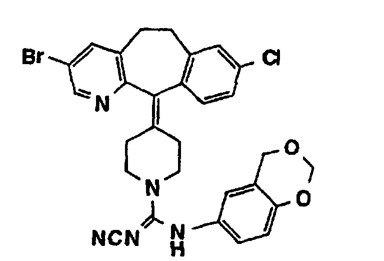

Dissolve 4-(3-bromo-8-chloro-5,6-dihydro-11H-benzo[5.6]cyclohepta[1,2-b]pyridin-11-ylidene)-N-(4H-1,3-benzodioxin-6-yl)-1-piperidinecarbothioamide (0.1 gm, 0.198 mmol) in dry N,N-dimethylformamide (DMF). Add lead cyanamide (98 mg, 0.39 mmol) and benzyltriethylammonium chloride (5 mg) and stir at 90 °C for 2 days. ). Add lead cyanamide (98 mg, 0.39 mmol) and benzyltriethylammonium chloride (5 mg) again and sitr for 24 hrs. Add to brine and extract with ethyl acetate three times. Dry the extract over magnesium sulfate, filter, and evaporate to dryness. Chromatograph on silica gel using 5% methanol/methylene chloride as eluent to obtain 39 mg (38%) of title compound. FABMS M+1= 701

### Example 8. [[4-(3, 10-dibromo-8-chlor-5,6-dihydro-11H-benzo[5.6]cyclohepta[1,2-b]pyridin-11-ylidene)-1-piperidinyl](3-pyridyinyl)methylene]cyanamide

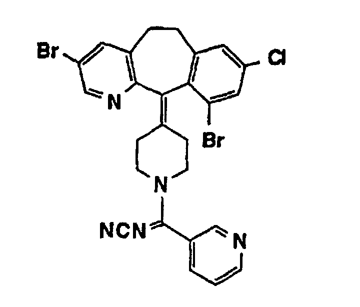

Dissolve 4-(3,10-dibromo-8-chloro-5,6-dihydro-11H-benzo[5.6]cyclohepta[1,2-b]pyridin-11-ylidene)-1-piperidine (0.2 gm, 0.43 mmol) in 2 mL of DMF. Add isopropyl-N-cyano-3-pyridylimidate [ref: Chem. Pharm. Bull. 42(12) 2475 (1994)] (0.16gm, 0.86 mmol) and stir at 75 °C for 24 hours. Add to brine and extract with ethyl acetate three times. Dry the extract over magnesium sulfate, filter, and evaporate to dryness. Chromatograph on silica gel using 5% methanol/methylene chloride as eluent to obtain 0.16 gm of title compound. FABMS M+1=599.

### Example 9. N-cyano-4-(3,10-dibromo-8-chloro-6,11-dihydro-5h-benzo[5,6]cyclohepta[1,2-b]pyridin-11-yl)-n'-(3-pyridinylmethyl)-1-piperidinecarboximidamide n1-oxide

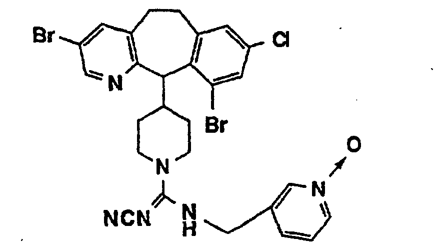

Dissolve methyl 4-(3-bromo-8-chloro-5,6-dihydro-11H-benzo[5.6]cyclohepta[1 ,2-b]pyridin-11-ylidene)-B-cyano-1-piperidinecarboximidothioate (1.0 g, 1.76 mmole) and 3-aminomethylpyridyl-N-oxide (0..65 gm, 5.8 mmol) in 10 mL of N,N-dimethylformamide under a dry nitrogen atmosphere at 135 °C while stirring. After stirring 2 hours, add the reaction mixture to brine and extract with 20 mL of methylene chloride three times. Combine the extracts, dry over magnesium sulfate, filter and evaporate to an oil. Chromatograph the oil on a silica gel coloumn using 2% to 10% methanol in methylene chloride to obtain 0.23 g of title compound as a solid. FABMS M+1=563

Using the processes described above and substituting appropriate reagents, the compounds described in the following table are prepared.

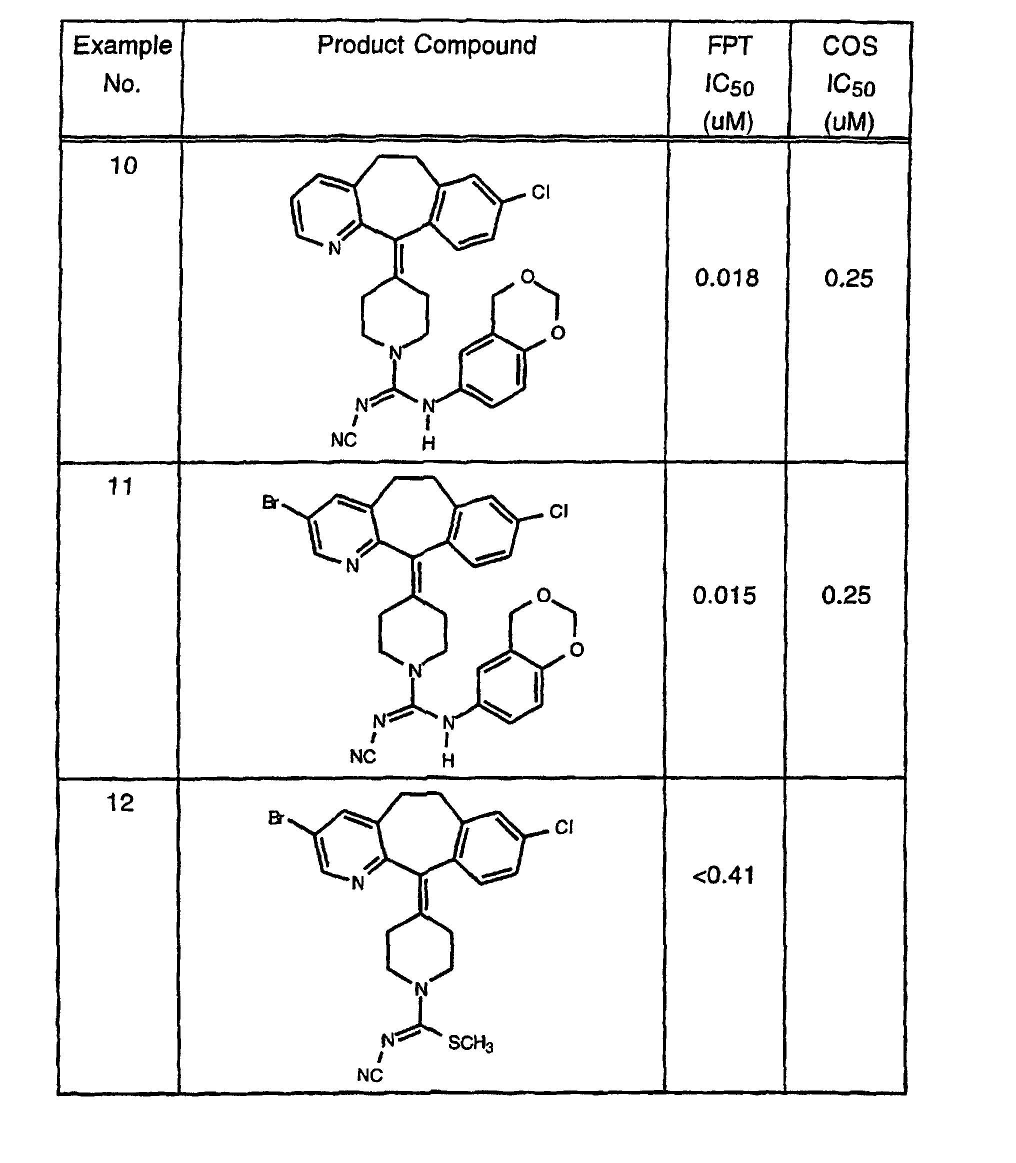

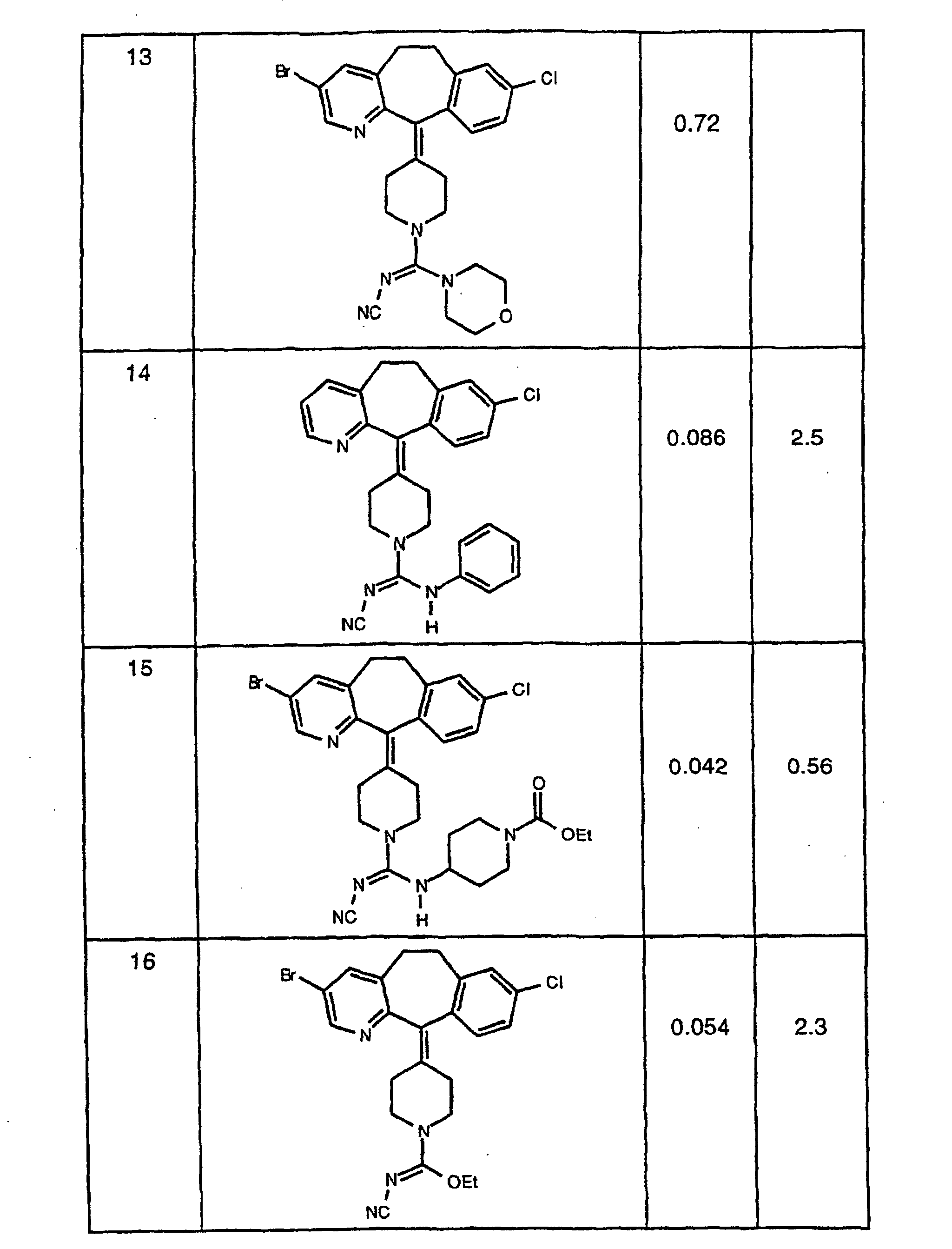

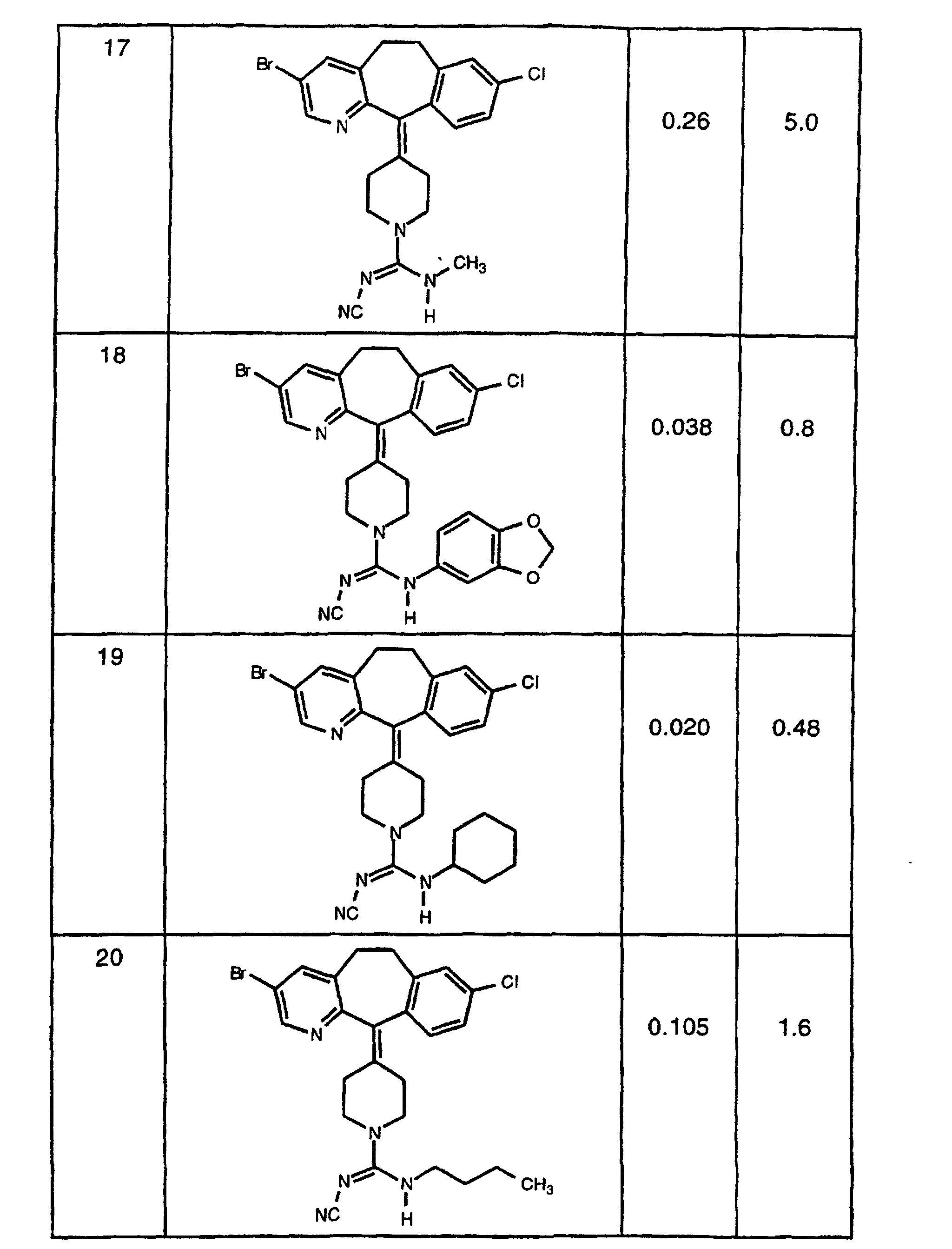

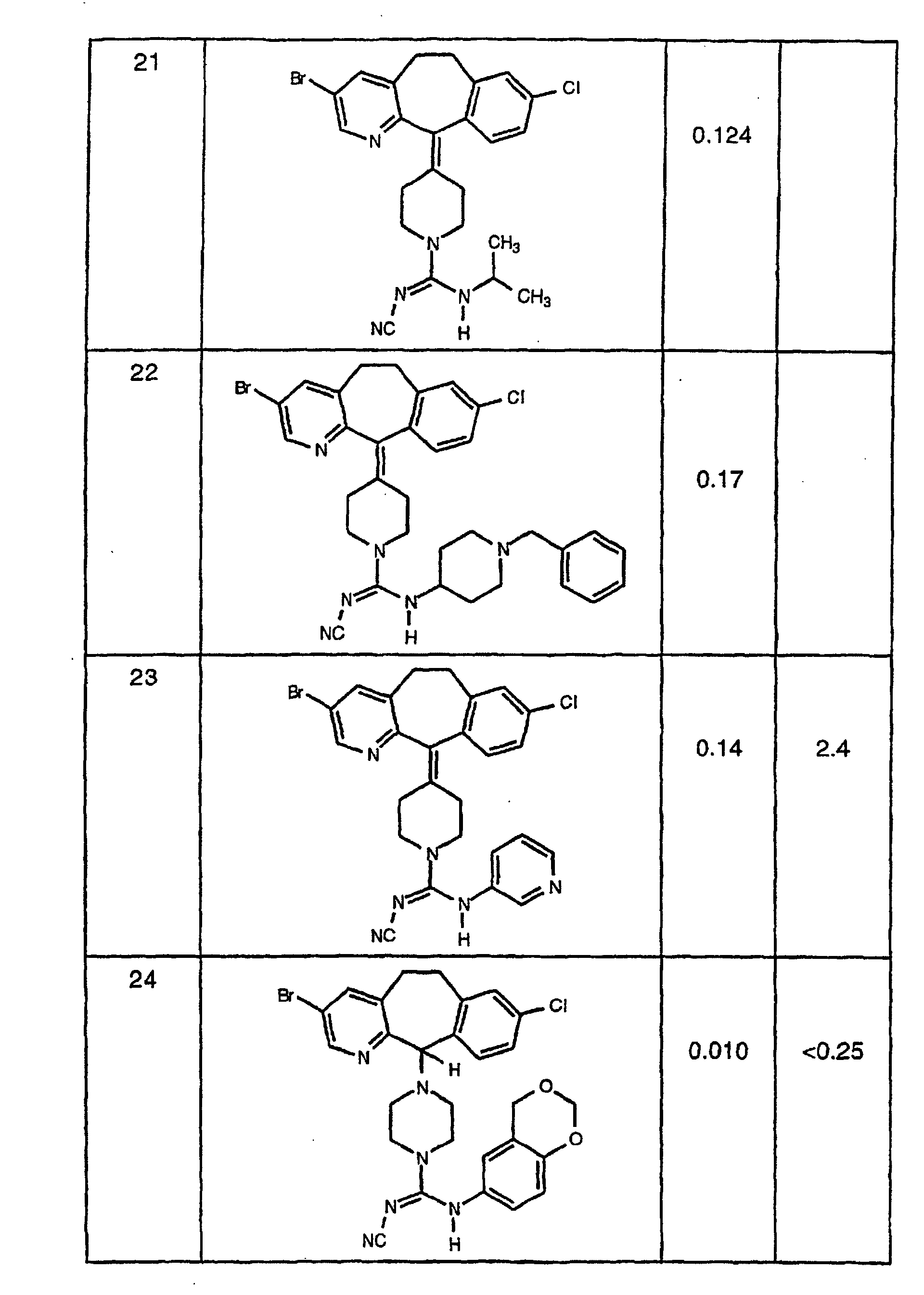

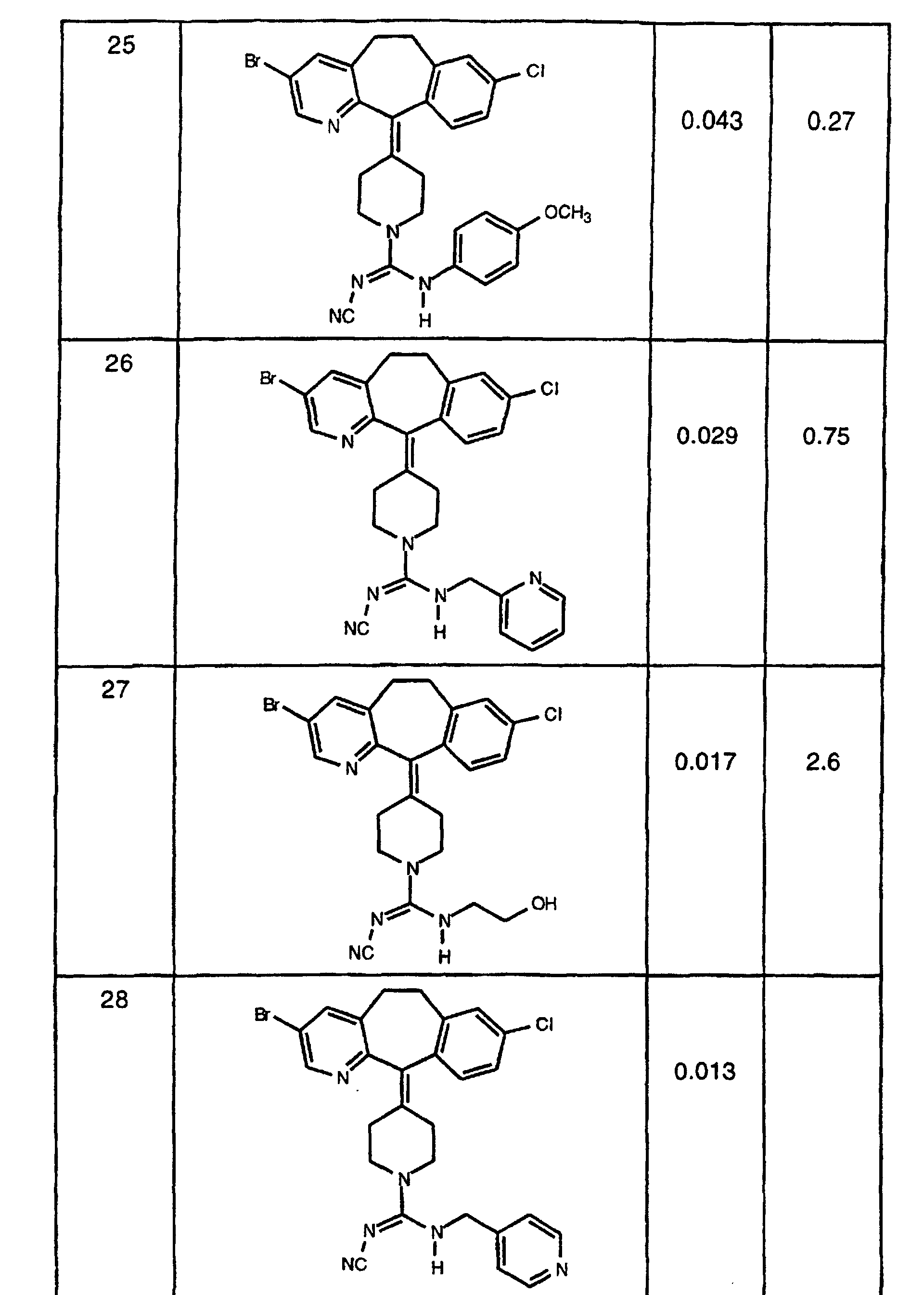

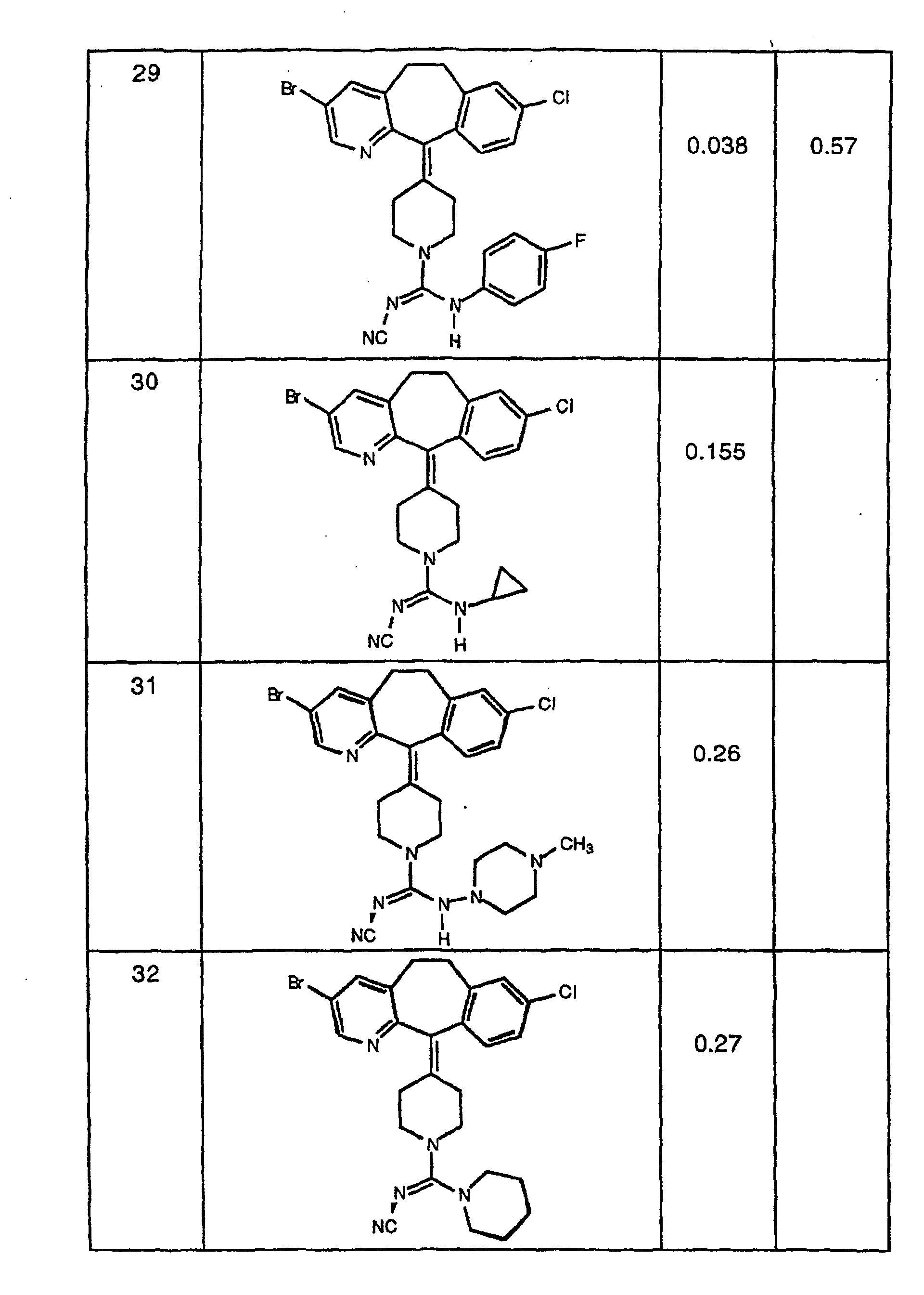

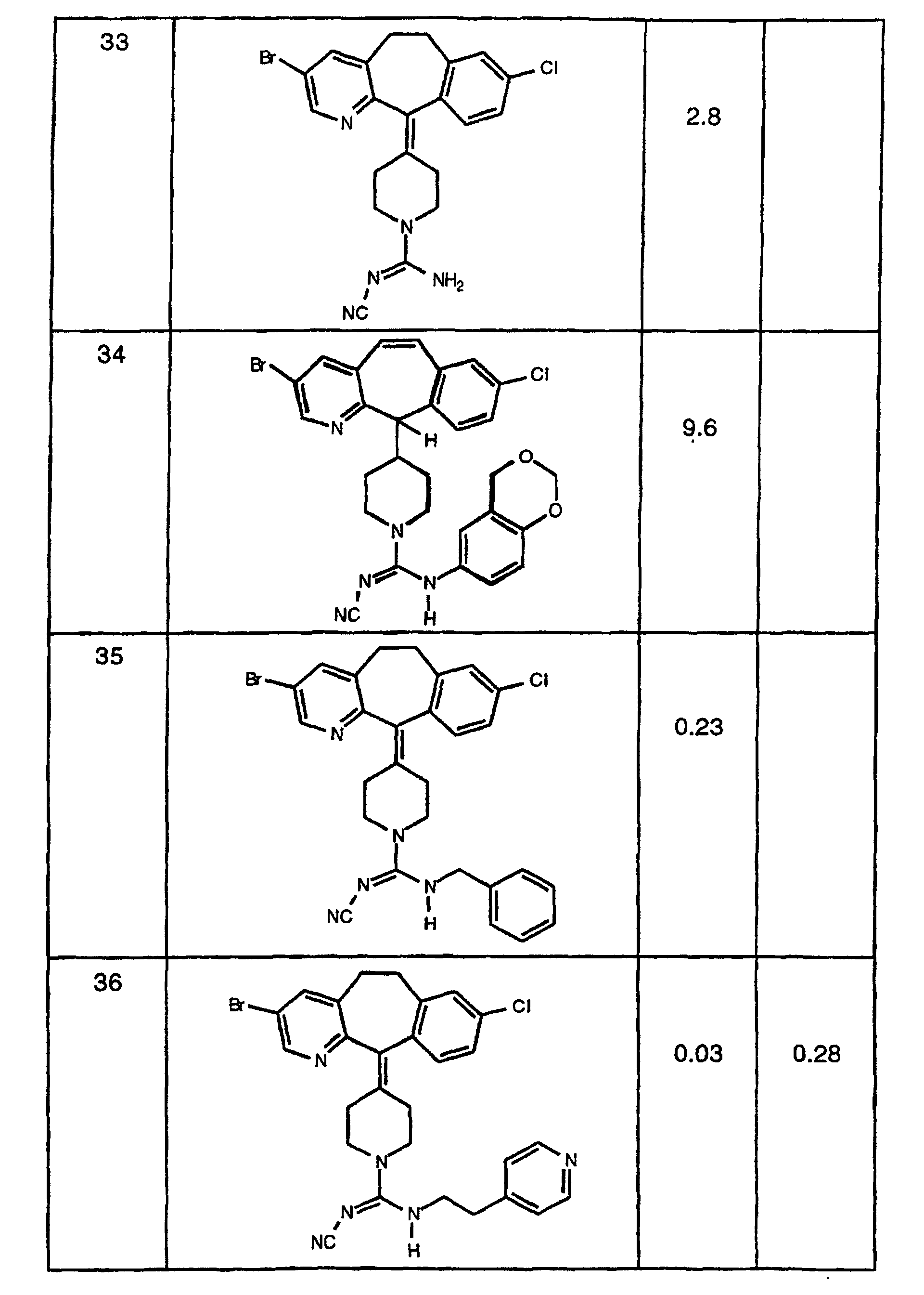

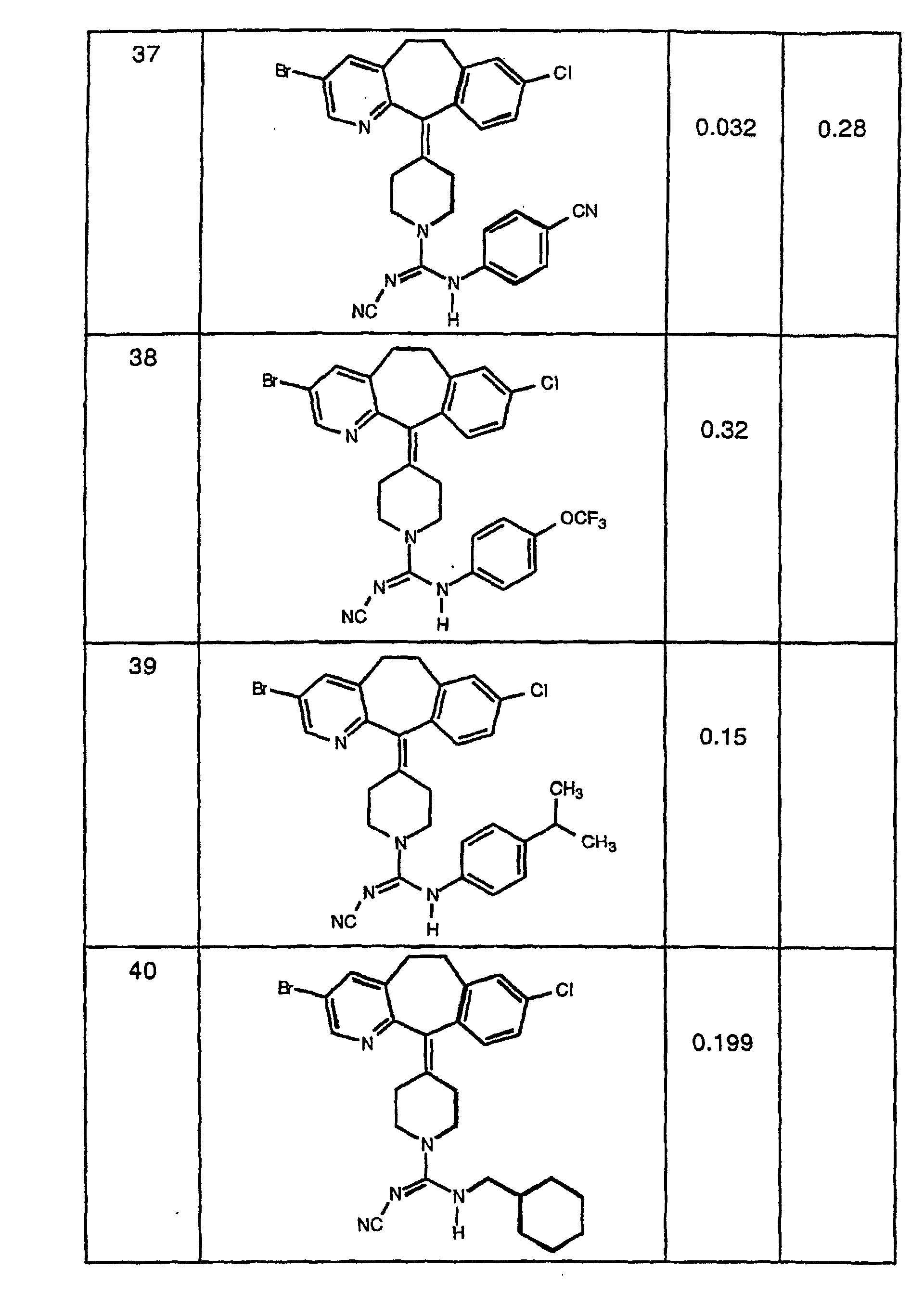

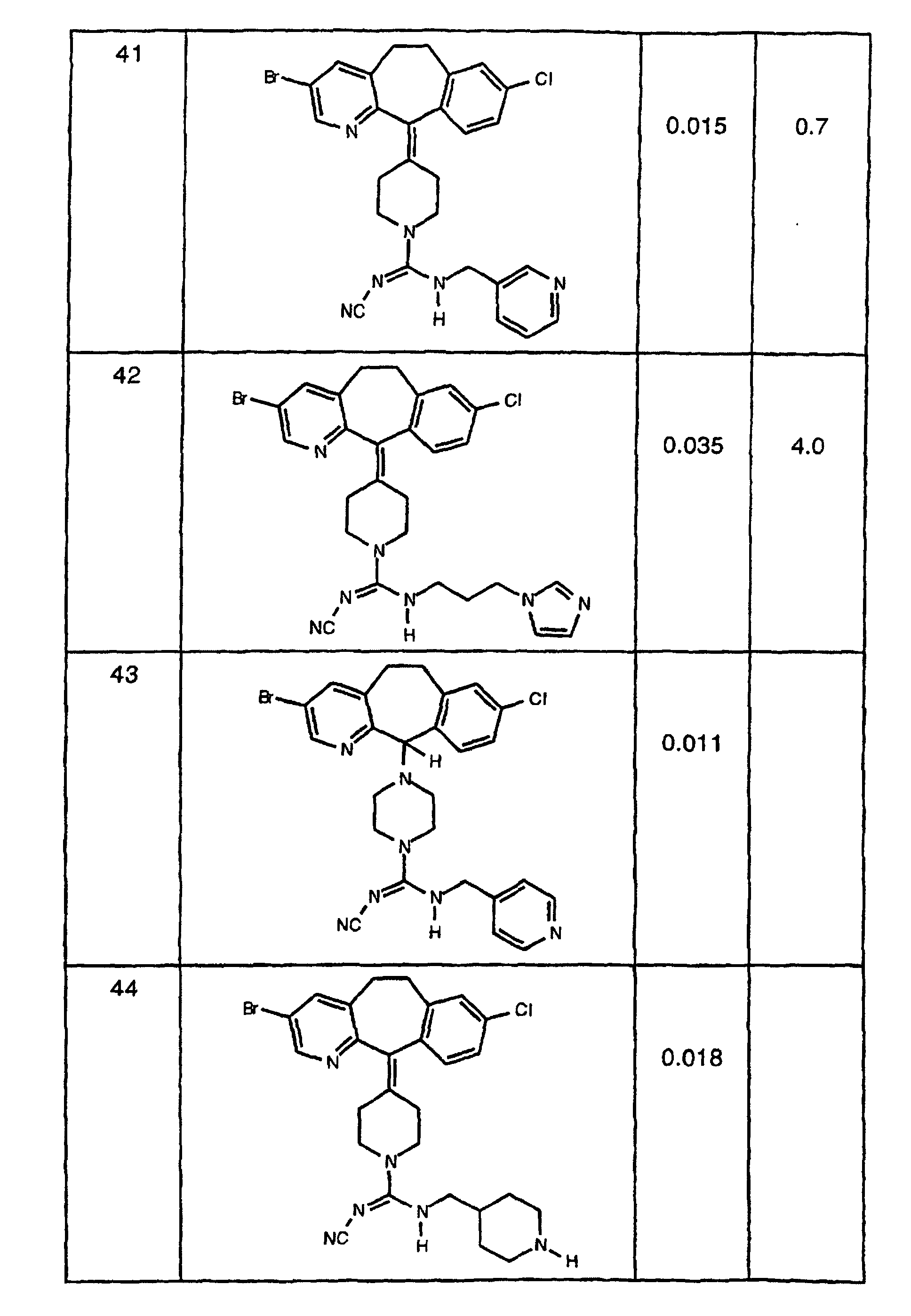

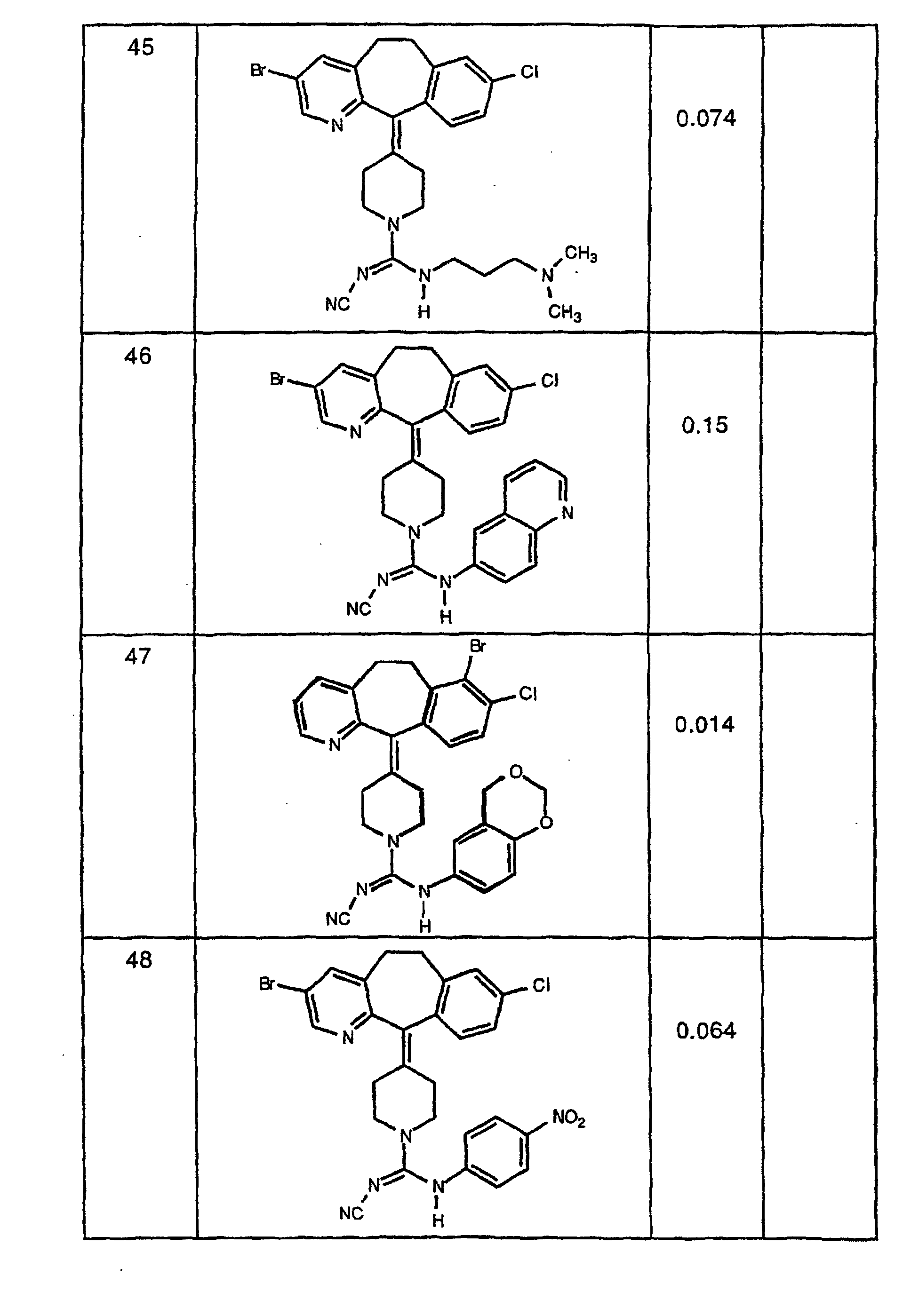

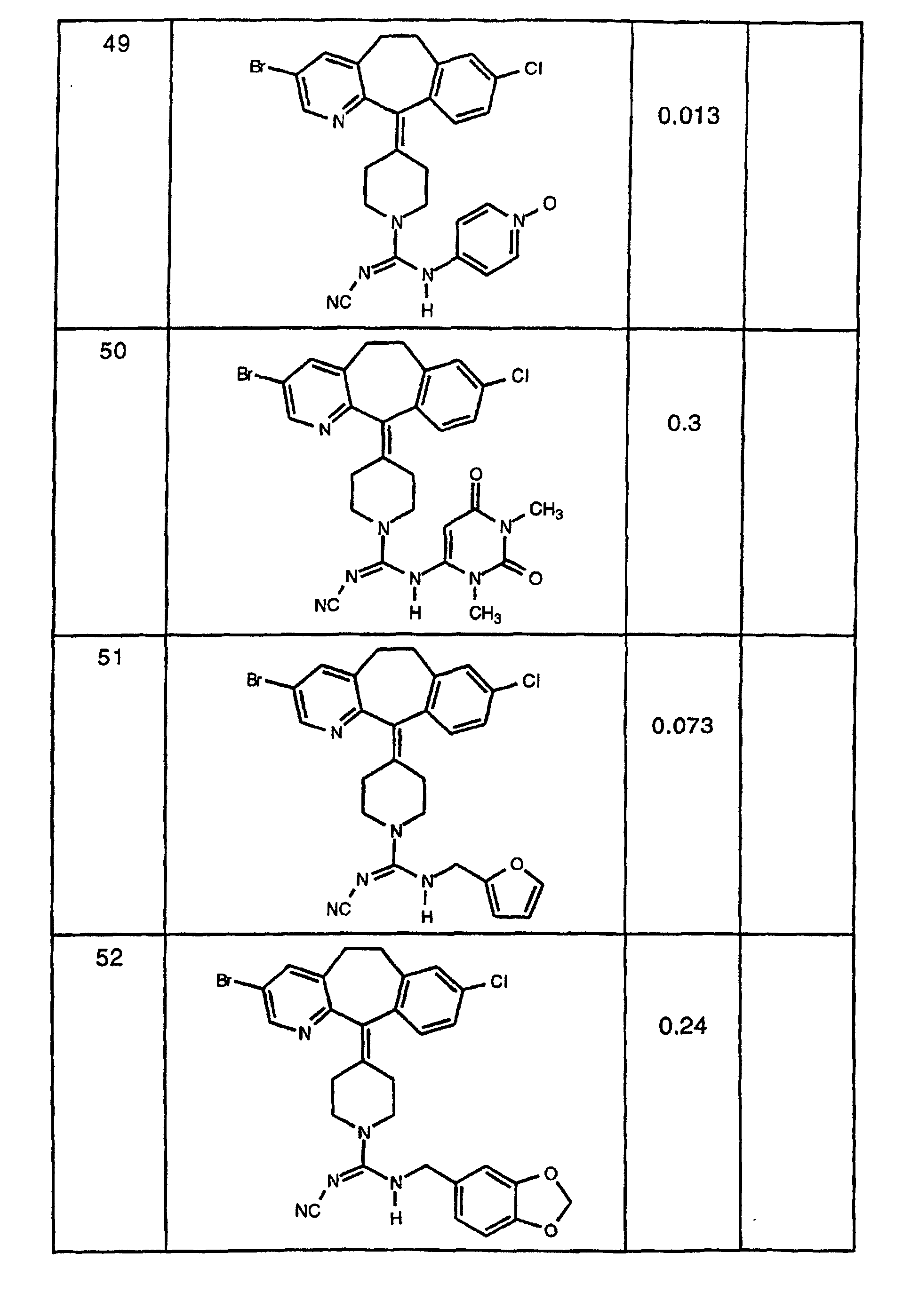

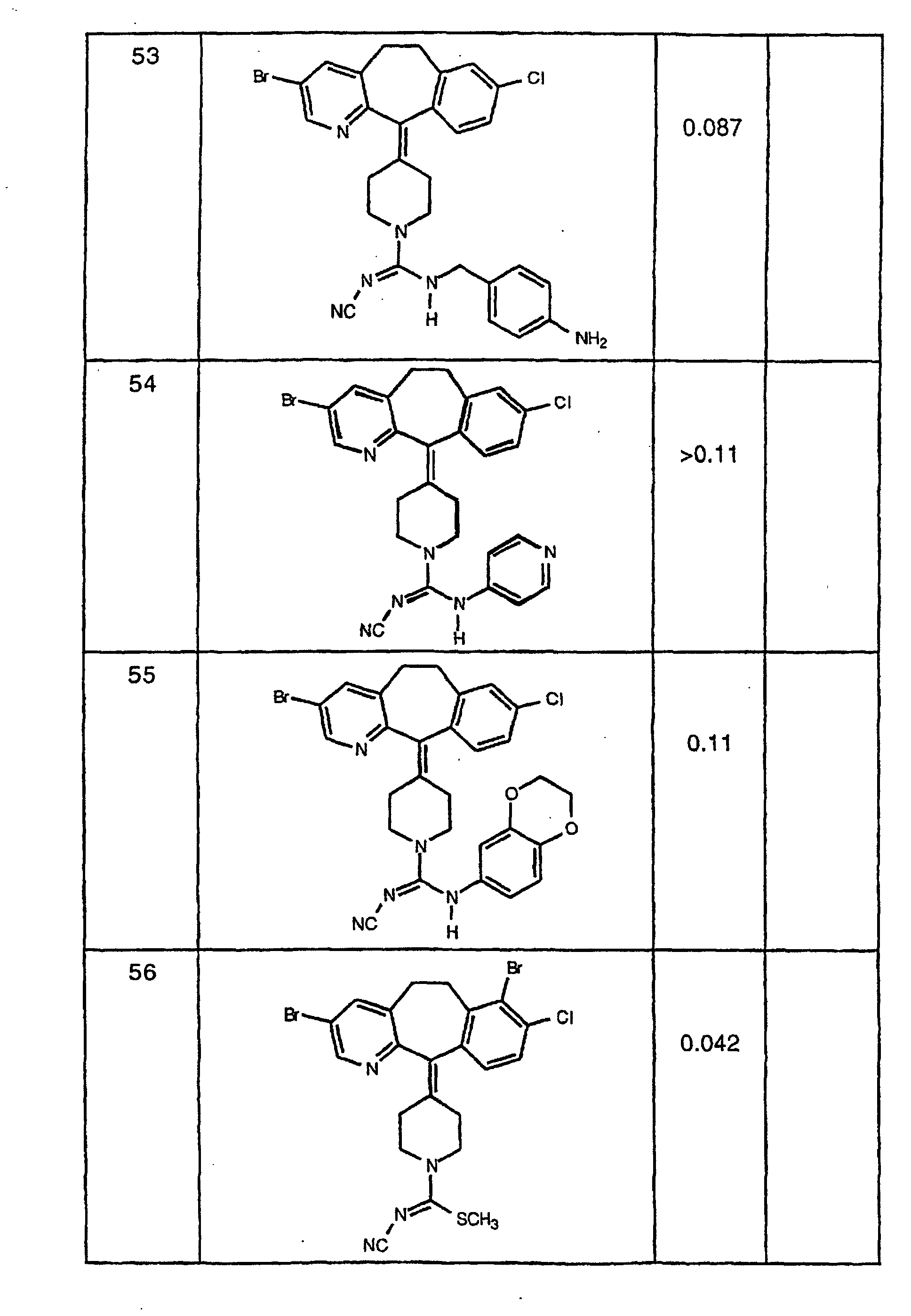

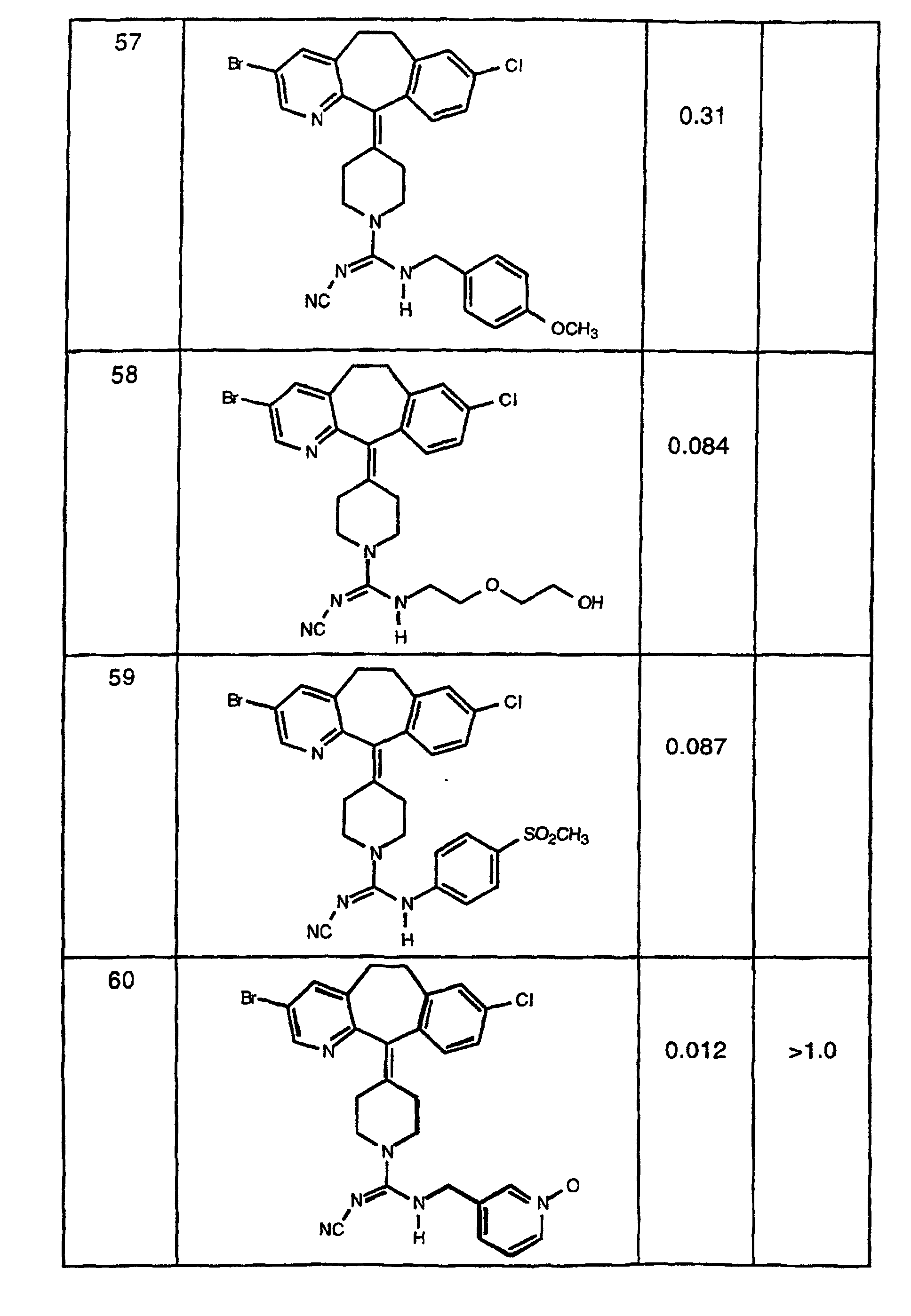

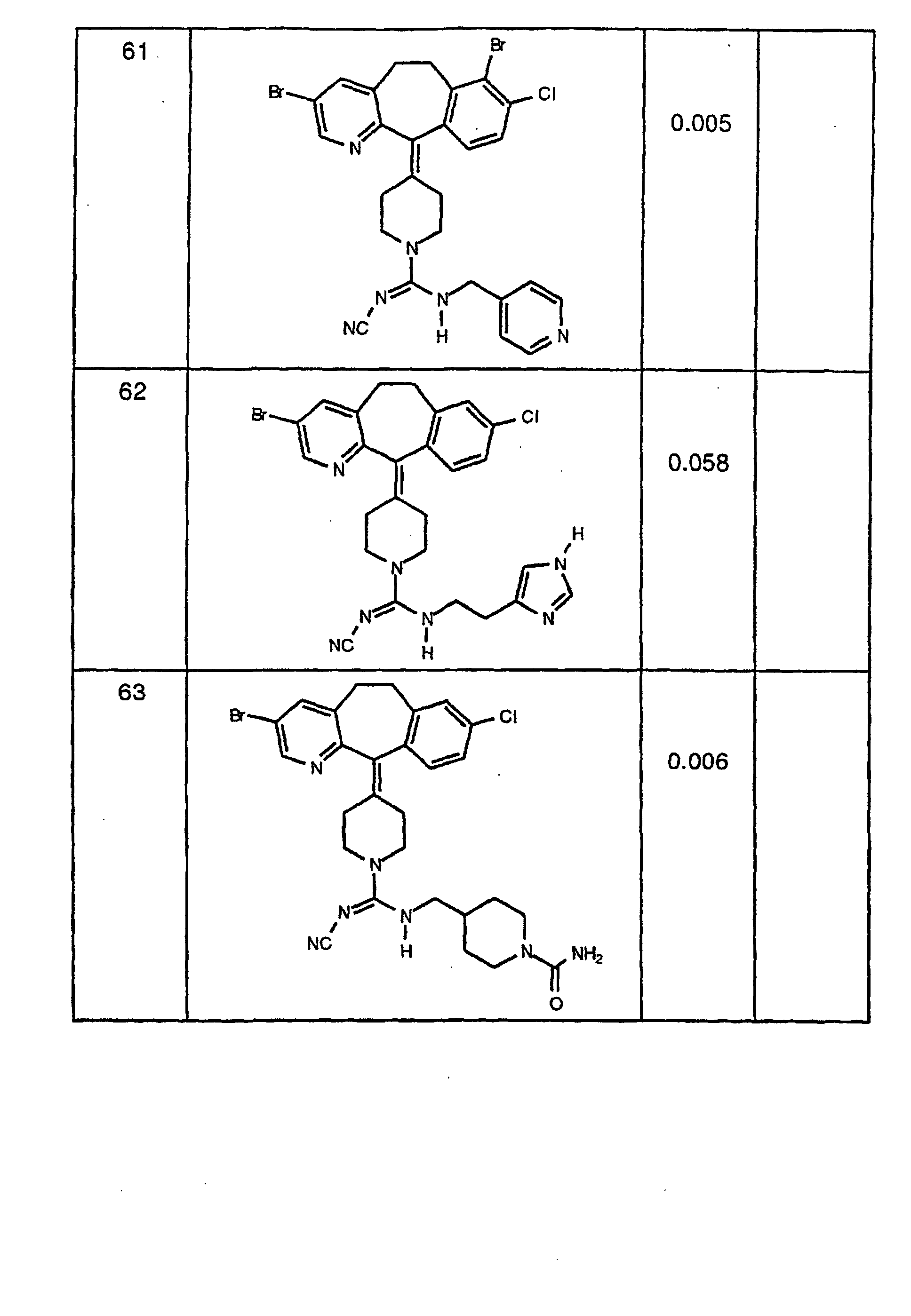

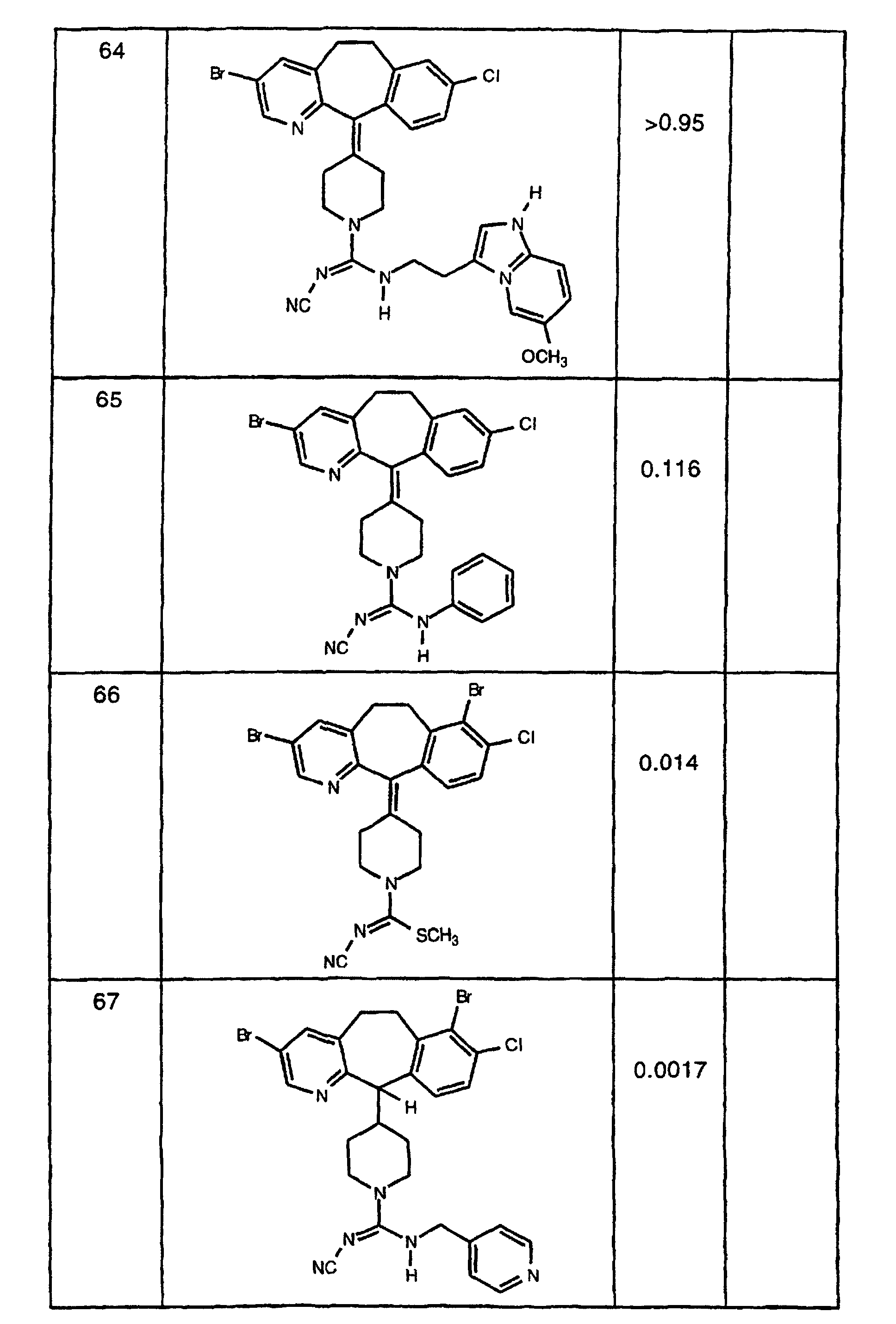

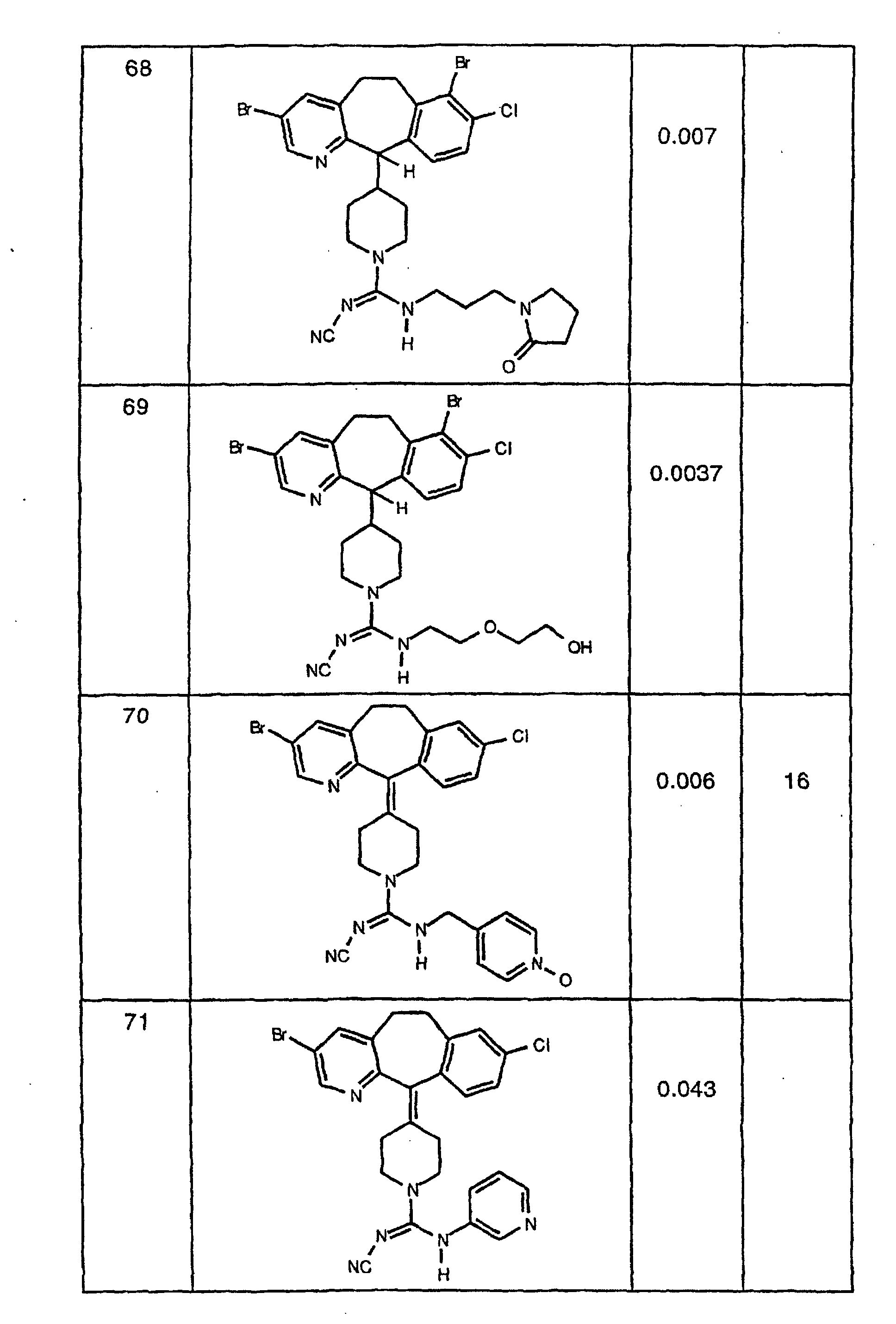

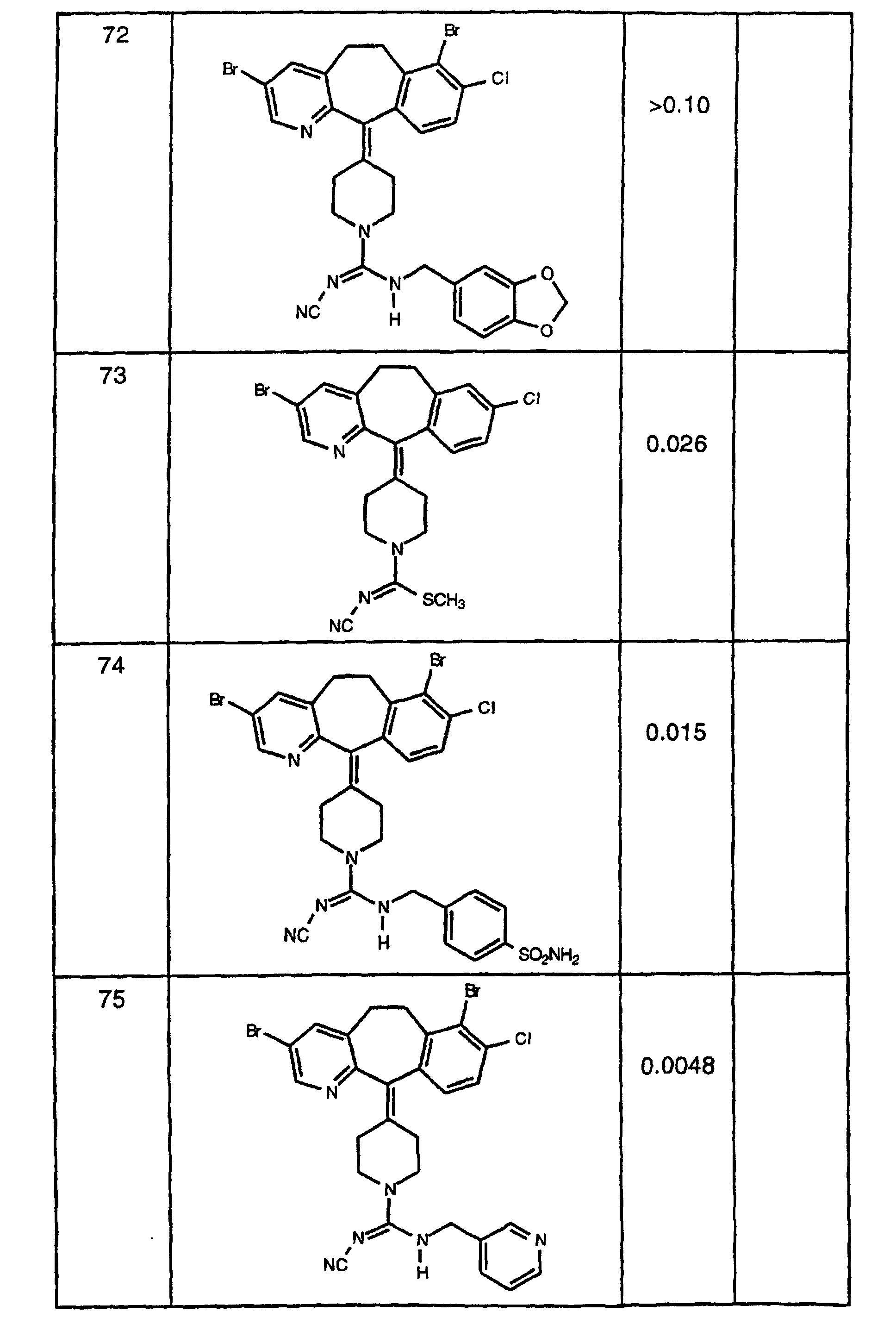

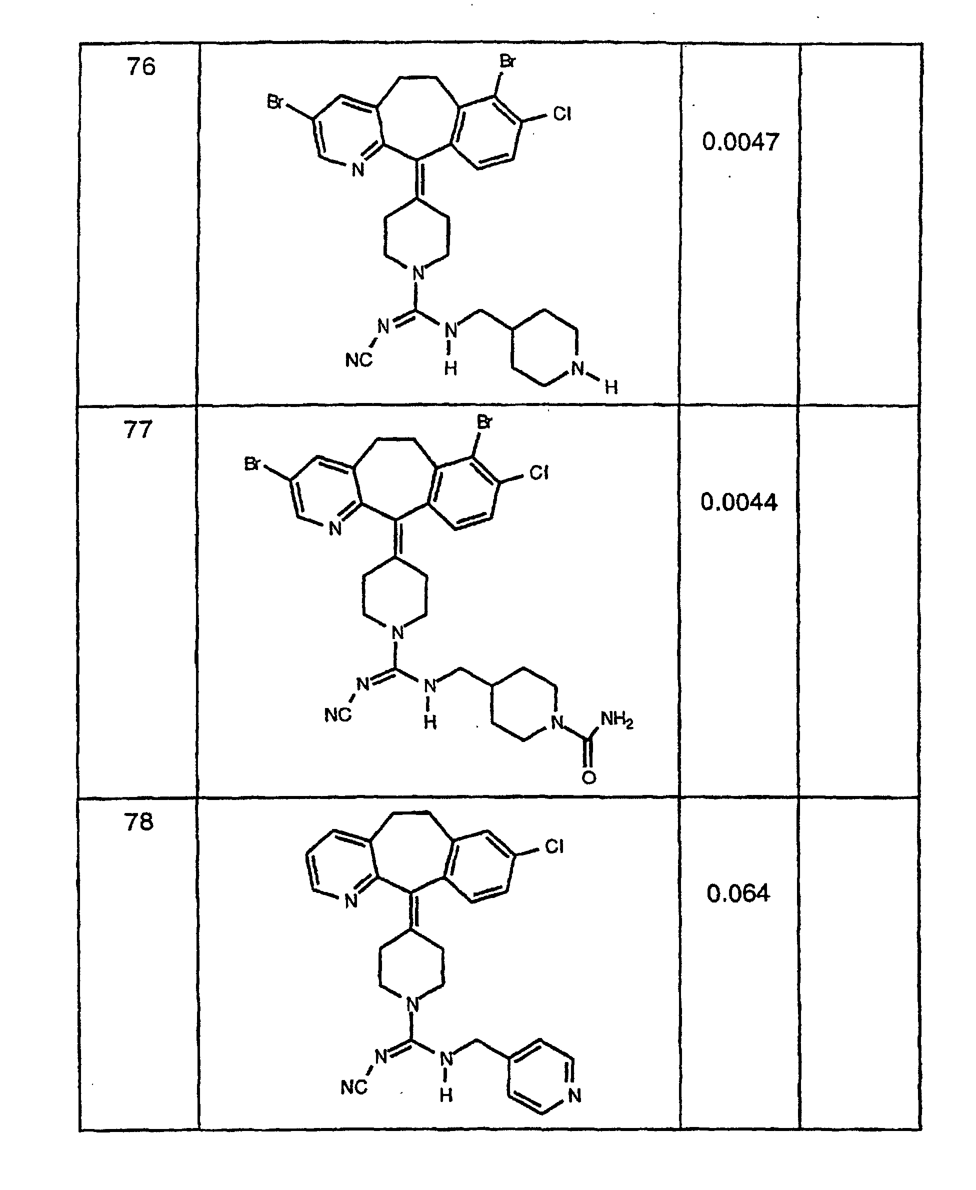

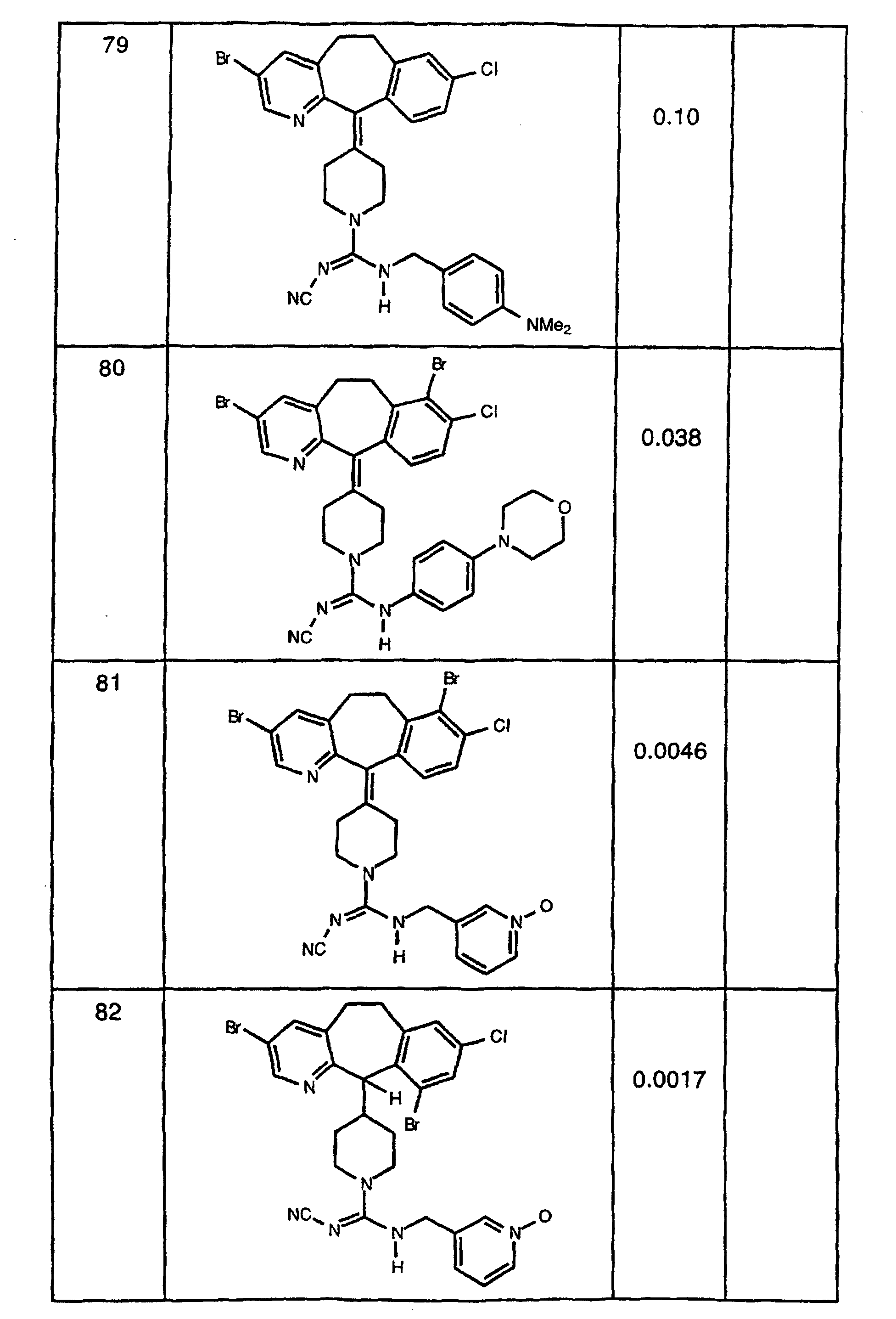

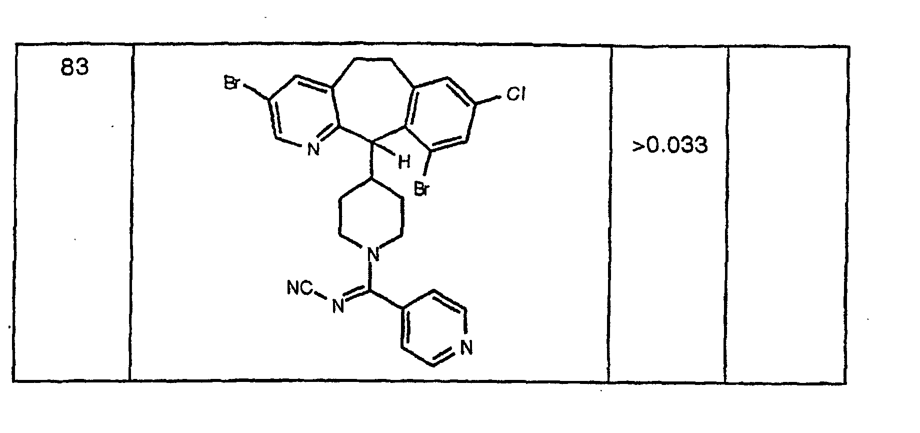

### PREPARATION OF STARTING MATERIALS

Starting materials useful in preparing the compounds of the present invention are exemplified by the following preparative examples, which should not be construed to limit the scope of the disclosure. The tricylic compounds used starting materials, such as compound (2.0), inorganic and organic bases, N-cyanoimidates and alcohols can be prepared using known methods in the art, such as taught in U.S. Patents 5,089,496; 5,151,423; 4,454,143; 4,355,036; PCT /US94/11390 (WO95/10514); PCT/US94/11391 (WO 95/10515); PCT/US94/11392 (WO95/10516); Stanley R. Sandler and Wolf Karo, Organic Functional Group Preparations, 2nd Edition, Academic Press, Inc., San Diego, California, Vol. 1-3, (1983), and in J. March, Advanced Organic Chemistry, Reactions & Mechanisms, and Structure, 3rd Edition, John Wiley & Sons, New York, 1346 pp. (1985). Alternative mechanistic pathways and analogous structures within the scope of the invention may be apparent to those skilled in the art.

### PREPARATIVE EXAMPLE 7

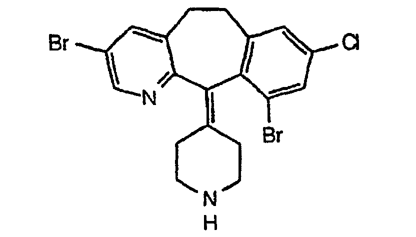

### Step A:

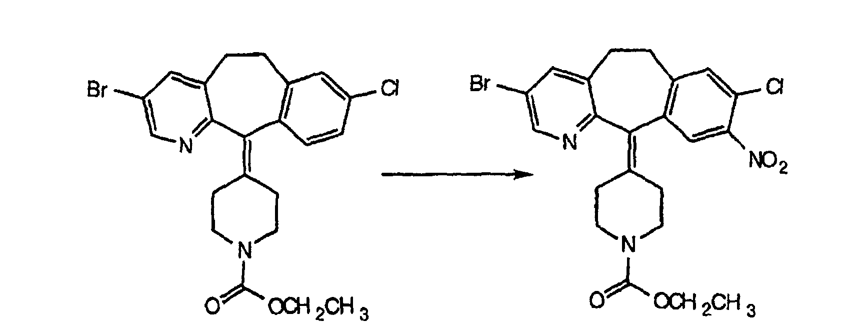

Combine 15 g (38.5 mmol) of 4-(8-chloro-3-bromo-5,6-dihydro-11 H-benzo[5,6]cyclohepta[1,2-b]pyridin-11-ylidene)-1-piperidine-1-carboxylic acid ethyl ester and 150 mL of concentrated H₂SO₄ at -5°C, then add 3.89 g (38.5 mmol) of KNO₃ and stir for 4 hours. Pour the mixture into 3 L of ice and basify with 50% NaOH (aqueous). Extract with CH₂Cl₂, dry over MgSO₄, then filter and concentrate *in vacuo* to a residue. Recrystallize the residue from acetone to give 6.69 g of the product.

### Step B:

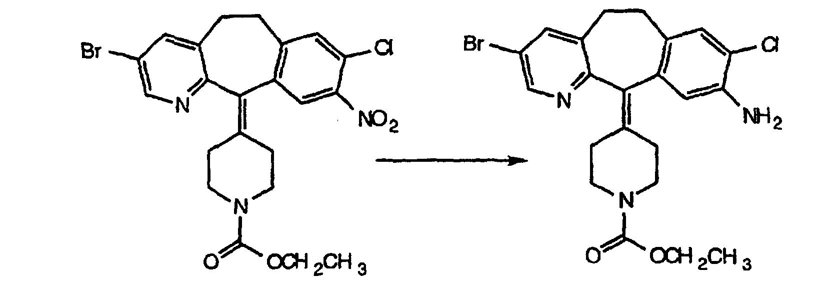

Combine 6.69 g (13.1 mmol) of the product of Step A and 100 mL of 85% EtOH/water, then add 0.66 g (5.9 mmol) of CaCl₂ and 6.56 g (117.9 mmol) of Fe and heat the mixture at reflux overnight. Filter the hot reaction mixture through celite® and rinse the filter cake with hot EtOH. Concentrate the filtrate *in vacuo* to give 7.72 g of the product.

### Step C:

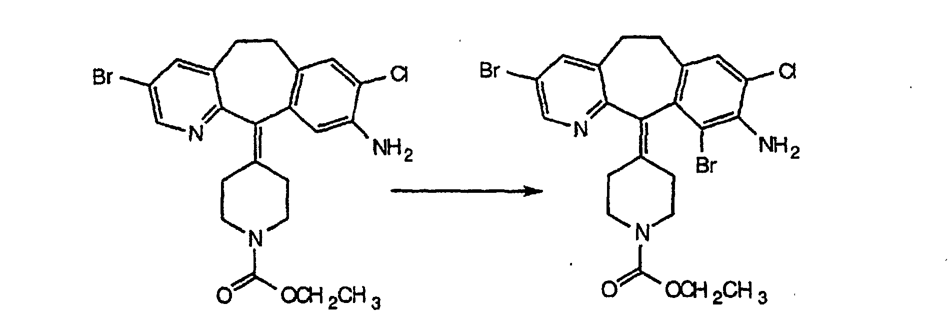

Combine 7.70 g of the product of Step B and 35 mL of HOAc, then add 45 mL of a solution of Br₂ in HOAc and stir the mixture at room temperature overnight. Add 300 mL of 1 N NaOH (aqueous) , then 75 mL of 50% NaOH (aqueous) and extract with EtOAc. Dry the extract over MgSO and concentrate *in vacuo* to a residue. Chromatograph the residue (silica gel, 20%-30% EtOAc/hexane) to give 3.47 g of the product (along with another 1.28 g of partially purified product).

### Step D:

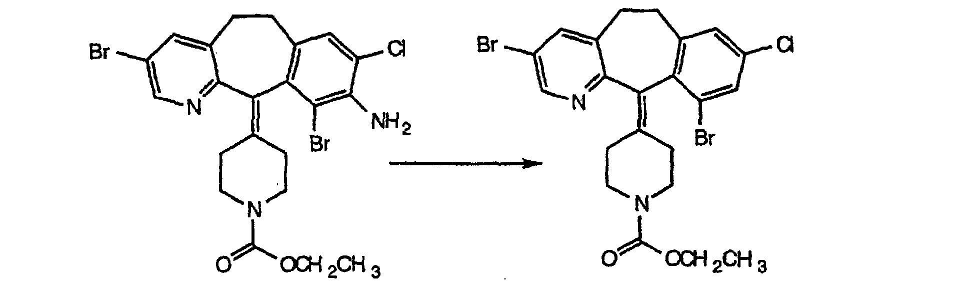

Combine 0.557 g (5.4 mmol) of t-butylnitrite and 3 mL of DMF, and heat the mixtre at to 60°-70°C. Slowly add (dropwise) a mixture of 2.00 g (3.6 mmol) of the product of Step C and 4 mL of DMF, then cool the mixture to room temperature. Add another 0.64 mL of t-butylnitrite at 40°C and reheat the mixture to 60°-70°C for 0.5 hrs. Cool to room temperature and pour the mixture into 150 mL of water. Extract with CH₂Cl₂, dry the extract over MgSO₄ and concentrate *in vacuo* to a residue. Chromatograph the residue (silica gel, 10%-20% EtOAc/hexane) to give 0.74 g of the product.

### Step E:

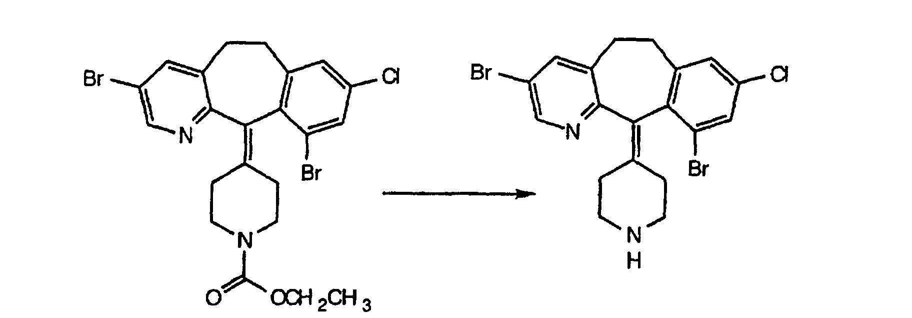

Combine 0.70 g (1.4 mmol) of the product of Step D and 8 mL of concentrated HCl (aqueous) and heat the mixture at reflux overnight. Add 30 mL of 1 N NaOH (aqueous), then 5 mL of 50% NaOH (aqueous) and extract with CH₂Cl₂. Dry the extract over MgSO₄ and concentrate *in vacuo* to give 0.59 g of the title compound.

### PREPARATIVE EXAMPLE 8

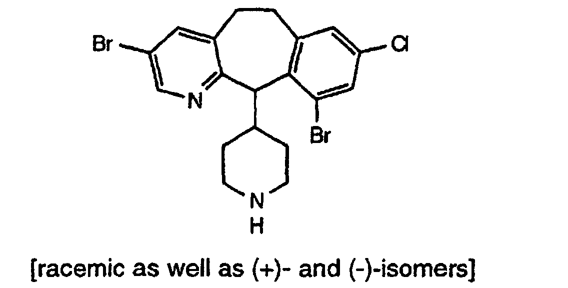

### Step A:

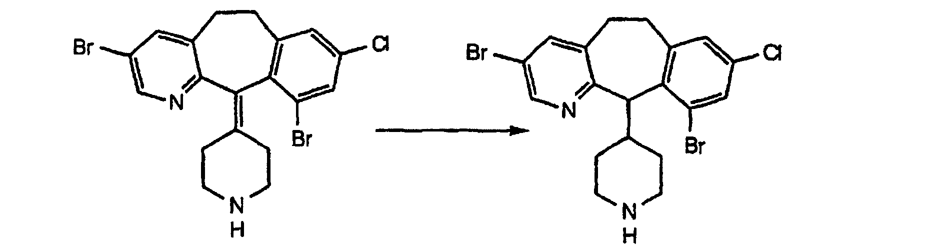

Prepare a solution of 8.1 g of the title compound from Preparative Example 7 in toluene and add 17.3 mL of a 1M solution of DIBAL in toluene. Heat the mixture at reflux and slowly add (dropwise) another 21 mL of 1 M DIBAL/toluene solution over a period of 40 min. Cool the reaction mixture to about 0°C and add 700 mL of 1 M HCI (aqueous). Separate and discard the organic phase. Wash the aqueous phase with CH₂Cl₂, discard the extract, then basify the aqueous phase by adding 50% NaOH (aqueous). Extract with CH₂Cl₂, dry the extract over MgSO₄ and concentrate *in vacuo* to give 7.30 g of the title compound, which is a racemic mixture of enantiomers.

### Step B - Separation of Enantiomers:

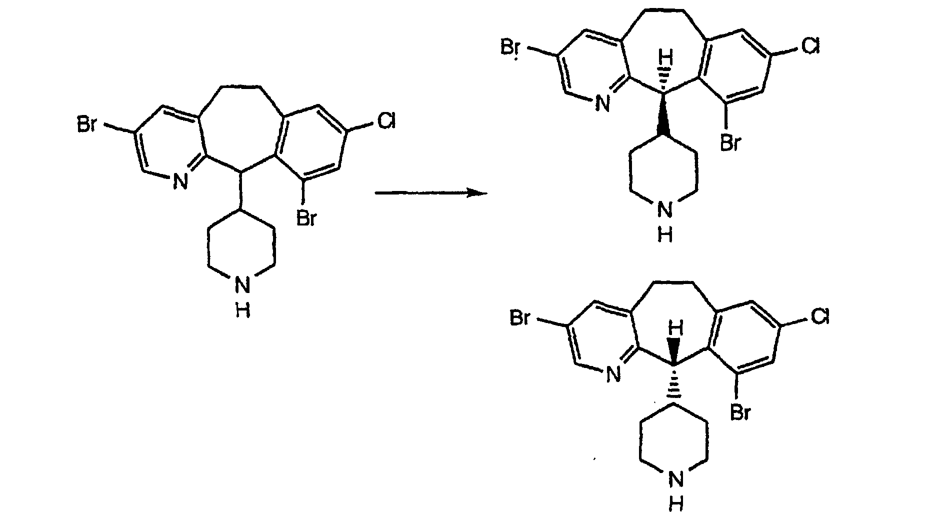

The racemic title compound of Step A is separated by preparative chiral chromatography (Chiralpack AD, 5 cm X 50 cm column, using 20% iPrOH/hexane + 0.2% diethylamine), to give the (+)-isomer and the (-)-isomer of the title compound.

### PREPARATIVE EXAMPLE 48

### Step A:

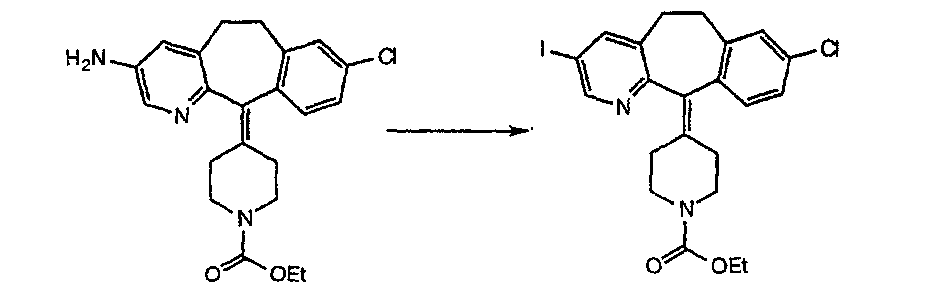

Combine 6 g (15.11 mmol) of the title compound of WO 95/10516's Preparative Example 47B, and benzene, and add 2.3 g (9.06 mmol) of iodine. Heat the mixture at reflux for 3 hours, cool, then dilute with 50 mL of CH₂Cl₂. Wash the organic phase with 5% NaHSO₃(aqeuous) (3 x 80 mL), then with 1M NaOH (aqueous) (2x 80 mL), and dry over MgSO₄. Concentrate to a residue chromatograph (silica gel, 30% EtOAc/hexanes), to give 3.2 g (42% yield) of the product iodo compound. Mass Spec.: MH+ = 509

### Step B:

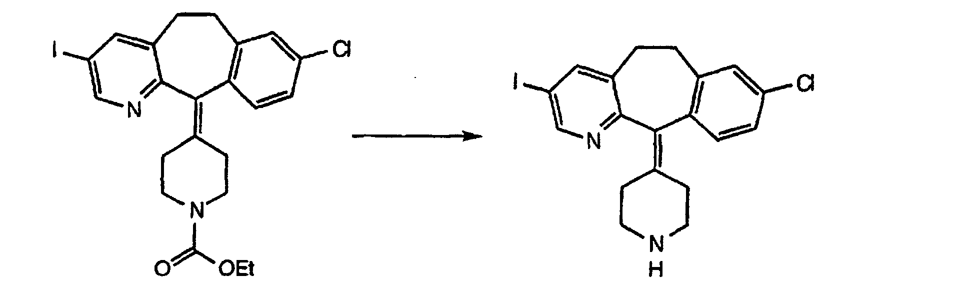

The product of Step A is hydrolyzed via substantially the same procedure as described in Example 358, Step A, of WO 95/10516, to give the iodoamine product in 89% yield.

### PREPARATIVE EXAMPLE 49

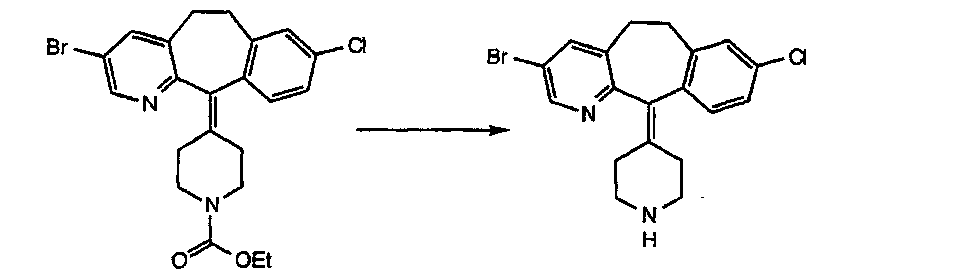

The product of Preparative Example 47, Step C, of WO 95/10516, (2.42 g) is hydrolyzed via substantially the same procedure as described in Example 358, Step A, of WO 95/10516, to give 1.39 g (69% yield) of the bromoamine product.

### PREPARATIVE EXAMPLE 51A

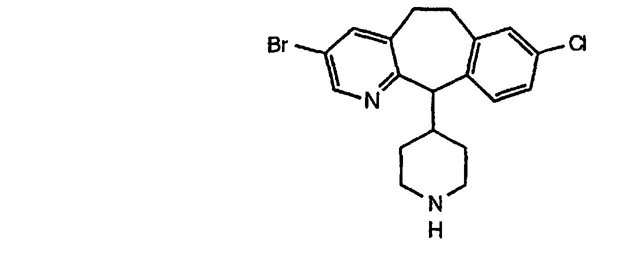

### Step A:

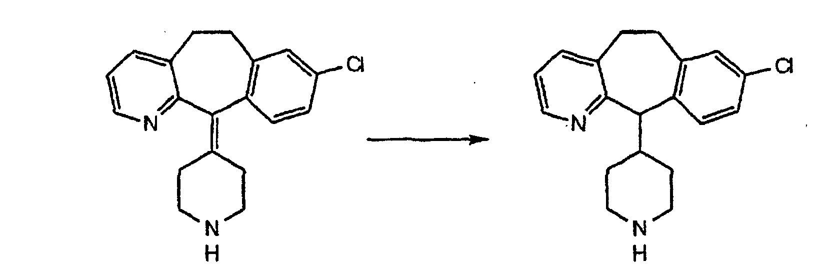

Combine 82.0 g (0.26 mole) of the product of Preparative Example 1, Step G, of WO 95/10516, and 1 L of toluene, then add 20.06 g (0.53 mole) of LiAlH₄ and heat the reaction mixture at reflux overnight. Cool the mixture to room temperature and add ∼1 L of Et₂O, followed by dropwise addition of saturated Na₂SO₄ (aqueous) until a precipitate forms. Filter and stir the filtrate over MgSO₄ for 30 minutes, then concentrate *in vacuo* to give the product compound in 83% yield. Mass Spec.: MH⁺ = 313.

### Step B:

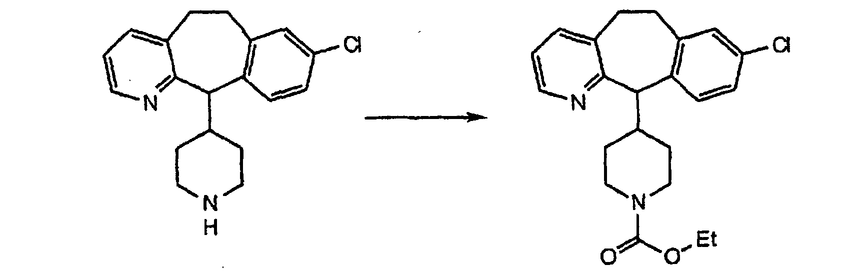

Combine 24.32 g (74.9 mmol) of the Product from Step A, 500 mL of toluene, 83 mL of Et₃N and 65.9 mL of ethyl chloroformate and heat the mixture at reflux overnight. Cool to 25°C, pour into 200 mL of water and extract with EtOAc. Dry the extract over MgSO₄, concentrate in *vacuo* to a residue and chromatograph (silica gel, 50% EtOAc/hexane) to give 15 g of the product compound. Mass Spec.: MH⁺ = 385.

### Step C:

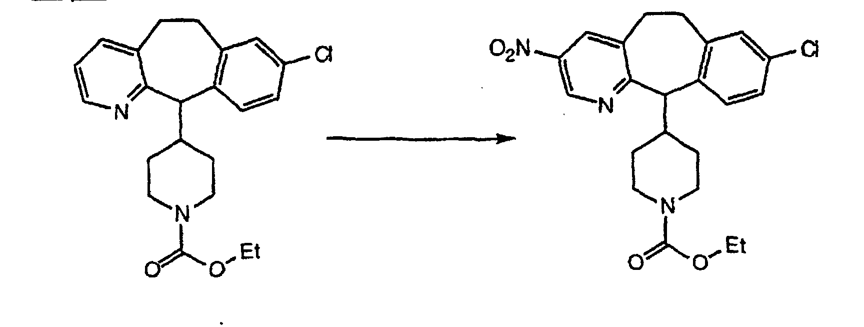

Dissolve 3.2 g (10.51 mmol) of tetra-n-butylammonium nitrate in 25 mL of CH₂Cl₂ and add 2.2 g (10.51 mmol, 1.5 mL) of TFAA. Cool to 0°C and add the mixture (via cannula) to a solution of 3.68 g (9.56 mmol) of the product of Step B in 50 mL of CH₂Cl₂ at 0°C, then stir at 0°C for 3 hours. Allow the mixture to warm to 25°C while stirring overnight, then extract with saturated NaHCO₃ (aqueous) and dry over MgSO₄. Concentrate *in vacuo* to a residue and chromatograph (silica gel, 30% EtOAc/hexane) to give 1.2 g of the product compound. Mass Spec.: MH⁺ = 430

### Step D:

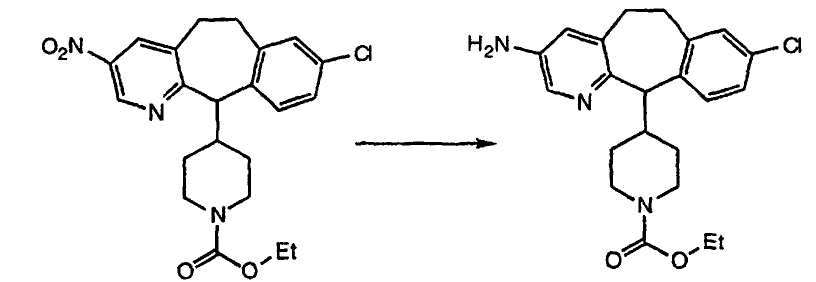

Combine 2.0 g (4.7 mmol) of the Product of Step C and 150 mL of 85% EtOH (aqueous), add 2.4 g (42 mmol) of Fe filings and 0.24 g (2.1 mmol) of CaCl₂, and heat at reflux for 16 hours. Filter the hot mixture through a bed of celite®, wash the celite® with hot EtOH. Concentrate the filtrate *in vacuo* to give a 100% yield of the product compound. Mass Spec.: MH⁺ = 400.

### Step E:

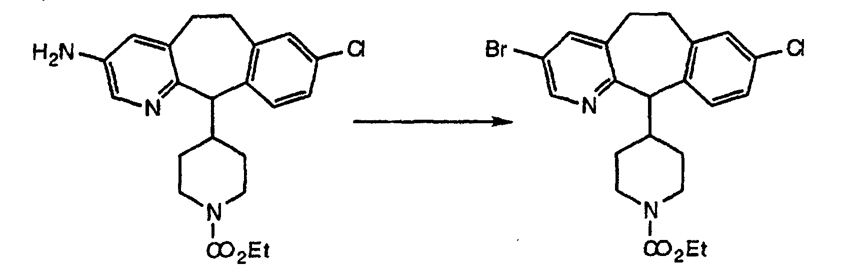

Combine 2.0 g (5.2 mmol) of the Product of Step D and 20 mL of 48% HBr, cool the mixture to -5°C. Stir the mixture at -5°C for 15 minutes and slowly add a solution of 1.07 g (15.5 mmol) of NaNO₂ in 10 mL of water. Stir for 45 minutes, then quench with 50% NaOH (aqueous) to pH ∼10. Extract with EtOAc, dry the combined extracts over MgSO₄ and concentrate *in vacuo* to give the product compound. Mass Spec.: MH⁺= 465

### Step F:

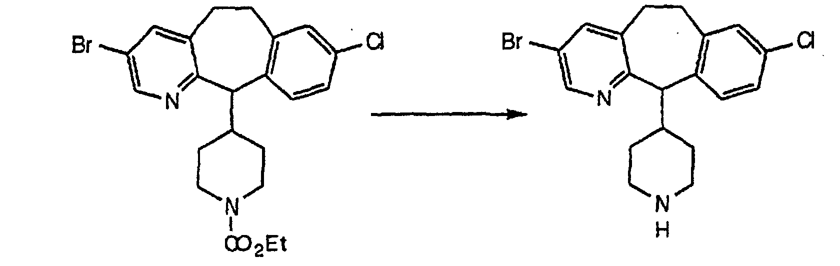

Hydroyze 4.0 g of the Product of Step E via substantially the same process as described for Example 358, Step A, of WO 95/10516, to give 1.39 g of the product compound. Mass Spec.: MH⁺ = 392

### PREPARATIVE EXAMPLE 53

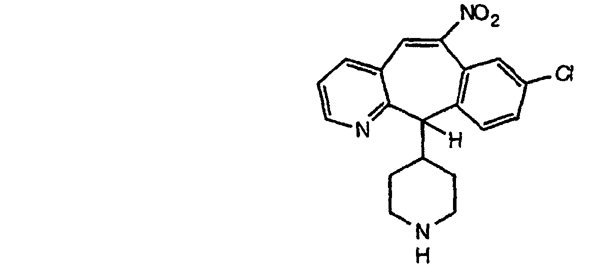

### Step A:

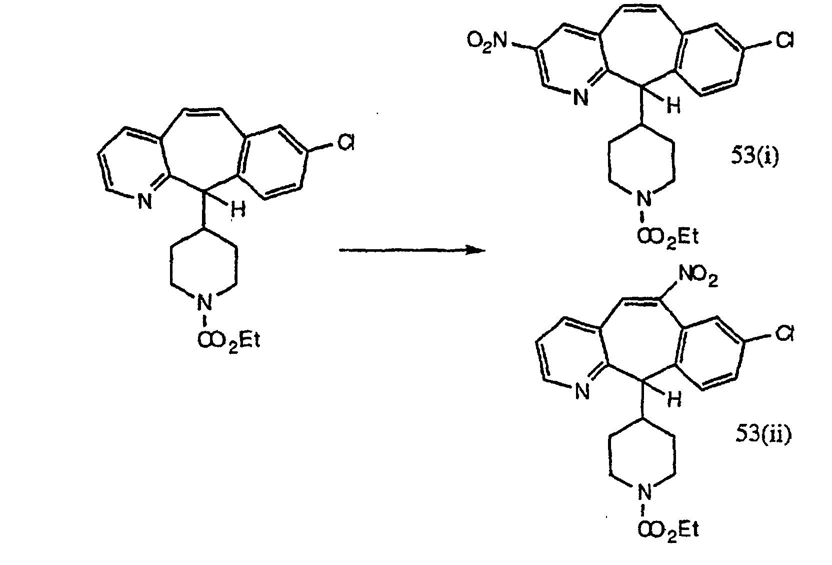

Combine 14.95 g (39 mmol) of the Product of Preparative Example 34A, of WO 95/10516, and 150 mL of CH₂Cl₂, then add 13.07 g (42.9 mmol) of (nBu)₄NNO₃ and cool the mixture to 0°C. Slowly add (dropwise) a solution of 6.09 mL (42.9 mmol) of TFAA in 20 mL of CH₂Cl₂ over 1.5 hours. Keep the mixture at 0°C overnight, then wash successively with saturated NaHCO₃ (aqueous), water and brine. Dry the organic solution over Na₂SO₄, concentrate *in vacuo* to a residue and chromatograph the residue (silica gel, EtOAc/hexane gradient) to give 4.32 g and 1.90 g of the two product compounds 53(i) and 53(ii), respectively.

Mass Spec.(53(i)); MH⁺ = 428.2; Mass Spec. (53(ii)): MH⁺ = 428.3

### Step B:

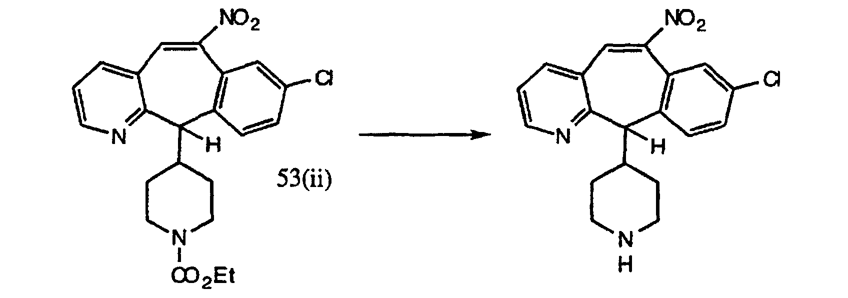

The compound 53(ii) from Step A (0.20 g) is hydrolyzed via substantially the same procedure as described for Example 358, Step A, of WO 95/10516 (published April 20, 1995), to give 0.16 g of the product compound.

Using the starting compound indicated and substantially the same procedure as described in Preparative Example 53, Step B, the compounds in Table 1 are prepared:

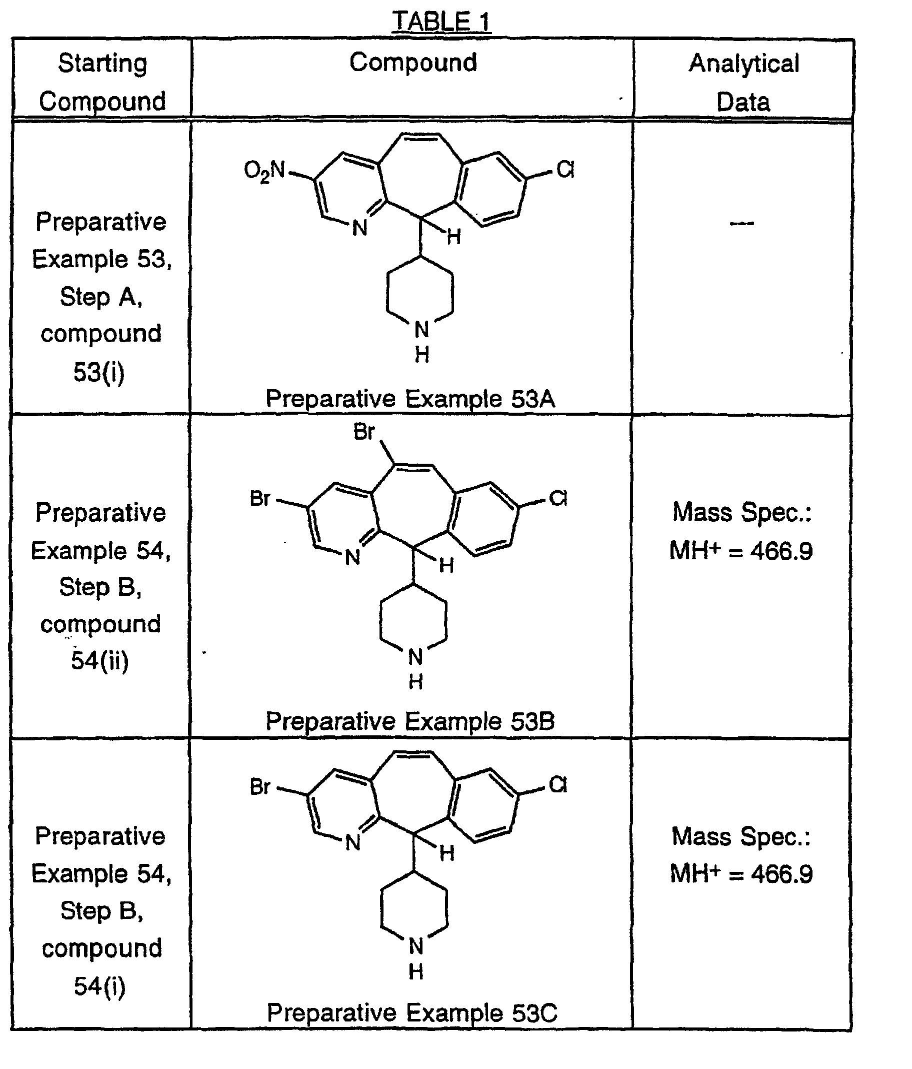

### PREPARATIVE EXAMPLE 54

### Step A:

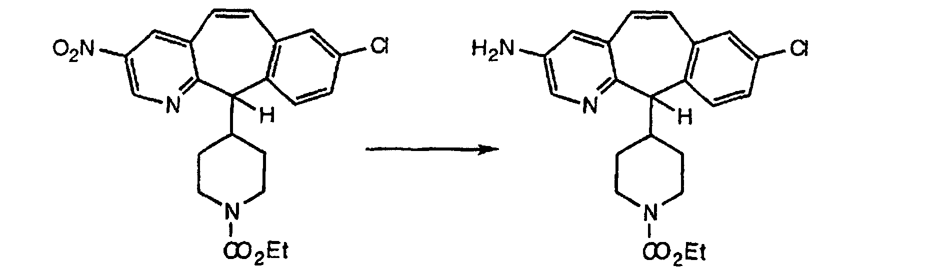

Combine 22.0 g (51.4 mmol) of the product 53(i) from Preparation 53, Step A, 150 mL of 85% EtOH (aqueous), 25.85 g (0.463 mole) of Fe powder and 2.42 g (21.8 mmol) of CaCl₂, and heat at reflux overnight. Add 12.4 g (0.222 mole) of Fe powder and 1.2 g (10.8 mmol) of CaCl₂ and heat at reflux for 2 hours. Add another 12.4 g (0.222 mole) of Fe powder and 1.2 g (10.8 mmol) of CaCl₂ and heat at reflux for 2 hours more. Filter the hot mixture through celite®, wash the celite® with 50 mL of hot EtOH and concentrate the filtrate *in vacuo* to a residue. Add 100 mL of anhydrous EtOH, concentrate to a residue and chromatograph the residue (silica gel, MeOH/CH₂Cl₂ gradient) to give 16.47 g of the product compound.

### Step B:

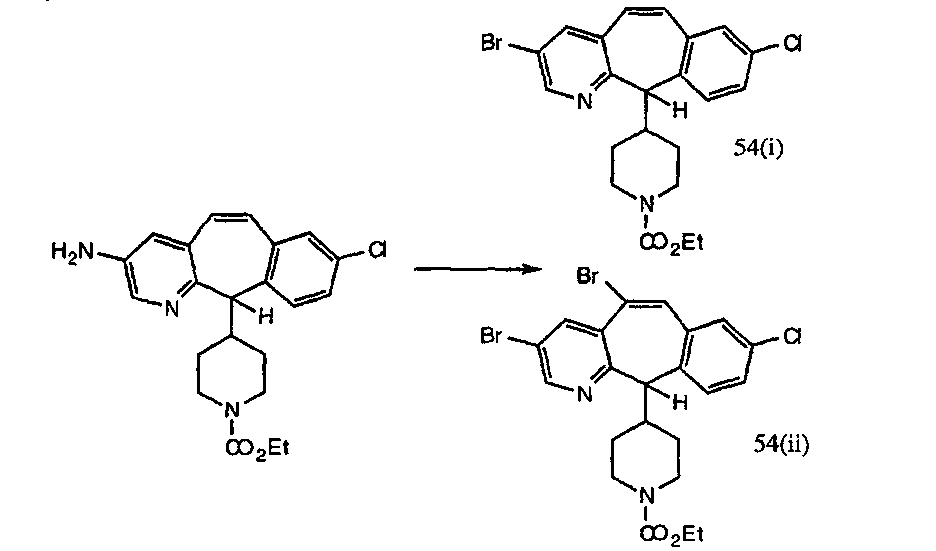

Combine 16.47 g (41.4 mmol) of the product compound from Preparative Example 54, Step A, and 150 mL of 48% HBr (aqueous) and cool to -3°C. Slowly add (dropwise) 18 mL of bromine, then slowly add (dropwise) a solution of 8.55 g (0.124 mole) of NaNO_{2β} in 85 mL of water. Stir for 45 minutes at -3° to 0°C, then adjust to pH = 10 by adding 50% NaOH (aqueous). Extract with EtOAc, wash the extracts with brine and dry the extracts over Na₂SO₄. Concentrate to a residue and chromatograph (silica gel, EtOAc/hexane gradient) to give 10.6 g and 3.28 g of the two product compounds 54(i) and 54(ii), respectively.

Mass Spec. (54(i)): MH⁺ = 461.2; Mass Spec. (54(ii)): MH⁺ = 539

### PREPARATIVE EXAMPLE 55

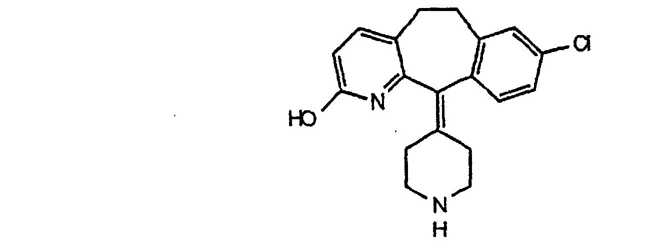

The title compound is known and is prepared by the procedure described in Bioorg. & Med. Chem. Lett., 3, (No. 6) 1073-1078 (1993).

### PREPARATIVE EXAMPLE 56

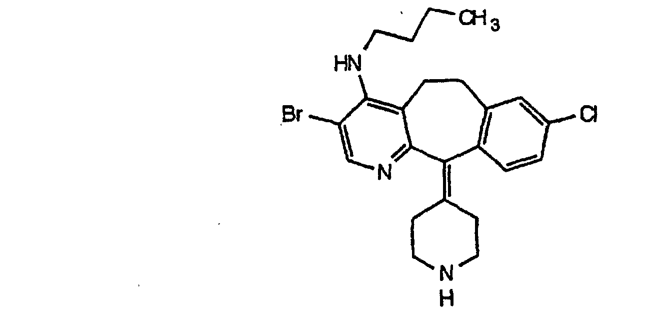

### Step A:

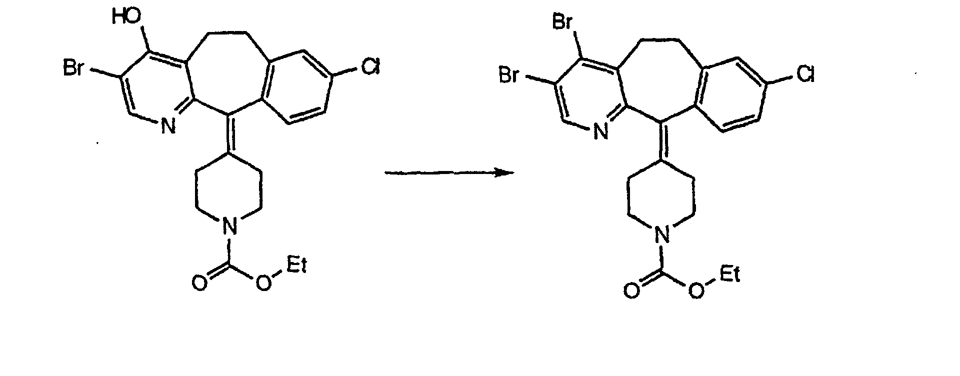

Combine 2.04 g of the product of Preparative Example 44, of WO 95/10516 (published April 20, 1995), 1.3 mL of PBr₃, 1.0 mL of Et₃N and 20 mL of CH₂Br₂, and heat the mixture at reflux overnight. Cool the mixture, dilute with CH₂Cl₂ and wash with 1 N NaOH (aqueous). Dry over MgSO₄ and concentrate *in vacuo* to give 1.22 g (53% yield) of the product compound. Mass Spec.: MH⁺ = 541

### Step B:

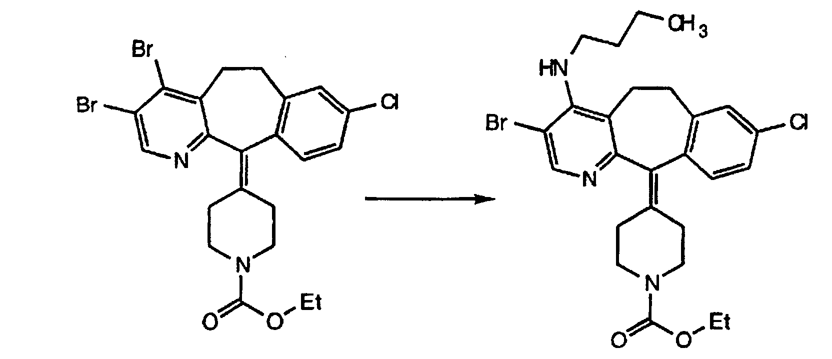

Combine 0.3 g of the product compound from Preparative Example 56, Step A, and 8 mL of n-butylamine and stir at 120°C in a sealed tube for 48 hours. Concentrate *in vacuo* to a residue and purify by preparative plate chromatography (silica gel, 1.5-2.5% MeOH/CH₂Cl₂) to give 80 mg (27%) yield of the product compound. Mass Spec.: MH⁺ = 534

### Step C:

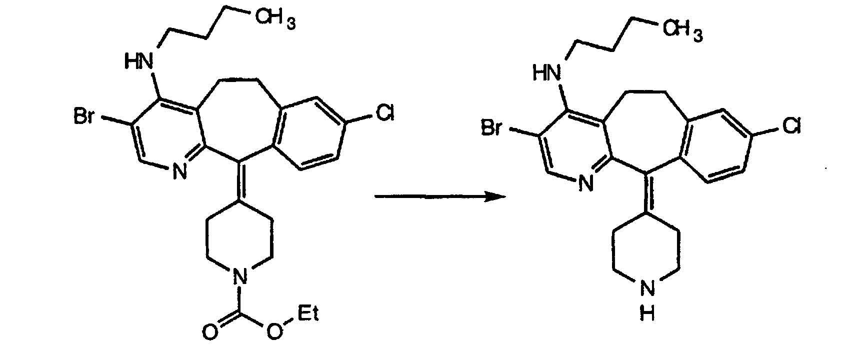

Combine 66 mg of the product compound from Preparative Example 56, Step B, 4 mL of anhydrous EtOH, and 15 mL of concentrated HCI stir at reflux for 60 hours. Cool the reaction mixture to about 0°C and basify by adding KOH. Extract with CH₂Cl₂, dry the extract over MgSO₄, and concentrate *in vacuo* to give 46 mg (81% yield) of the product compound. Mass Spec.: MH⁺ = 462

### PREPARATIVE EXAMPLE 57

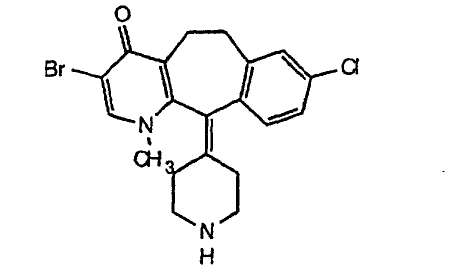

### Step A:

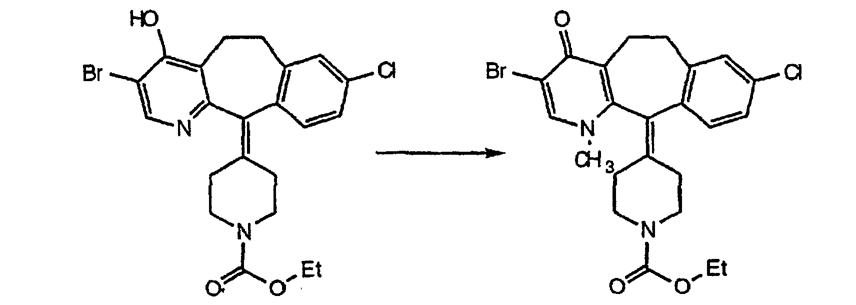

Combine 1.19 g of the product of Preparative Example 44, of WO 95/10516, 10 mL of anhydrous DMF, 0.2 g of NaH (60% in mineral oil) and 0.19 mL of methyl iodide, and stir at room temperature overnight. Concentrate *in vacuo* to a residue, dilute the residue with CH₂Cl₂, wash with saturated NaHCO₃ (aqueous), and dry over MgSO₄. Concentrate *in vacuo* to give 1.13 g (92% yield) of the product compound. Mass Spec.: MH⁺ = 493.

### Step B:

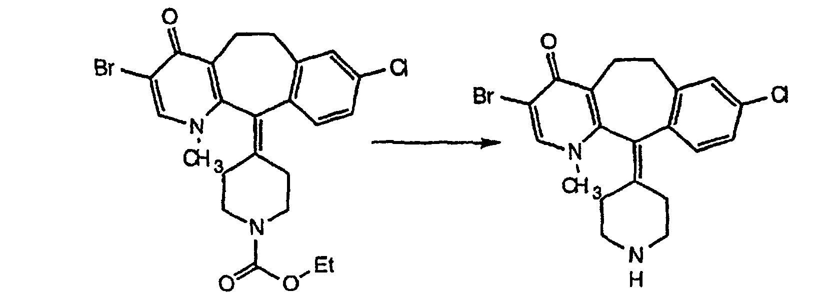

Hydrolyze 1.13 g of the product of Step A via substantially the same procedure as described for Preparative Example 56, Step C, to give 0.61 g (63% yield) of the product compound.

### PREPARATIVE EXAMPLE 58

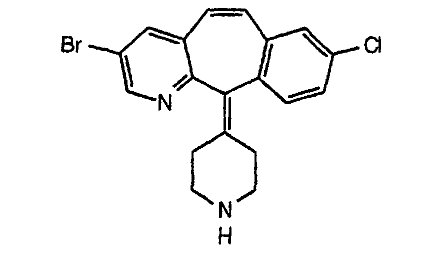

### Step A:

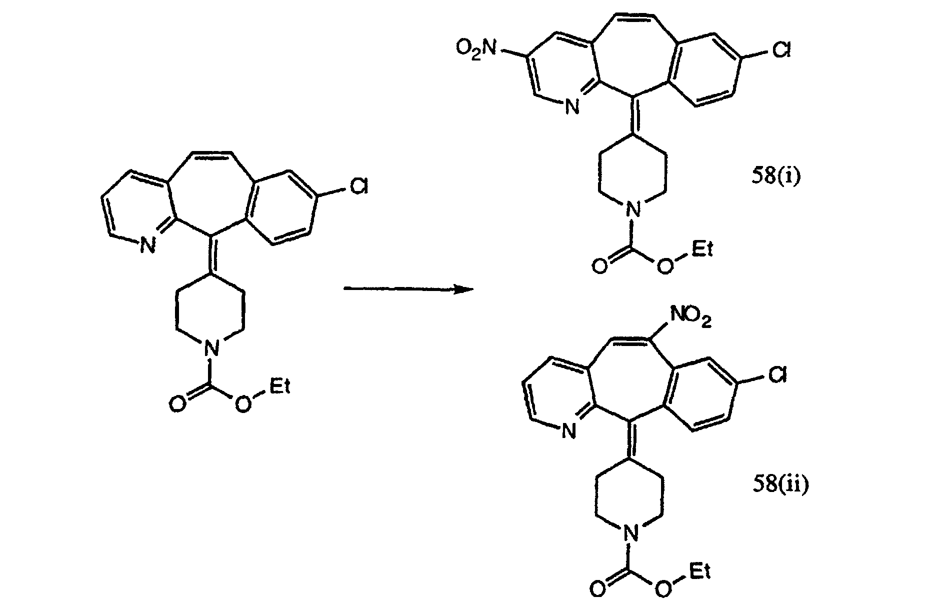

Combine 1.07 g (3.52 mmol) of tetrabutylammonium nitrate, 4 mL of anhydrous CH₂Cl₂ and 0.743 g (3.52 mmol) of TFAA, and add the resulting mixture to a solution of 1.22 g (3.20 mmol) of the title compound of Preparative Example 37, of WO 95/10516, in 8 mL of anhydrous CH₂Cl₂ at room temperature. Stir at room temperature overnight, then wash with 20 mL of saturated NaHCO₃ (aqueous) and 20 mL of brine, and dry over MgSO₄. Concentrate *in vacuo* and chromatograph the resulting residue (silica gel, EtOAc/hexane) to give 0.216 g of the product compound 58(i) and 0.27 g of the product compound 58(ii). Mass Spec. (58(i)): MH⁺ =426. m.p. (58(i)) 97.5° - 99.2°C.

### Step B:

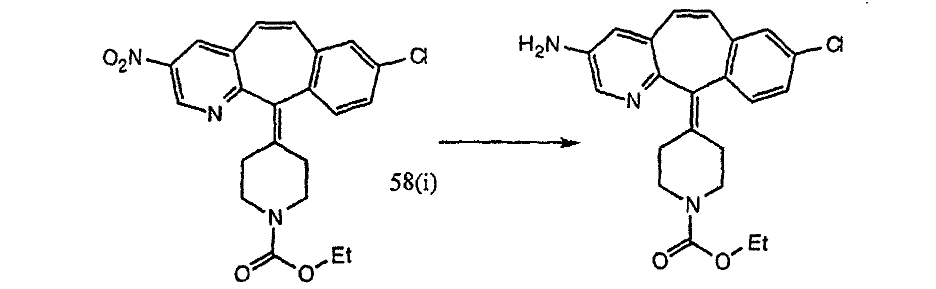

Reduce the product 58(i) from Step A via essentially the same procedure as described in Preparative Example 47, Step B, of WO 95/10516, to give the product compound. Mass Spec.: MH⁺ = 396

### Step C:

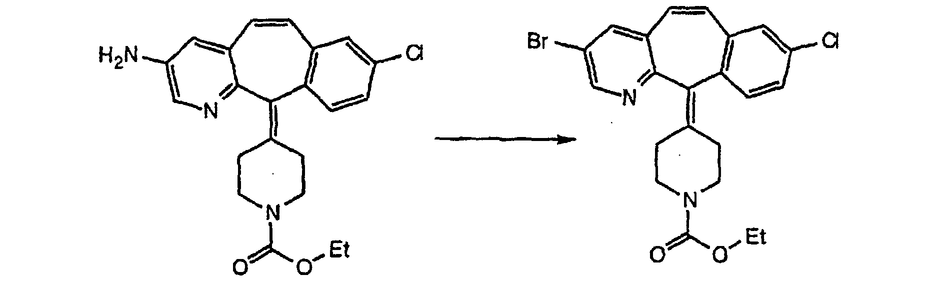

React the product from Step B with HBr and bromine via essentially the same procedure as described in Preparative Example 47, Step C, of WO 95/10516, to give the product compound. Mass Spec.: MH⁺ = 459

### Step D:

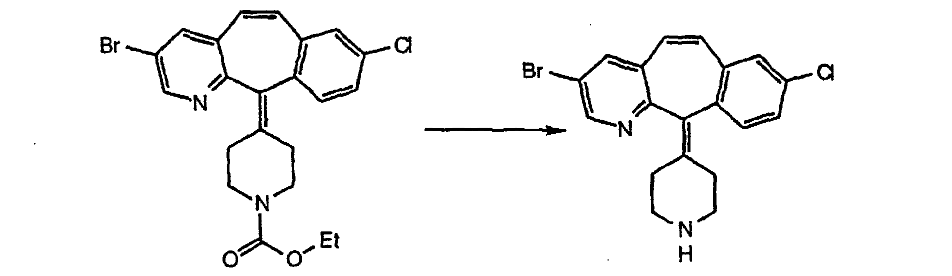

Hydrolyze 0.83 g of the product from Step C via essentially the same procedure as described in Preparative Example 56, Step C, to give 0.56 g of the product compound. Mass Spec.: MH⁺ = 387

### PREPARATIVE EXAMPLE 60

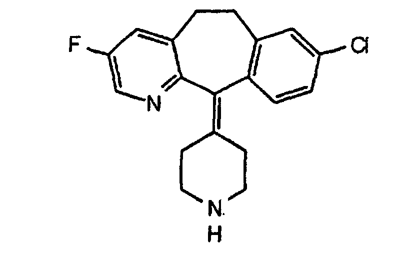

### Step A:

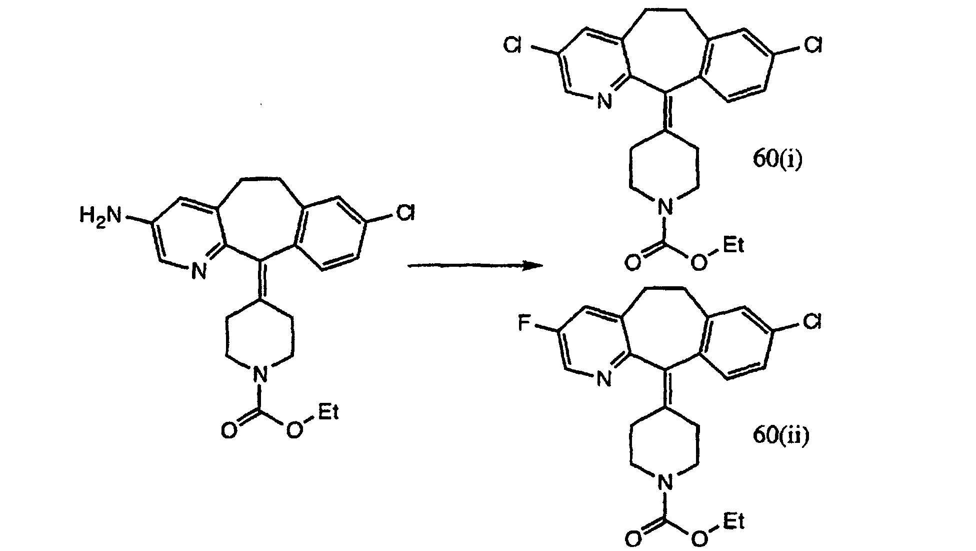

Combine 16.25 g (40.83 mmol) of the product of Preparative Example 47, Step B, of WO 95/10516, and a slurry of 7.14 g (61.11 mmol) of NOBF₄ in 100 mL of CH₂Cl₂ and stir the mixture for 3 hours. Add 100 mL of o-dichiorobenzene and heat for 5 hours, distilling the CH₂Cl₂ from the mixture. Concentrate *in vacuo* to a residue, add 200 mL of CH₂Cl₂ and wash with water (2 X 200 mL). Dry over MgSO₄, concentrate *in vacuo* to a residue, and chromatograph (silica gel, 20% EtOAc/hexane) to give 4.1 g of product compound 60(i) and 4.01 g of Product compound 60(ii). Mass Spec. (60 (i)): MH⁺ = 418. Mass Spec. (60 (ii)): MH⁺ = 401

### Step B:

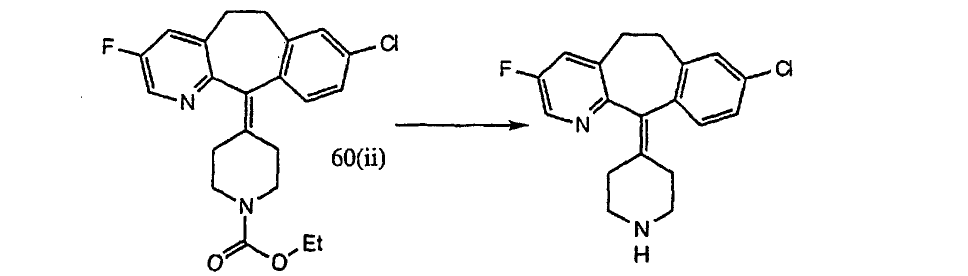

Hydrolyze 3.4 g of the product 60 (ii) from Step A via essentially the same process as described for Example 358, Step A, of WO 95/10516, to give 3.01 g of product compound. Mass Spec.: MH⁺ = 329

Using compound 60(i) from Preparative Example 60, Step A, and following substantially the same procedure as described in Preparative Example 60, Step B, the compound: 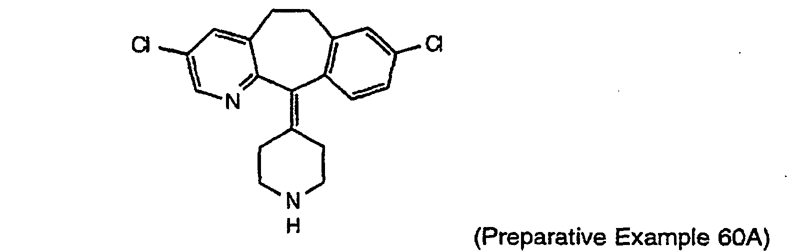 was prepared. Mass Spec.: MH⁺ = 346.

### PREPARATIVE EXAMPLE 66

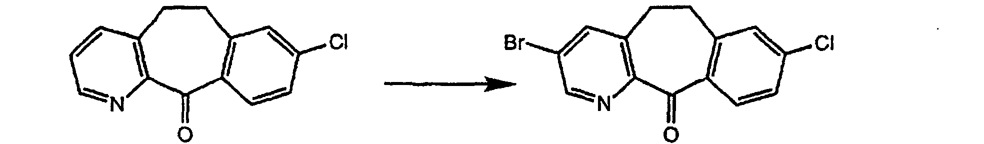

Cool 50.0 g (20.5 mmol) of 8-chloro-5,6-dihydro-11H-benzo[5,6]cyclohepta[1,2-b]pyridin-11-one to 0°C, slowly add 75 mL (93.69 mmol) of sulfur monochloride over 20 minutes, then slowly add 25 mL (48.59 mmol) of Br₂ over 15. Heat at 95°C for 20 hour, add 12.5 mL (24.3 mmol) of Br₂ and heat for a another 24 hours. Cool the mixture, and slowly add to a mixture of CH₂Cl₂ and 1N NaOH (aqueous) at 0°C. Wash the organic phase with water, dry over MgSO₄ and concentrate *in vacuo* to a residue. Chromatograph the residue (silica gel, 500 mL CH₂Cl₂ then 0.2%-5% (10% NH₄OH in MeOH)/CH₂Cl₂), then chromatograph again (silica gel, 3%-8.5% EtOAc/hexane) to give 8.66 g of the product compound. Mass Spec.: MH⁺ = 322

### PREPARATIVE EXAMPLE 67

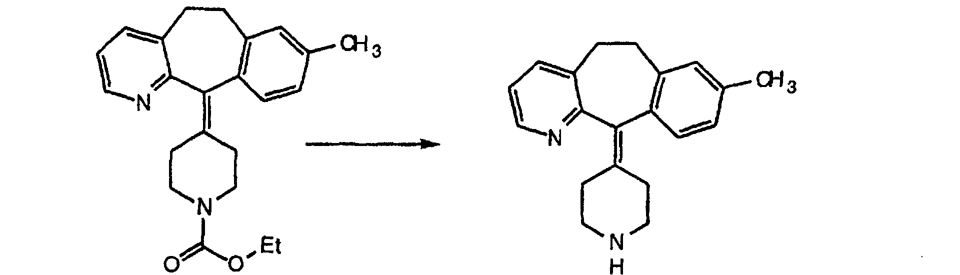

Dissolve 0.16 g (0.46 mmol) of 4-(8-methyl-5,6-dihydro-11H-benzo[5,6]cyclohepta[1,2-b]pyridin-11-ylidine)-1-ethoxycarbonylpiperidine, in 2 mL EtOH and add 4 mL of 12 N HCl. Heat the solution for 3 hours at 85°C, then cool to 25°C. Adjust to pH = 10 with 50% NaOH (aqueous) and extract several times with 50 mL of EtOAc. Combine the organic layers, dry them over MgSO₄, and concentrate *in vacuo* to give the product compound.

### PREPARATIVE EXAMPLE 68

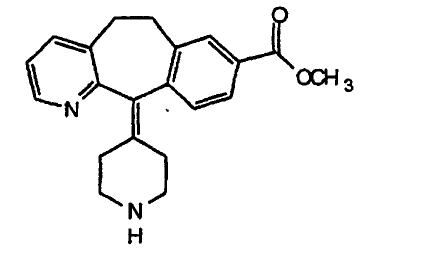

### Step A:

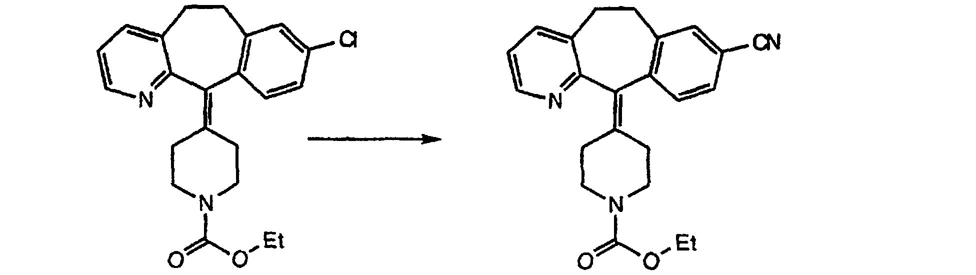

Disolve 2 g (5.22 mmol) of the title compound of Preparative Example 1F, of WO 95/10516, in 2.6 mL of dry N-methylpyrrolidinone. Add 0.87 g (9.4 mmol) of CuCN and 0.139 g (0.93 mmol) of sodium iodide. Heat the mixture at 200°C under nitrogen for 20 hours, cool to 25°C and repeatedly grind and mix with five 50 mL portions of CH₂Cl₂ and 7 M NH₄OH (aqueous). Wash the organic layer with 7 M NH₄OH until the organic layer is no longer blue or green. Dry the combined organic layers over MgSO₄ and concentrate *in vacuo* to a residue. Chromatograph (silica gel 70% EtOAc/hexane), then recrystallize from EtOAc/hexane to give the product compound. m.p. = 152.4°-153.5°C; Mass Spec.: MH⁺ = 374

### Step B:

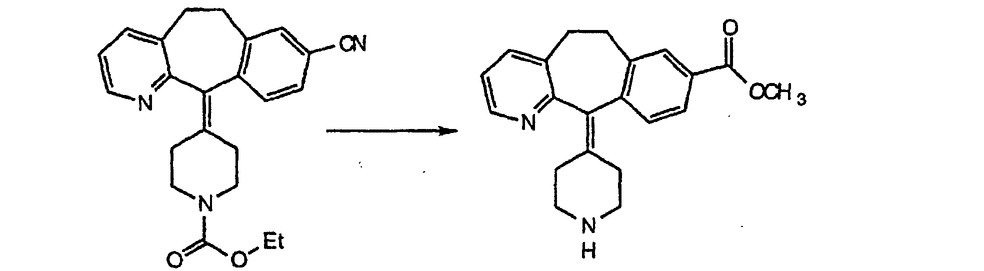

Dissolve 4.08 g (10.93 mmol) of the product of Step A in 12 M HCl and heat at 85°C for 18 hours. Concentrate *in vacuo* to a residue. Dissolve the residue in 175 mL of MeOH, saturate with HCl gas, and heat at reflux for 18 hours. Concentrate *in vacuo* to give the product compound as its HCl salt. Mass Spec.: MH⁺ =335

### PREPARATIVE EXAMPLE 69

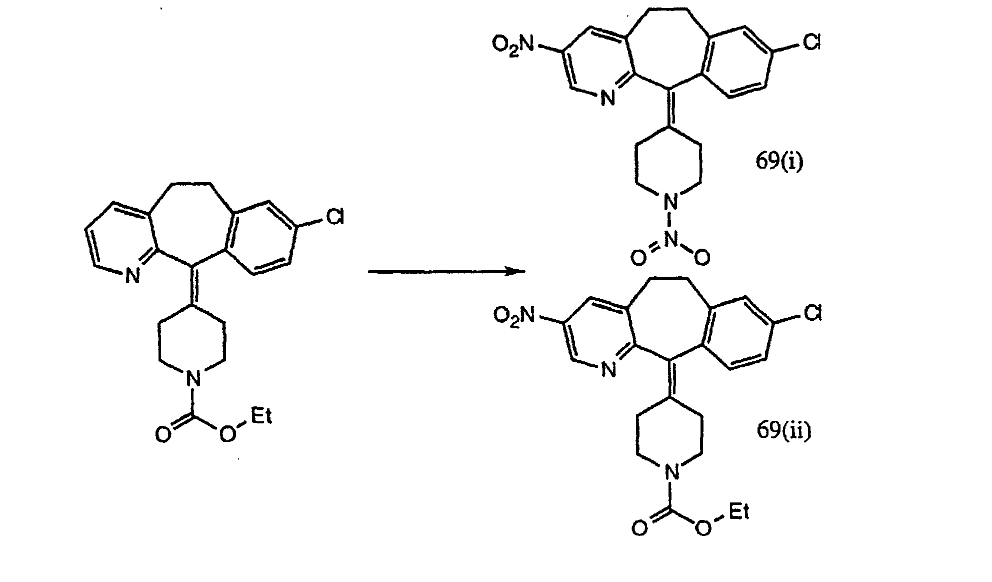

Combine 75 g (0.196 mole) of the Product of Example 1, Step F, of WO 95/10516, and 300 mL of CH₂Cl₂ at 0°C, and slowly add (dropwise) a solution of 72 g (0.236 mole) of tetrabutylammonium nitrate and 35 mL (0.247 mole) of TFAA in 500 mL of CH₂Cl₂. Stir at 25°C overnight, slowly add (dropwise) 1 L of saturated NaHCO₃ (aqueous). Separate the layers, wash the organic phase with brine and dry over MgSO₄. Concentrate *in vacuo* to a residue, chromatograph twice (1 kg silica gel, gradient of EtOAc/CH₂Cl₂) to give 8.63 g of product compound 69(i), and 34 g of product compound (ii). Recrystallize compound 69(i) from CH₂Cl₂/hexane to give the purified product compound 69(i). m.p.= 186°-187°C; Mass Spec.: (FAB) MH⁺ = 401

### PREPARATIVE EXAMPLE 69A

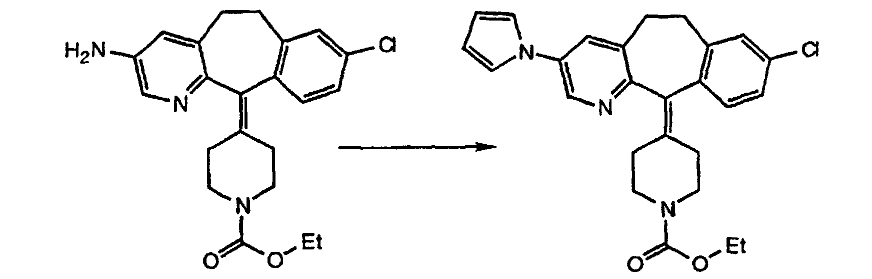

Combine 0.4 g (1 mmol) of the Product of Example 47, Step B, of WO 95/10516 (published April 20, 1995), and 0.2 mL (1.2 mmoles) of 2, 5-diethoxytetrahydrofuran in 3 mL of glacial HOAc, and heat at reflux for 1.5 hours. Cool the mixture, wash with saturated NaHCO₃ (aqueous), then with brine, dry over MgSO₄, and concentrate *in vacuo* to a residue. Chromatograph (silica gel, 5%-15% EtOAc/CH₂Cl₂) to give 0.34 g of the product compound. Mass Spec.: (FAB) MH⁺ = 448

### PREPARATIVE EXAMPLE 70

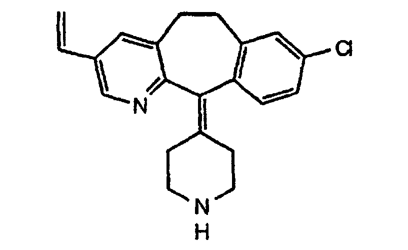

### Step A:

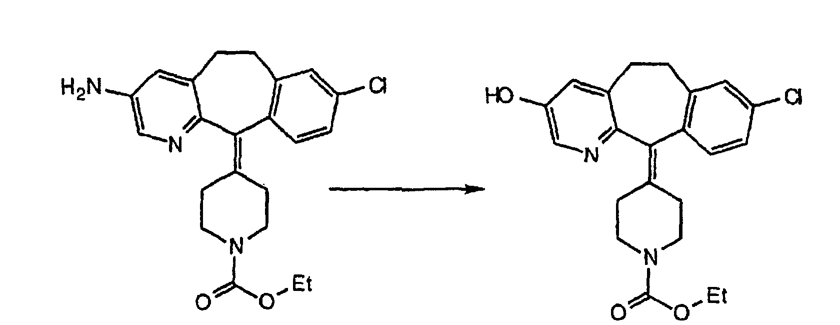

Combine 13.8 g (34.7 mmol) of the Product of Example 47, Step B, of WO 95/10516, and 90 mL of water at 0°C, add a solution of 6.9 mL of concentrated H₂SO₄ in 45 mL of water and stir the mixture. Slowly add (dropwise) a solution of 2.55 g (40 mmol) of NaNO₂ in 75 mL of water and stir at 0°-5°C for 0.5 hours. Add a boiling solution of 35.1 g CuSO₄ in 135 mL of water and heat at 100°C for 15 min. Cool the mixture, extract with CH₂Cl₂ (2 X 200 mL), wash the extracts with brine, dry over MgSO₄, and concentrate *in vacuo* to a residue. Chromatograph (silica gel, 1.5%-10% MeOH/ CH₂Cl₂) to give 11.36 g of the product compound.

### Step B:

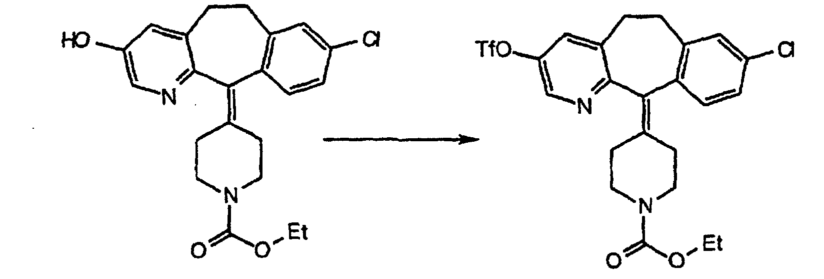

Combine 11.36 g (28.5 mmol) of the Product of Step A and 12.4 g (34.7 mmol) of N-phenyltriflimide in 120 mL of dry CH₂Cl₂ at 0°C, add 4.6 mL (33 mmol) of Et₃N and stir at 25°C overnight. Concentrate in *vacuo* to a residue and chromatograph (silica gel, 2%-5% EtOAc/ CH₂Cl₂) to give 10.95 g of the product compound. Recrystallize from hot MeOH. m.p. = 154.5°-156°C; Mass Spec.: (FAB) MH⁺=531

### Step C:

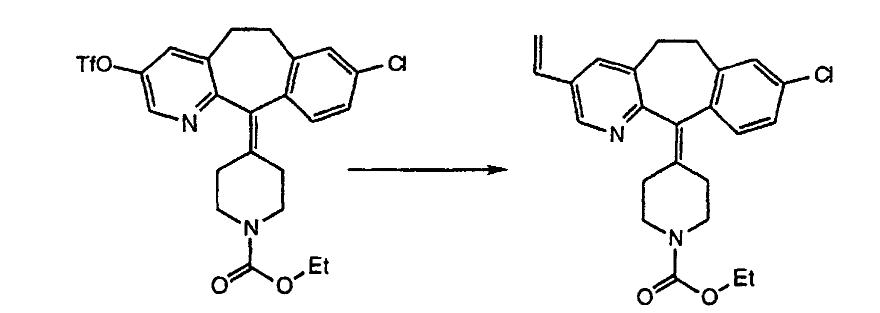

Combine 12.2 g (23 mmol) of the Product of Step B and 85 mL of 1-methyl-2-pyrrolidinone at 25°C, then add 2.84 g LiCl, 0.212 g of trisfurylphosphine and 0.585 g of dipalladiumtribenzylideneacetone and stir for 15 min. Slowly add (dropwise) 7.5 mL (25.77 mmol) of tributylvinyltin and stir at 25°C for 2.5 hours. Dilute with 500 mL of water at 0°C and extract with 6700 mL of EtOAc. Filter the organic phase through celite®, wash the celite with EtOAc, then wash the filtrate twice with 30% NaF (aqueous). Filter the organic solution, wash with brine and dry over MgSO₄. Concentrate *in vacuo* to a residue and chromatograph (silica gel, 15%-40% EtOAc/hexane) to give 8.58 g of the product compound. Mass Spec.: (FAB) MH⁺ = 409

Using 2-(tributylstannyl)thiophene and the compound of Preparative Example 70, Step B, and following substantially the same procedure as described for Preparative Example 70, Step C, the compound: 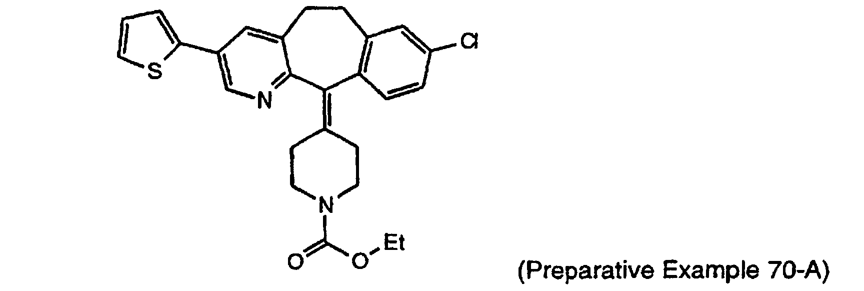 was prepared. m.p. = 155°∼157°C, Mass Spec.: MH⁺ = 465.

### Step D:

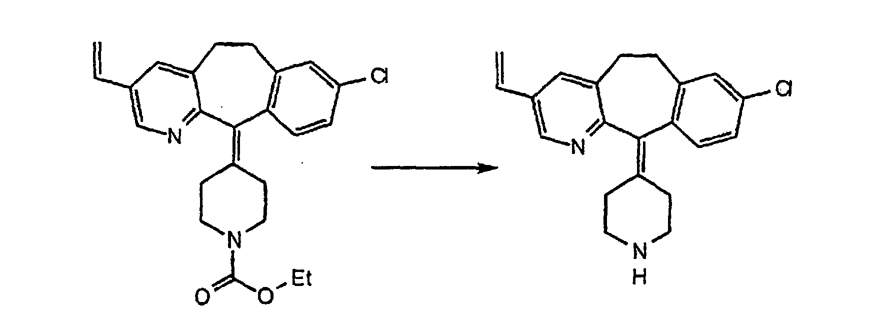

Hydrolyze 1.18 g (2.89 mmol) of the product of Step C via substantially the same procedure as described in Example 358, Step A, of WO 95/10516 ,to give 0.95 g of the product compound. Mass Spec.: (FAB) MH⁺ = 337

### PREPARATIVE EXAMPLE 71

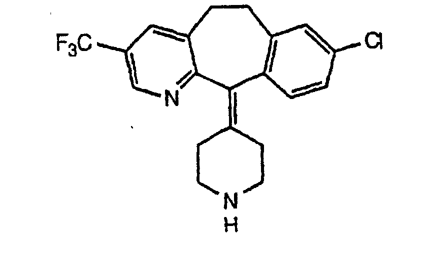

### Step A:

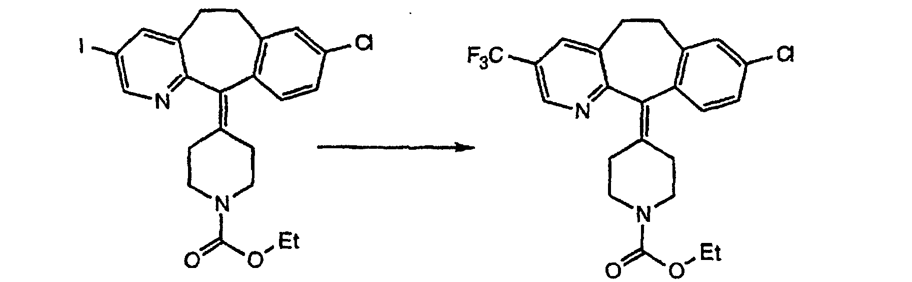

Combine 1.01 g (19.9 mmol) of the Product of Preparative Example 48, Step A, 30 mL of DMF, 1.33 g (6.96 mmol) of methyl 2,2-difluoro-2-(fluorosulfonyl)-acetate and 0.75 g (3.97 g) of CuI. Heat the mixture at 60°-80°C for 3 hours, then concentrate to a residue. Dilute the residue with water, extract with CH₂Cl₂, and concentrate *in vacuo* to a residue. Chromatograph (silica gel, 30% EtOAc/hexane, then 10% MeOH/CH₂Cl₂ +NH₄OH) to give 0.15 g of the product compound. Mass Spec.: MH+ = 451.1

### Step B:

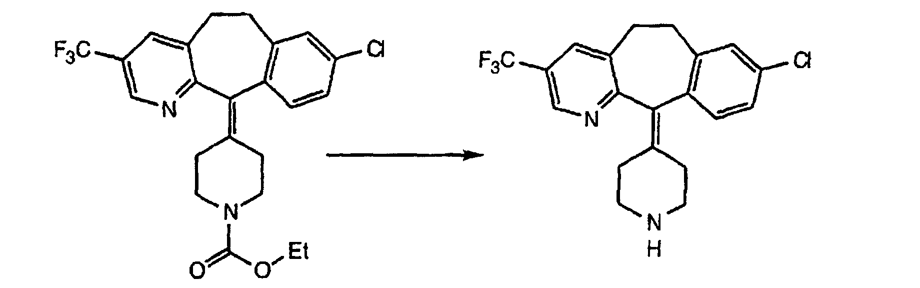

Hydrolyze the product of Step A using essentially the same procedure as described in Preparative Example 1, Step G, of WO 95/10516, to give the product compound. Mass Spec.: MH⁺ = 379

### PREPARATIVE EXAMPLE 72

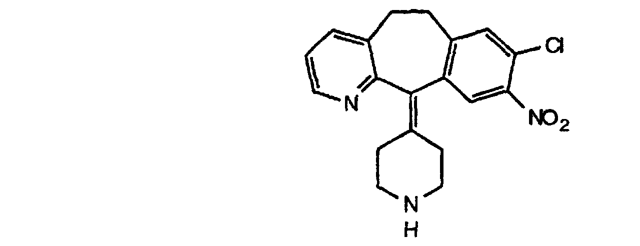

### Step A:

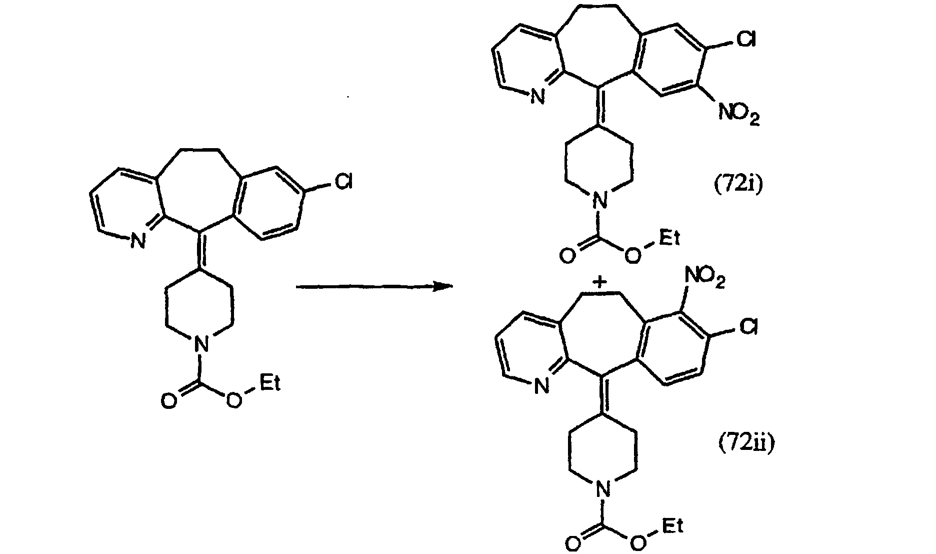

Dissolve 20 g (50 mmol) of the Product of Preparative Example 1, Step F, of WO 95/10516, in 400 mL of concentrated H₂SO₄, cool to -5°C and add 5.1 g (50 mmol) of KNO₃ in small portions. Stir for 3 hours, cool the mixture and slowly basify with 50% NaOH (aqueous). Extract with CH₂Cl₂ (3 X 500 mL), dry the combined extracts over MgSO₄, and concentrate *in vacuo* to a residue. Chromatograph (silica gel, 50% EtOAc/hexane) to give 16.33 g of the product compound (72i) and 2.6 g of the product compound (72ii). Mass Spec. (72(i) and (72(ii)): MH⁺ = 428

### Step B:

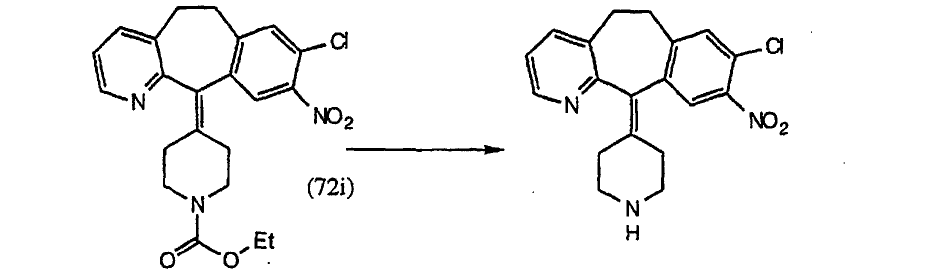

Hydrolyze 5.46 g (12.76 mmol) of the Product of (72i) from Step A, via substantially the same procdure as described for Example 358, Step A, of WO 95/10516, to give 4.34 g of the product compound. Mass Spec.: MH⁺ = 356

### PREPARATIVE EXAMPLE 73

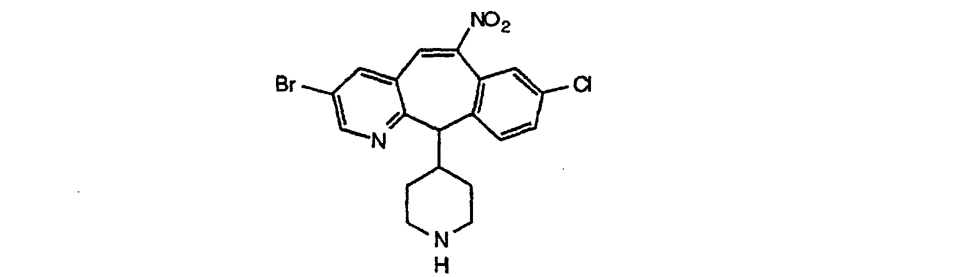

### Step A:

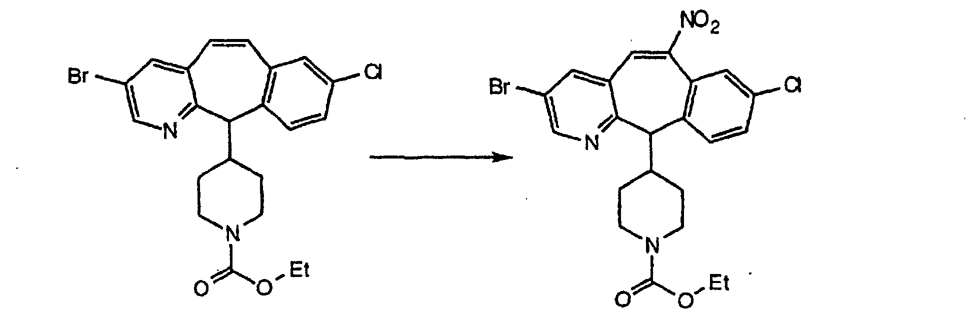

Combine 1.6 g of the Product (54i) of Preparative Example 54, Step B, 12 mL of CH₂Cl₂, and 1.16 g of tetrabutylammonium nitrate, cool to 0°C and slowly add (dropwise) a solution of 0.8 g of TFAA in 2 mL of CH₂Cl₂. Stir for 6 hours at 0°C, let the mixture stand at 0°C overnight, then wash successively with saturated NaHCO₃ (aqueous), water and brine, and dry over Na₂SO₄. Concentrate *in vacuo* to a residue, then chromatograph (silica gel, 30% EtOAc/hexane) to give 0.38 g of the product compound.

### Step B:

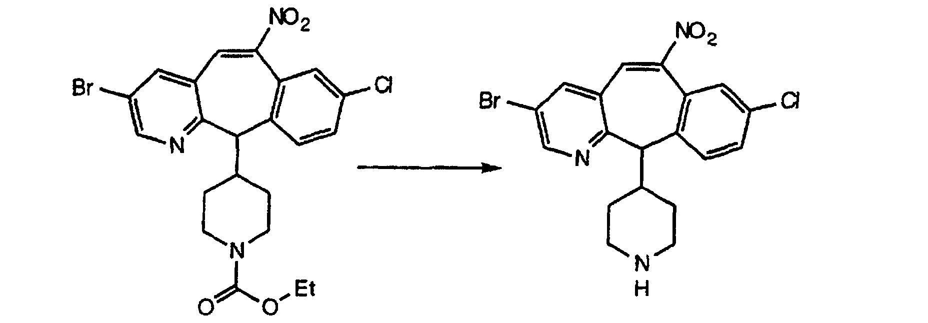

Hydrolyze 0.38 g of the Product of Step A via substantially the same procedure as described for Example 358, Step A, of WO 95/10516, to give 0.235 g of the product compound.

### Preparative Example 75. 4-(3-bromo-8-chloro-5,6-dihydro-11H-benzo[5.6]cyclohepta[1,2-b]pyridin-11-ylidene)-N-(4H-1,3-benzodioxin-6-yl)-1-piperidinecarbothioamide

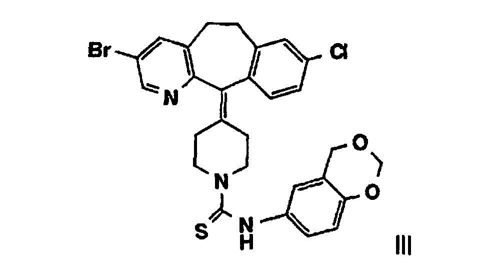

Dissolve 4-(3-bromo-8-chloro-5,6-dihydro-11H-benzo[5.6]cyclohepta[1,2-b]pyridin-11-ylidene)-1-piperidine (0.5 gm, 1.61 mmol) in 5 mL of dry tetrahydrofuran. Add 4H-1,3-benzodioxin-6-yl) isothiocyanate (0.34 gm, 1.77 mmol) and stir at ambient temperature for 24 hours. Evaporate the reaction mixture to an oil and chromatograph on silica gel using 1% up to 5% methanol/methylene chloride as the eluent to obtain 0.893 g of title compound. FABMS M+1= 694

### ASSAYS

1. In vitro enzyme assays: FPT IC₅₀ (inhibition of farnesyl protein transferase, in vitro enzyme assay) are determined by the methods disclosed in WO/10515 or WO 95/10516. The data demonstrate that the compounds of the invention are inhibitors of Ras-CVLS famesylation by partially purified rat brain famesyl protein transferase (FPT). The data also show that there are compounds of the invention which can be considered as potent (IC₅₀ <10 µM) inhibitors of Ras-CVLS farnesylation by partially purified rat brain FPT.
2. Cell-based assay. COS IC₅₀ values refer to the COS cells activity inhibition of Ras processing, are determined by the methods disclosed in WO/10515 or WO 95/10516.

For preparing pharmaceutical compositions from the compounds described by this invention, inert, pharmaceutically acceptable carriers can be either solid or liquid. Solid form preparations include powders, tablets, dispersible granules, capsules, cachets and suppositories. The powders and tablets may be comprised of from about 5 to about 70 percent active ingredient. Suitable solid carriers are known in the art, e.g. magnesium carbonate, magnesium stearate, talc, sugar, lactose. Tablets, powders, cachets and capsules can be used as solid dosage forms suitable for oral administration.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

Liquid form preparations include solutions, suspensions and emulsions. As an example may be mentioned water or water-propylene glycol solutions for parenteral injection.

Liquid form preparations may also include solutions for intranasal administration.

Aerosol preparations suitable for inhalation may include solutions and solids in powder form, which may be in combination with a pharmaceutically acceptable carrier, such as an inert compressed gas.

Also included are solid form preparations which are intended to be converted, shortly before use, to liquid form preparations for either oral or parenteral administration. Such liquid forms include solutions, suspensions and emulsions.

The compounds of the invention may also be deliverable transdermally. The transdermal compositions can take the form of creams, lotions, aerosols and/or emulsions and can be included in a transdermal patch of the matrix or reservoir type as are conventional in the art for this purpose.

Preferably the compound is administered orally.

Preferably, the pharmaceutical preparation is in unit dosage form. In such form, the preparation is subdivided into unit doses containing appropriate quantities of the active component, e.g., an effective amount to achieve the desired purpose.

The quantity of active compound in a unit dose of preparation may be varied or adjusted from about 0.1 mg to 1000 mg, more preferably from about 1 mg. to 300 mg, according to the particular application.

The actual dosage employed may be varied depending upon the requirements of the patient and the severity of the condition being treated. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

The amount and frequency of administration of the compounds of the invention and the pharmaceutically acceptable salts thereof will be regulated according to the judgment of the attending clinician considering such factors as age, condition and size of the patient as well as severity of the symptoms being treated. A typical recommended dosage regimen is oral administration of from 10 mg to 2000 mg/day preferably 10 to 1000 mg/day, in two to four divided doses to block tumor growth. The compounds are non-toxic when administered within this dosage range.

The following are examples of pharmaceutical dosage forms which contain a compound of the invention. The scope of the invention in its pharmaceutical composition aspect is not to be limited by the examples provided.

### Pharmaceutical Dosage Form Examples

### EXAMPLE A-Tablets

| No. | Ingredients | mg/tablet | mg/tablet |
|---|---|---|---|
| 1. | Active compound | 100 | 500 |
| 2. | Lactose USP | 122 | 113 |
| 3. | Corn Starch, Food Grade, as a 10% paste in Purified Water | 30 | 40 |
| 4. | Corn Starch, Food Grade | 45 | 40 |
| 5. | Magnesium Stearate | 3 | 7 |
| Total | | 300 | 700 |

### Method of Manufacture

Mix Item Nos. 1 and 2 in a suitable mixer for 10-15 minutes. Granulate the mixture with Item No. 3. Mill the damp granules through a coarse screen (e.g., 1/4", 0.63 cm) if necessary. Dry the damp granules. Screen the dried granules if necessary and mix with Item No. 4 and mix for 10-15 minutes. Add Item No. 5 and mix for 1-3 minutes. Compress the mixture to appropriate size and weigh on a suitable tablet machine.

### EXAMPLE B-Capsules

| No. | Ingredient | mg/capsule | mg/capsule |
|---|---|---|---|
| 1. | Active compound | 100 | 500 |
| 2. | Lactose USP | 106 | 123 |
| 3. | Corn Starch, Food Grade | 40 | 70 |
| 4. | Magnesium Stearate NF | 7 | 7 |
| Total | | 253 | 700 |

### Method of Manufacture

Mix Item Nos. 1, 2 and 3 in a suitable blender for 10-15 minutes. Add Item No. 4 and mix for 1-3 minutes. Fill the mixture into suitable two-piece hard gelatin capsules on a suitable encapsulating machine.

## Claims

1. A compound of the formula: 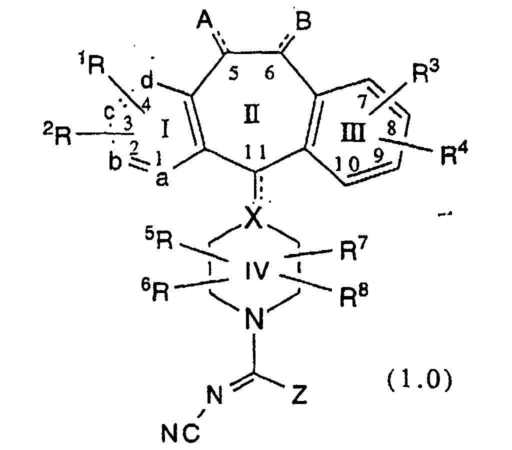 or a pharmaceutically acceptable salt or solvate thereof, wherein:
one of a, b, c and d represents N or NR⁹ wherein R⁹ is O⁻, -CH₃ or -(CH₂)ₙCO₂H wherein n is 1 to 3, and the remaining a, b, c and d groups represent CR¹ or CR²; or
each of a, b, c, and d are independently selected from CR¹ or CR²; each R¹ and each R² is independently selected from H, halo, -CF₃, -OR¹⁰ (e.g., -OCH₃), -COR¹⁰, -SR¹⁰ (e.g., -SCH₃ and -SCH₂C₆H₅), -S(O)ₜR¹¹ (wherein t is 0, 1 or 2, e.g., -SOCH₃ and -SO₂CH₃), -SCN, -N(R¹⁰)₂, -NR¹⁰R¹¹, -NO₂, -OC(O)R¹⁰, -CO₂R¹⁰, -OCO₂R¹¹, -CN, -NHC(O)R¹⁰, -NHSO₂R¹⁰, -CONHR¹⁰, -CONHCH₂CH₂OH, -NR¹⁰COOR¹¹, -SR¹¹C(O)OR¹¹ (e.g., -SCH₂CO₂CH₃), -SR¹¹N(R⁷⁵)₂ wherein each R⁷⁵ is independently selected from H and -C(O)OR¹¹ (e.g., -S(CH₂)₂NHC(O)O-t-butyl and -S(CH₂)₂NH₂), benzotriazol-1-yloxy, tetrazol-5-ylthio, or substituted tetrazol-5-ylthio (e.g., alkyl substituted tetrazol5-ylthio such as 1-methyl-tetrazol-5-ylthio), alkynyl, alkenyl or alkyl, said alkyl or alkenyl group optionally being substituted with halo, -OR¹⁰ or -CO₂R¹⁰;
R³ and R⁴ are the same or different and each independently represents H, any of the substituents of R¹ and R², or R³ and R⁴ taken together represent a saturated or unsaturated C₅-C₇ fused ring to the benzene ring (Ring III);
R₅, R₆, R₇ and R₈ each independently represents H, -CF₃, -COR¹⁰, alkyl or aryl, said alkyl or aryl optionally being substituted with -OR¹⁰, -SR¹⁰, -S(O)ₜR¹¹, -NR¹⁰COOR¹¹, -N(R¹⁰)₂, -NO₂, -COR¹⁰, -OCOR¹⁰, -OCO₂R¹¹, -CO₂R¹⁰, OPO₃R¹⁰ or one of R⁵, R⁶, R⁷ and R⁸ can be taken in combination with R⁴⁰ as defined below to represent -(CH₂)ᵣ wherein r is 1 to 4 which can be substituted with lower alkyl, lower alkoxy, -CF₃ or aryl, or R⁵ is combined with R⁶ to represent =O or =S and/or R⁷ is combined with R⁸ to represent =O or =S;
R¹⁰ and R¹² independently represent H, alkyl, cycloalkyl, cycloalkylalkyl, heteroaryl, aryl, aralkyl or -NR⁴⁰R⁴² wherein R⁴⁰ and R⁴² independently represent H, aryl, alkyl, aralkyl, heteroaryl, heteroarylalkyl, heterocycloalkyl, heterocycloalkylalkyl, heteroalkyl, cycloalkyl, cycloalkylalkyl, alkenyl and alkynyl;
R¹¹ represents alkyl or aryl;
X represents N, CH or C, such that when X is N or CH, there is a single bond to carbon atom 11 as represented by the solid line; or when X is C, there is a double bond to carbon atom 11, as represented by the solid and dotted lines;
the dotted line between carbon atoms 5 and 6 represents an optional double bond, such that when a double bond is present, A and B independently represent -NO₂, -R¹⁰, halo, -OR¹¹, -OCO₂R¹¹ or -OC(O)R¹⁰, and when no double bond is present between carbon atoms 5 and 6, A and B each independently represent H₂, -(OR¹¹)₂, H and halo, dihalo, alkyl and H, (alkyl)₂, -H and -OC(O)R¹⁰, H and -OR¹⁰, oxy, aryl and H, =NOR¹⁰ or -O-(CH₂)ₚ-O- wherein p is 2, 3 or 4; and
Z represents alkyl, aryl, aralkyl, heteroalkyl, heteroaryl, heteroarylalkyl, heterocycloalkyl, heterocycloalkylalkyl, -OR⁴⁰, -SR⁴⁰, -CR⁴⁰R⁴² or -NR⁴⁰R⁴² wherein R⁴⁰ and R⁴² are defined hereinbefore, wherein unless indicated otherwise, the following definitions apply:
alkyl-(including the alkyl portions of alkoxy, alkylamino and dialkylamino)-represents straight and branched carbon chains and contains from one to twenty carbon atoms, preferably one to six carbon atoms; for example methyl, ethyl, propyl, iso-propyl, n-butyl, t-butyl, n-pentyl, isopentyl, hexyl and the like; wherein said alkyl group may be optionally and independently substituted with one, two, three or more of the following: halo, alkyl, aryl, alkoxy, amino, alkylamino, cyano, -CF₃, dialkylamino, hydroxy, oxy (=O), phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰;
alkoxy-an alkyl moiety of one to 20 carbon atoms covalently bonded to an adjacent structural element through an oxygen atom, for example, methoxy, ethoxy, propoxy, butoxy, pentoxy, hexoxy and the like; wherein said alkoxy group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰;
alkenyl-represents straight and branched carbon chains having at least one carbon to carbon double bond and containing from 2 to 12 carbon atoms, preferably from 2 to 6 carbon atoms and most preferably from 3 to 6 carbon atoms; wherein said alkenyl group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, NHSO₂, -NO₂, -CONR¹⁰, -NCOR¹⁰ or -COOR¹⁰;
alkynyl-represents straight and branched carbon chains having at least one carbon to carbon triple bond and containing from 2 to 12 carbon atoms, preferably from 2 to 6 carbon atoms; wherein said alkynyl group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰;
aryl (including the aryl portion of aralkyl)-represents a carbocyclic group containing from 6 to 15 carbon atoms and having at least one aromatic ring (e.g., aryl is phenyl), wherein said aryl group optionally can be fused with aryl, cycloalkyl, heteroaryl or heterocycloalkyl rings; and wherein any of the available substitutable carbon and nitrogen atoms in said aryl group and/or said fused ring(s) may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰;
aralkyl - represents an alkyl group, as defined above, wherein one or more hydrogen atoms of the alkyl moiety have been substituted with one or more aryl groups; wherein said aralkyl group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰; Representative aralkyl groups include benzyl and diphenylmethyl;
cycloalkyl-represents saturated carbocyclic rings branched or unbranched of from 3 to 20 carbon atoms, preferably 3 to 7 carbon atoms; wherein said cycloalkyl group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰;
cycloalkylalkyl - represents an alkyl group, as defined above, wherein one or more hydrogen atoms of the alkyl moiety have been substituted with one or more cycloalkyl groups; wherein said cycloalkylalkyl group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰;
halo-represents fluoro, chloro, bromo and iodo;
heteroalkyl-represents straight and branched carbon chains containing from one to twenty carbon atoms, preferably one to six carbon atoms interrupted by 1 to 3 heteroatoms selected from -O-, -S- and -N-; wherein any of the available substitutable carbon and nitrogen atoms in said heteroalkyl chain may be optionally and independendently substituted with one, two, three or more of the following: halo, C₁-C₆ alkyl, aryl, cyano, hydroxy, alkoxy, oxy, phenoxy, -CF₃, -OCF₃, amino, alkylamino, dialkylamino, heterocycloalkyl, -SO₂NH₂, -NHSO₂R¹⁰, -SO₂NHR¹⁰, -SO₂R¹⁰, -SOR¹⁰, -SR¹⁰, or -NHSO₂, -NO₂, -CONR¹⁰, -NCOR¹⁰ or -COOR¹⁰;
heteroaryl-represents cyclic groups having at least one heteroatom selected from O, S and N, said heteroatom(s) interrupting a carbocyclic ring structure and having a sufficient number of delocalized pi electrons to provide aromatic character, with the aromatic heterocyclic groups containing from 2 to 14 carbon atoms, wherein said heteroaryl group optionally can be fused with one or more aryl, cycloalkyl, heteroaryl or heterocycloalkyl rings; and wherein any of the available substitutable carbon or nitrogen atoms in said heteroaryl group and/or said fused ring(s) may be optionally and independendently substituted with one, two, three or more of the following: halo, C₁-C₆ alkyl, aryl, cyano, hydroxy, alkoxy, oxy, phenoxy, -CF₃, -OCF₃, amino, alkylamino, dialkylamino, heterocycloalkyl, -SO₂NH₂, -NHSO₂R¹⁰, -SO₂NHR¹⁰, -SO₂R¹⁰, -SOR¹⁰, -SR¹⁰, or -NHSO₂, -NO₂, -CONR¹⁰, -NCOR¹⁰ or -COOR¹⁰. Representative heteroaryl groups can include, for example, furanyl, imidazoyl, pyrimidinyl, triazolyl, 2-, 3- or 4-pyridyl or 2-, 3- or 4-pyridyl N-oxide wherein pyridyl N-oxide can be represented as: 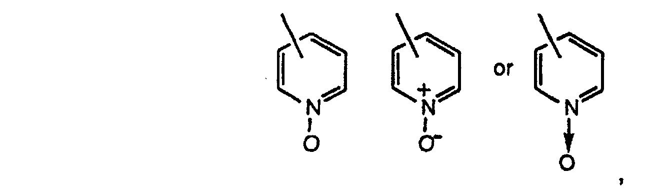
heteroarylalkyl - represents an alkyl group, as defined above, wherein one or more hydrogen atoms have been replaced by one or more heteroaryl groups; wherein said heteroarylalkyl group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰; as exemplified by 2-, 3- or 4-pyridylmethyl or 2-, 3- or 4-pyridylmethyl N-oxide;
heterocycloalkyl-represents a saturated, branched or unbranched carbocylic ring containing from 3 to 15 carbon atoms, preferably from 4 to 6 carbon atoms, which carbocyclic ring is interrupted by 1 to 3 heteroatoms selected from -O-, -S- and -N-, wherein optionally, said ring may contain one or two unsaturated bonds which do not impart aromatic character to the ring; and wherein any of the available substitutable carbon and nitrogen atoms in the ring may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰. Representative heterocycloalkyl groups can include 2- or 3-tetrahydrofuranyl, 2- or 3- tetrahydrothienyl, 1-, 2-, 3- or 4-piperidinyl, 2- or 3-pyrrolidinyl, 1-, 2- or 3-piperizinyl, 2- or 4-dioxanyl, morpholinyl, 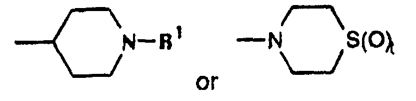 wherein R¹ is defined hereinbefore and t is 0, 1 or 2;
heterocycloalkylalkyl- represents an alkyl group, as defined above, wherein one or more hydrogen atoms have been replaced by one or more heterocycloalkyl groups; wherein optionally, said ring may contain one or two unsaturated bonds which do not impart aromatic character to the ring; and wherein said heterocycloalkylalkyl group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰.

2. A compound according to Claim 1 wherein there is a single bond at carbon atom 11; X is carbon; positions 3, 8 and 10 are substituted on the ring, preferably with halo; and Z is -NHR⁴⁰, preferably wherein R⁴⁰ is heteroarylalkyl, more preferably 3- or 4-methyl pyridyl N-oxide.

3. A compound according to Claim 1 wherein a is N, and R¹ is H; R², R³ and R⁴ are halo; X is CH; and R⁵, R⁶, R⁷ and R⁸ are hydrogen.

4. A compound according to Claim 3 wherein Z is -NR⁴⁰R⁴².

5. A compound according to Claim 4 wherein R⁴⁰ is H and R⁴² is heteroarylalkyl.

6. A compound according to Claim 5 wherein R⁴² is 2-, 3- or 4-pyridylmethyl N-oxide

7. A compound according to Claim 1 selected from any of Examples 1 to 83.

8. A compound according to Claim 1 having the structure:

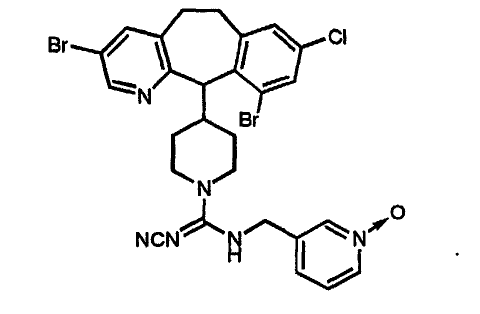

9. A pharmaceutical composition for inhibiting the abnormal growth of cells comprising an effective amount of a compound of any of Claims 1 to 8 in combination with a pharmaceutically acceptable carrier.

10. Use of a compound as defined in any of Claims 1 to 8 for the manufacture of a medicament for inhibiting the abnormal growth of cells.

11. Use according to Claim 10 wherein the cells inhibited are tumor cells expressing an activated ras oncogene.

12. Use according to Claim 11 wherein the cells inhibited are pancreatic tumor cells, lung cancer cells, myeloid leukemia tumor cells, thyroid follicular tumor cells, myelodysplastic tumor cells, epidermal carcinoma tumor cells, bladder carcinoma tumor cells or colon tumors cells.

13. Use according to Claim 10 wherein the inhibition of the abnormal growth of cells occurs by the inhibition of ras farnesyl protein transferase.

14. Use according to Claim 10 wherein the inhibition is of tumor cells wherein the Ras protein is activated as a result of oncogenic mutation in genes other than the Ras gene.

## Patentansprüche

1. Verbindung der Formel: 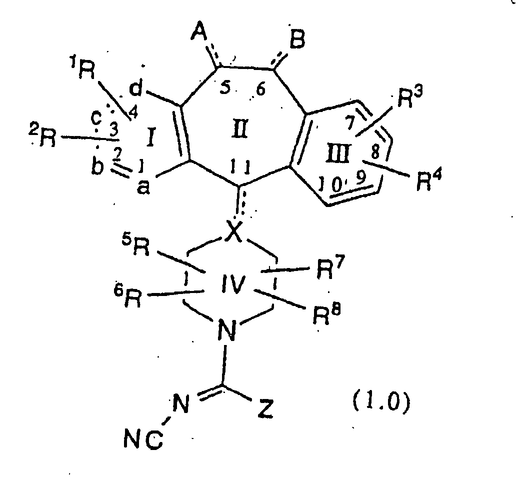 oder ein pharmazeutisch akzeptables Salz davon, bei der:
eines von a, b, c und d N oder NR⁹ repräsentiert, wobei R⁹ O-, -CH₃ oder -(CH₂)ₙCO₂H ist, wobei n 1 bis 3 ist, und die verbleibenden a-, b-, c- und d-Gruppen CR¹ oder CR² repräsentieren; oder
jedes a, b, c, und d unabhängig ausgewählt ist aus CR¹ oder CR²; jedes R¹ und jedes R² unabhängig ausgewählt ist aus H, Halogen, -CF₃, -OR¹⁰ (z.B. -OCH₃), -COR¹⁰, -SR¹⁰ (z.B. -SCH₃ und -SCH₂C₆H₅), -S(O)ₜR¹¹ (wobei t 0, 1 oder 2 ist, z.B. -SOCH₃ und -SO₂CH₃), -SCN, -N(R¹⁰)₂, -NR¹⁰R¹¹, -NO₂, -OC(O)R¹⁰, -CO₂R¹⁰, -OCO₂R¹¹, -CN, -NHC(O)R¹⁰, -NHSO₂R¹⁰, -CONHR¹⁰, -CONHCH₂CH₂OH, -NR¹⁰COOR¹¹, -SR¹¹C(O)OR¹¹ (z.B. -SCH₂CO₂CH₃), -SR¹¹N(R⁷⁵)₂, wobei jedes R⁷⁵ unabhängig ausgewählt ist aus H und -C(O)OR¹¹ (z.B. -S(CH₂)₂NHC(O)O-tert-butyl und -S(CH₂)₂NH₂), Benzotriazol-1-yloxy, Tetrazol-5-ylthio oder substituiertes Tetrazol-5-ylthio (z.B. alkylsubstituiertes Tetrazol-5-ylthio wie 1-Methyl-tetrazol-5-ylthio), Alkynyl, Alkenyl oder Alkyl, wobei die Alkyl- oder Alkenylgruppe mit Halogen, -OR¹⁰ oder -CO₂R¹⁰ substituiert sein kann;
R³ und R⁴ identisch oder unterschiedlich sind und jeweils unabhängig H, einen der Substituenten von R¹ und R² repräsentieren, das R⁴ und R⁵ zusammen einen gesättigten oder ungesättigten an den Benzolring (Ring III) kondensierten C₅- bis C₇-Ring repräsentieren;
R⁵, R⁶, R⁷ und R⁸ jeweils unabhängig H, -CF₃, -COR¹⁰, Alkyl oder Aryl repräsentieren, das Alkyl oder Aryl mit -OR¹⁰, -SR¹⁰, -S(O)ₜR¹¹, -NR¹⁰COOR¹¹, -N(R¹⁰)₂, -NO₂, -COR¹⁰, -OCOR¹⁰, -OCO₂R¹¹, -CO₂R¹⁰, OPO₃R¹⁰ substituiert sein kann oder eines von R⁵, R⁶, R⁷ und R⁸ in Kombination mit R⁴⁰ wie unten definiert genommen werden kann, um -(CH₂)ᵣ- zu repräsentieren, bei dem r 1 bis 4 ist und das mit niederem Alkyl, niederem Alkoxy, -CF₃ oder Aryl substituiert sein kann, oder R⁵ mit R⁶ kombiniert ist, um =O oder =S zu repräsentieren und/oder R⁷ mit R⁸ kombiniert ist, um =O oder =S zu repräsentieren;
R¹⁰ und R¹² unabhängig H, Alkyl, Cycloalkyl, Cycloalkylalkyl, Heteroaryl, Aryl, Aralkyl oder -NR⁴⁰R⁴² repräsentieren, in dem R⁴⁰ und R⁴² unabhängig H, Aryl, Alkyl, Aralkyl, Heteroaryl, Heteroarylalkyl, Heterocycloalkyl, Heterocycloalkylalkyl, Heteroalkyl, Cycloalkyl, Cycloalkylalkyl, Alkenyl und Alkinyl repräsentieren;
R¹¹ Alkyl oder Aryl repräsentiert;
X N, CH oder C repräsentiert, so dass, wenn X N oder CH ist, eine Einfachbindung zum Kohlenstoffatom 11 vorliegt, wie durch die durchgehende Linie repräsentiert wird; oder, wenn X C ist, eine Doppelbindung zum Kohlenstoffatom 11 vorliegt, wie durch die durchgehenden und unterbrochenen Linien repräsentiert wird;
die unterbrochene Linie zwischen Kohlenstoffatom 5 und 6 eine optionale Doppelbindung repräsentiert, so dass, wenn eine Doppelbindung vorliegt, A und B unabhängig -NO₂, -R¹⁰, Halogen, -OR¹¹, -OCO₂R¹¹ oder -OC(O)R¹⁰ repräsentieren, und wenn zwischen den Kohlenstoffatomen 5 und 6 keine Doppelbindung vorliegt, A und B jeweils unabhängig H₂, -(OR¹¹)₂, H und Halogen, Dihalogen, Alkyl und H, (Alkyl)₂, -H und -OC(O)R¹⁰, H und -OR¹⁰, Oxy, Aryl und H, =NOR¹⁰ oder -O-(CH₂)ₚ-O-, wobei p 2, 3 oder 4 ist, repräsentieren; und
Z Alkyl, Aryl, Aralkyl, Heteroalkyl, Heteroaryl, Heteroarylalkyl, Heterocycloalkyl, Heterocycloalkylalkyl, -OR⁴⁰, -SR⁴⁰, -CR⁴⁰R⁴² oder -NR⁴⁰R⁴² repräsentiert, wobei R⁴⁰ und R⁴² oben definiert sind,
wobei, wenn nicht anders angegeben, die folgenden Definitionen gelten:
Alkyl - (inklusive der Alkylanteile von Alkoxy, Alkylamino und Dialkylamino) - repräsentiert lineare und verzweigte Kohlenstoffketten und enthält 1 bis 20 Kohlenstoffatome, bevorzugt 1 bis 6 Kohlenstoffatome; z.B. Methyl, Ethyl, Propyl, Isopropyl, n-Butyl, t-Butyl, n-Pentyl, Isopentyl, Hexyl und andere; wobei besagte Alkylgruppen gegebenenfalls und unabhängig substituiert sein können mit einem, zwei, drei oder mehr der Folgenden: Halogen, Alkyl, Aryl, Alkoxy, Amino, Alkylamino, Cyano, -CF₃, Dialkylamino, Hydroxy, Oxy (=O), Phenoxy, -OCF₃, Heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ oder -COOR¹⁰;
Alkoxy - repräsentiert eine Alkyleinheit von 1 bis 20 Kohlenstoffatomen, die durch ein Sauerstoffatom kovalent an ein benachbartes Strukturelement gebunden sind, z.B. Methoxy, Ethoxy, Propoxy, Butoxy, Pentoxy, Hexoxy und andere; wobei die Alkoxygruppe gegebenenfalls und unabhängig substituiert sein kann mit einem, zwei, drei oder mehr der Folgenden: Halogen, Alkyl, Aryl, Alkoxy, Amino, Alkylamino, Cyano, -CF₃, Dialkylamino, Hydroxy, Oxy, Phenoxy, -OCF₃, Heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ oder -COOR¹⁰;
Alkenyl - repräsentiert lineare und verzweigte Kohlenstoffketten mit wenigstens einer Kohlenstoff-Kohlenstoff-Doppelbindung, die 2 bis 12 Kohlenstoffatome, bevorzugt 2 bis 6 Kohlenstoffatome und besonders bevorzugt 3 bis 6 Kohlenstoffatome enthalten; wobei die Alkenylgruppen gegebenenfalls und unabhängig voneinander substituiert sein können mit ein, zwei, drei oder mehr der Folgenden: Halogen, Alkyl, Aryl, Alkoxy, Amino, Alkylamino, Cyano, -CF₃, Dialkylamino, Hydroxy, Oxy, Phenoxy, -OCF₃, Heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ oder -COOR¹⁰;
Alkinyl- repräsentiert lineare und verzweigte Kohlenstoffketten mit wenigstens einer Kohlenstoff-Kohlenstoff-Dreifachbindung, die 2 bis 12 Kohlenstoffatome, bevorzugt 2 bis 6 Kohlenstoffatome enthalten; wobei die Alkinylgruppe gegebenenfalls und unabhängig substituiert sein kann mit ein, zwei, drei oder mehr der Folgenden: Halogen, Alkyl, Aryl, Alkoxy, Amino, Alkylamino, Cyano, -CF₃, Dialkylamino, Hydroxy, Oxy, Phenoxy, -OCF₃, Heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ oder -COOR¹⁰;
Aryl (inklusive des Arylanteils von Aralkyl) - repräsentiert eine carbocyclische Gruppe, die 6 bis 15 Kohlenstoffatome und wenigstens einen aromatischen Ring enthält (z.B. ist Phenyl ein Aryl), dabei kann die Arylgruppe gegebenenfalls mit Aryl-, Cycloalkyl-, Heteroaryl- oder Heterocycloalkylringen kondensiert sein; und wobei jedes der verfügbaren substituierbaren Kohlenstoff- und Stickstoffatome in der Arylgruppe und/oder den kondensierten Ring(en) gegebenenfalls und unabhängig substituiert sein kann mit ein, zwei, drei oder mehr der Folgenden: Halogen, Alkyl, Aryl, Alkoxy, Amino, Alkylamino, Cyano, -CF₃, Dialkylamino, Hydroxy, Oxy, Phenoxy, -OCF₃, Heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ oder -COOR¹⁰;
Aralkyl - repräsentiert eine Alkylgruppe, wie oben definiert, in der ein oder mehrere Wasserstoffatome der Alkyleinheit durch eine oder mehrere Arylgruppen substituiert sind; wobei die Aralkylgruppe gegenenfalls und unabhängig substituiert sein kann mit ein, zwei, drei oder mehr der Folgenden: Halogen, Alkyl, Aryl, Alkoxy, Amino, Alkylamino, Cyano, -CF₃, Dialkylamino, Hydroxy, Oxy, Phenoxy, -OCF₃, Heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ oder -COOR¹⁰; repräsentative Aralkylgruppen beinhalten Benzyl und Diphenylmethyl;
Cycloalkyl - repräsentiert gesättigte verzweigte oder unverzweigte carbocyclische Ringe mit 3 bis 20 Kohlenstoffatomen, bevorzugt 3 bis 7 Kohlenstoffatomen; wobei die Cycloalkylgruppe gegebenenfalls und unabhängig substituiert sein kann mit einer, zwei, drei oder mehr der Folgenden: Halogen, Alkyl, Aryl, Alkoxy, Amino, Alkylamino, Cyano, -CF₃, Dialkylamino, Hydroxy, Oxy, Phenoxy, -OCF₃, Heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ oder -COOR¹⁰;
Cycloalkylalkyl - repräsentiert eine Alkylgruppe, wie oben definiert, in der ein oder mehrere Wasserstoffatome der Alkyleinheit durch eine oder mehrere Cycloalkylgruppen substituiert sind; wobei die Cycloalkylalkylgruppe gegebenenfalls und unabhängig substituiert sein kann mit ein, zwei, drei oder mehr der Folgenden: Halogen, Alkyl, Aryl, Alkoxy, Amino, Alkylamino, Cyano, -CF₃, Dialkylamino, Hydroxy, Oxy, Phenoxy, -OCF₃, Heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ oder -COOR¹⁰ ;
Halogen - repräsentiert Fluor, Chlor, Brom und Iod;
Heteroalkyl - repräsentiert lineare und verzweigte Kohlenstoffketten, die 1 bis 20 Kohlenstoffatome enthalten, bevorzugt 1 bis 6 Kohlenstoffatome, und durch 1 bis 3 Heteroatome unterbrochen sind, die aus -O-, -S- und -N- ausgewählt sind; wobei jedes der verfügbaren substituierbaren Kohlenstoffund Stickstoffatome in der Heteroalkylkette gegebenenfalls und unabhängig substituiert sein kann mit ein, zwei, drei oder mehr der Folgenden: Halogen, C₁- bis C₆-Alkyl, Aryl, Cyano, Hydroxy, Alkoxy, Oxy, Phenoxy, -CF₃, -OCF₃, Amino, Alkylamino, Dialkylamino, Heterocycloalkyl, -SO₂NH₂, -NHSO₂R¹⁰, -SO₂NHR¹⁰, -SO₂R¹⁰, -SOR¹⁰, -SR¹⁰, oder -NHSO₂, -NO₂, -CONR¹⁰, -NCOR¹⁰ oder -COOR¹⁰;
Heteroaryl - repräsentiert cyclische Gruppen, die wenigstens ein Heteroatom ausgewählt aus O, S und N aufweisen, und in denen das Heteroatom/die Heteroatome eine carbocyclische Ringstruktur unterbrechen und eine ausreichende Anzahl von delokalisierten Pi-Elektronen haben, um einen aromatischen Charakter zu ergeben, wobei die aromatischen heterocyclischen Gruppen 2 bis 14 Kohlenstoffatome enthalten, die Heteroarylgruppen mit einem oder mehreren Aryl-, Cycloalkyl-, Heteroaryl- oder Heterocycloalkylringen kondensiert sein können; und wobei jedes der verfügbaren substituierbaren Kohlenstoff- oder Stickstoffatome in der Heteroarylgruppe und/oder dem/n kondensierten Ring/kondensierten Ringen gegebenenfalls und unabhängig substituiert sein kann mit ein, zwei, drei oder mehr der Folgenden: Halogen, C₁- bis C₆-Alkyl, Aryl, Cyano, Hydroxy, Alkoxy, Oxy, Phenoxy, -CF₃, -OCF₃, Amino, Alkylamino, Dialkylamino, Heterocycloalkyl, -SO₂NH₂, -NHSO₂R¹⁰, -SO₂NHR¹⁰, -SO₂R¹⁰, -SOR¹⁰, -SR¹⁰, oder -NHSO₂, -NO₂, -CONR¹⁰, -NCOR¹⁰ oder -COOR¹⁰;
repräsentative Arylgruppen können z.B. Furanyl, Imidazoyl, Pyrimidinyl, Triazolyl, 2-, 3- oder 4-Pyridyl oder 2-, 3-oder 4-Pyridyl-N-oxid sein, wobei Pyridyl-N-oxid als 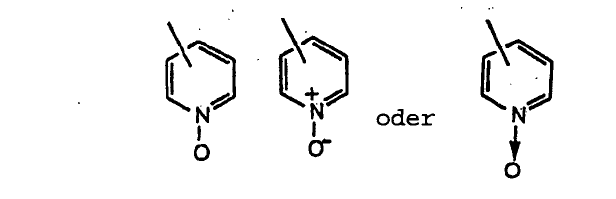 repräsentiert werden kann,
Heteroarylalkyl - repräsentiert eine Alkylgruppe, wie oben definiert, in der ein oder mehrere Wasserstoffatome durch eine oder mehrere Heteroarylgruppen ersetzt wurden; wobei die Heteroarylalkylgruppe gegebenenfalls und unabhängig substituiert sein kann mit ein, zwei, drei oder mehr der Folgenden: Halogen, Alkyl, Aryl, Alkoxy, Amino, Alkylamino, Cyano, -CF₃, Dialkylamino, Hydroxy, Oxy, Phenoxy, -OCF₃, Heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ oder -COOR¹⁰; wie durch 2-, 3- oder 4-Pyridylmethyl oder 2-, 3- oder 4-Pyridylmethyl-N-oxid beispielhaft dargestellt wird;
Heterocycloalkyl - repräsentiert einen gesättigten, verzweigten oder unverzweigten carbocyclischen Ring, der 3 bis 15 Kohlenstoffatome, bevorzugt 4 bis 6 Kohlenstoffatome enthält, dieser carbocyclische Ring durch 1 bis 3 Heteroatome unterbrochen ist, die aus -O-, -S- und -N- ausgewählt sind, wobei der Ring optional eine oder zwei ungesättigte Bindungen enthalten kann, die dem Ring nicht aromatischen Charakter verleihen; und wobei sämtliche verfügbaren substituierbaren Kohlenstoff- und Stickstoffatome im Ring gegebenenfalls und unabhängig substituiert sein können mit ein, zwei, drei oder mehr der Folgenden: Halogen, Alkyl, Aryl, Alkoxy, Amino, Alkylamino, Cyano, -CF₃, Dialkylamino, Hydroxy, Oxy, Phenoxy, -OCF₃, Heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ oder -COOR¹⁰;
repräsentative Heterocycloalkylgruppen können 2- oder 3-Tetrahydrofuranyl, 2- oder 3-Tetrahydrothienyl, 1-, 2-, 3-oder 4-Piperidinyl, 2- oder 3-Pyrrolidinyl, 1-, 2- oder 3-Piperizinyl, 2- oder 4-Dioxanyl, Morpholinyl, 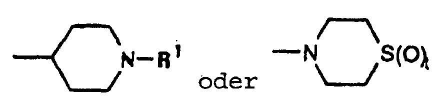 imfassen, dabei ist R¹ wie oben definiert und t ist 0, 1 oder 2;
Heterocycloalkylalkyl - repräsentiert eine Alkylgruppe, wie oben definiert, bei der ein oder mehrere Wasserstoffatome durch ein oder mehrere Heterocycloalkylgruppen ersetzt wurden; wobei der Ring ein oder zwei ungesättigte Bindungen, die dem Ring nicht aromatischen Charakter verleihen, enthalten kann; und wobei die Heterocycloalkylalkylgruppe gegebenenfalls und unabhängig substituiert sein kann mit ein, zwei, drei oder mehr der Folgenden: Halogen, Alkyl, Aryl, Alkoxy, Amino, Alkylamino, Cyano, -CF₃, Dialkylamino, Hydroxy, Oxy, Pherioxy, -OCF₃, Heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ oder -COOR¹⁰.

2. Verbindung nach Anspruch 1, bei der sich eine Einfachbindung an Kohlenstoffatom 11 befindet; X Kohlenstoff ist; die Positionen 3, 8 und 10 am Ring substituiert sind, bevorzugt mit Halogen; und Z -NHR⁴⁰ ist, wobei bevorzugt R⁴⁰ Heteroarylalkyl, besonders bevorzugt 3- oder 4-Methylpyridyl-N-oxid ist.

3. Verbindung nach Anspruch 1, bei der a N ist, und R¹ H ist; R², R³ und R⁴ Halogen sind; X CH ist; und R⁵, R⁶, R⁷ und R⁸ Wasserstoff sind.

4. Verbindung nach Anspruch 3, bei der Z -NR⁴⁰R⁴² ist.

5. Verbindung nach Anspruch 4, bei der R⁴⁰ H ist und R⁴² Heteroarylalkyl ist.

6. Verbindung nach Anspruch 5, bei der R⁴² 2-, 3- oder 4-Pyridylmethyl-N-oxid ist.

7. Verbindung nach Anspruch 1, ausgewählt aus einem der Beispiele 1 bis 83.

8. Verbindung nach Anspruch 1, mit der Struktur:

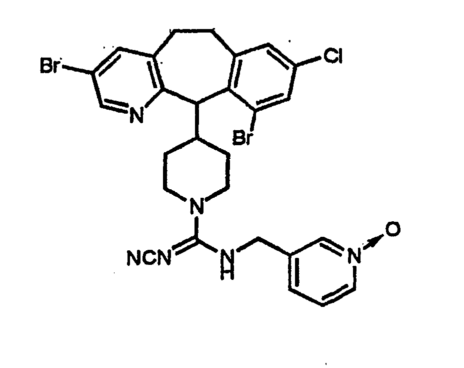

9. Pharmazeutische Zusammensetzung zur Inhibierung des abnormalen Wachstums von Zellen, die eine wirksame Menge einer Verbindung gemäß einem der Ansprüche 1 bis 8 in Kombination mit einem pharmazeutisch akzeptablen Träger enthält.

10. Verwendung einer Verbindung gemäß einem der Ansprüche 1 bis 8 zur Herstellung eines Medikaments zur Inhibierung des abnormalen Wachstums von Zellen.

11. Verwendung nach Anspruch 10, bei der die inhibierten Zellen Tumorzellen sind, die ein aktiviertes ras-Onkogen exprimieren.

12. Verwendung nach Anspruch 11, bei der die inhibierten Zellen pankreatische Tumorzellen, Lungenkrebszellen, myeloide Leukämietumorzellen, thyroide Follikulartumorzellen, myelodysplastische Tumorzellen, epidermale Karzinomtumorzellen, Blasenkarzinomtumorzellen oder kolonale Tumorzellen sind.

13. Verwendung nach Anspruch 10, bei der die Inhibierung des abnormalen Wachstums von Zellen durch die Inhibierung von ras-Farnesyl-Proteintransferase erfolgt.

14. Verwendung nach Anspruch 10, bei der die Inhibierung von Tumorzellen erfolgt, deren ras-Protein als Ergebnis einer onkogenen Mutation in anderen Genen als dem ras-Gen aktiviert ist.

## Revendications

1. Composé de formule : 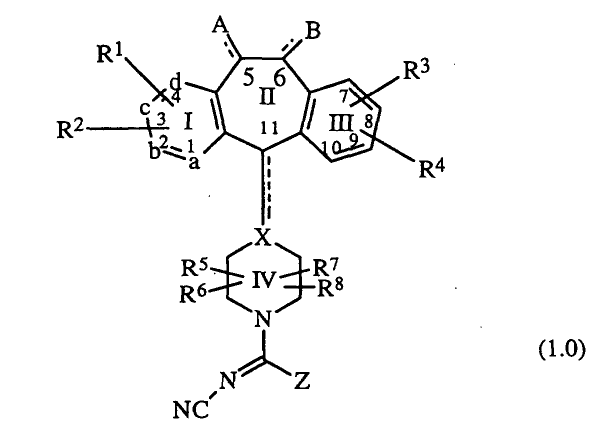 ou sel ou composé d'addition de solvant d'un tel composé, admissible en pharmacie, dans laquelle formule
l'un des symboles a, b, c et d représente N ou NR⁹ où R⁹ représente O⁻, -CH₃ ou -(CH₂)ₙCO₂H où n vaut de 1 à 3, et les symboles a, b, c et d restants représentent CR¹ ou CR², ou bien chacun des symboles a, b, c et d représente indépendamment CR¹ ou CR² ;
chacun des symboles R¹ et R² représente indépendamment un atome d'hydrogène ou d'halogène ou un groupe -CF₃, -OR¹⁰ (par exemple -OCH₃), -COR¹⁰, -SR¹⁰ (par exemple -SCH₃ et -SCH₂C₆H₅), -S(O)ₜR¹¹ où t vaut 0, 1 ou 2 (par exemple -SOCH₃ et -SO₂CH₃), -SCN, -N(R¹⁰)₂, -NR¹⁰R¹¹, -NO₂, -OC(O)R¹⁰, -CO₂R¹⁰, -OCO₂R¹¹, -CN, -NHC(O)R¹⁰, -NHSO₂R¹⁰, -CONHR¹⁰, -CONHCH₂CH₂OH, -NR¹⁰COOR¹¹, -SR¹¹C(O)OR¹¹ (par exemple -SCH₂CO₂-CH₃), -SR¹¹N(R⁷⁵)₂ où chaque symbole R⁷⁵ représente indépendamment un atome d'hydrogène ou un groupe -C(O)OR¹¹ (par exemple -S(CH₂)₂NH-C(O)O-t-butyle et -S(CH₂)₂NH₂), benzotriazol-1-yloxy, tétrazol-5-ylthio ou tétrazol-5-ylthio substitué (par exemple tétrazol-5-ylthio portant un substituant alkyle, comme 1-méthyl-tétrazol-5-ylthio), alcynyle, alcényle ou alkyle, ledit groupe alkyle ou alcényle pouvant éventuellement porter un substituant halogéno, -OR¹⁰ ou -CO₂R¹⁰ ;
R³ et R⁴ sont identiques ou différents et représentent chacun, indépendamment, un atome d'hydrogène ou l'un des substituants représentés par R¹ ou R², ou bien R³ et R⁴ représentent ensemble un cycle en C₅₋₇, saturé ou insaturé, condensé avec le cycle benzénique III ;
R⁵, R⁶, R⁷ et R⁸ représentent chacun, indépendamment, un atome d'hydrogène ou un groupe -CF₃, -COR¹⁰, alkyle ou aryle, ce groupe alkyle ou aryle portant éventuellement un ou des substituants -OR¹⁰, -SR¹⁰, -S(O)ₜR¹¹, -NR¹⁰COOR¹¹, -N(R¹⁰)₂, -NO_{2,} -COR¹⁰, -OCOR¹⁰, -CO₂R¹⁰, -OCO₂R¹¹ ou -OPO₃R¹⁰, ou bien l'un des symboles R⁵, R⁶, R⁷ et R⁸ représente, conjointement avec R⁴⁰ dont il est question plus loin, -(CH₂)ᵣ- où r vaut de 1 à 4 et qui peut porter un ou des substituants alkyle inférieur, alcoxy inférieur, -CF₃ ou aryle, ou bien encore R⁵ et R⁶ représentent conjointement =O ou =S et/ou R⁷ et R⁸ représentent conjointement =O ou =S ;
R¹⁰ et R¹² représentent chacun, indépendamment, un atome d'hydrogène ou un groupe alkyle, cycloalkyle, cycloalkyl-alkyle, hétéroaryle, aryle, aralkyle ou -NR⁴⁰R⁴² où R⁴⁰ et R⁴² représentent chacun, indépendamment, un atome d'hydrogène ou un groupe aryle, alkyle, aralkyle, hétéroaryle, hétéroaryl-alkyle, hétérocycloalkyle, hétérocycloalkyl-alkyle, hétéroalkyle, cycloalkyle, cycloalkyl-alkyle, alcényle ou alcynyle ;
R¹¹ représente un groupe alkyle ou aryle ;
X représente N, CH ou C, de sorte que, si X représente N ou CH, la liaison vers l'atome de carbone 11 est une liaison simple, représentée par le trait continu, et si X représente C, la liaison vers l'atome de carbone 11 est une liaison double, représentée par le trait continu et le trait en pointillés ;
le trait en pointillés entre les atomes de carbone 5 et 6 représente une éventuelle double liaison, de sorte que, s'il y a une double liaison, A et B représentent chacun, indépendamment, un atome d'halogène ou un groupe -NO₂,
-R¹⁰, -OR¹¹, -OCO₂R¹¹ ou -OC(O)R¹⁰, et s'il n'y a pas de double liaison entre les atomes de carbone 5 et 6, A et B représentent chacun, indépendamment, une paire d'atomes d'hydrogène H₂, une paire de groupes (-OR¹¹)₂, un atome d'hydrogène et un atome d'halogène, une paire d'atomes d'halogène, un groupe alkyle et un atome d'hydrogène, une paire de groupes alkyle, un atome d'hydrogène et un groupe -OC(O)R¹⁰, un atome d'hydrogène et un groupe -OR¹⁰, un substituant oxo, un atome d'hydrogène et un groupe aryle, un groupe =NOR¹⁰, ou un groupe -O-(CH₂)ₚ-O- où p vaut 2, 3 ou 4 ;
et Z représente un groupe alkyle, aryle, aralkyle, hétéroalkyle, hétéroaryle, hétéroaryl-alkyle, hétérocycloalkyle, hétérocycloalkyl-alkyle, -OR⁴⁰, -SR⁴⁰, -CR⁴⁰R⁴² ou -NR⁴⁰R⁴², où R⁴⁰ et R⁴² ont les significations indiquées plus haut ;
et les définitions suivantes s'appliquant, sauf indication contraire :
le groupe alkyle (y compris les fragments alkyle des groupes alcoxy, alkylamino et dialkylamino) désigne les chaînes d'atomes de carbone linéaires ou ramifiées et comportant de 1 à 20 atomes de carbone et de préférence de 1 à 6 atomes de carbone (par exemple méthyle, éthyle, propyle, isopropyle, n-butyle, t-butyle, n-pentyle, isopentyle, hexyle, etc.), ledit groupe alkyle pouvant éventuellement et indépendamment porter 1, 2, 3 substituants ou plus parmi les suivants : halogéno, alkyle, aryle, alcoxy, amino, alkylamino, cyano, -CF₃, dialkylamino, hydroxy, oxo (=O), phénoxy, -OCF₃, hétérocycloalkyle, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ et -COOR¹⁰ ;
le groupe alcoxy désigne un fragment alkyle comportant de 1 à 20 atomes de carbone et lié par liaison covalente à un élément structural adjacent par l'intermédiaire d'un atome d'oxygène, comme les groupes méthoxy, éthoxy, propoxy, butoxy, pentoxy, hexoxy, etc., ledit groupe alcoxy pouvant éventuellement et indépendamment porter 1, 2, 3 substituants ou plus parmi les suivants : halogéno, alkyle, aryle, alcoxy, amino, alkylamino, cyano, -CF₃, dialkylamino, hydroxy, oxo, phénoxy, -OCF₃, hétérocycloalkyle, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ et -COOR¹⁰ ;
le groupe alcényle désigne les chaînes d'atomes de carbone, linéaires ou ramifiées, contenant au moins une double liaison carbone-carbone et comportant de 2 à 12 atomes de carbone, de préférence de 2 à 6 atomes de carbone et mieux encore de 3 à 6 atomes de carbone, ledit groupe alcényle pouvant éventuellement et indépendamment porter 1, 2, 3 substituants ou plus parmi les suivants : halogéno, alkyle, aryle, alcoxy, amino, alkylamino, cyano, -CF₃, dialkylamino, hydroxy, oxo, phénoxy, -OCF₃, hétérocycloalkyle, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ et -COOR¹⁰ ;
le groupe alcynyle désigne les chaînes d'atomes de carbone, linéaires ou ramifiées, contenant au moins une triple liaison carbone-carbone et comportant de 2 à 12 atomes de carbone, et de préférence de 2 à 6 atomes de carbone, ledit groupe alcynyle pouvant éventuellement et indépendamment porter 1, 2, 3 substituants ou plus parmi les suivants : halogéno, alkyle, aryle, alcoxy, amino, alkylamino, cyano, -CF₃, dialkylamino, hydroxy, oxo, phénoxy, -OCF₃, hétérocycloalkyle, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ et -COOR¹⁰ ;
le groupe aryle (y compris le fragment aryle d'un groupe aralkyle) désigne un groupe carbocyclique comportant de 6 à 15 atomes de carbone et au moins un cycle aromatique (le groupe aryle étant par exemple un groupe phényle), ledit groupe aryle pouvant éventuellement être condensé avec un ou des cycles aryle, cycloalkyle, hétéroaryle ou hétérocycloalkyle, et n'importe lesquels des atomes de carbone et d'azote en état de porter un substituant, dans ledit groupe aryle et/ou ledit ou lesdits cycles condensés, pouvant éventuellement et indépendamment porter 1, 2, 3 substituants ou plus parmi les suivants : halogéno, alkyle, aryle, alcoxy, amino, alkylamino, cyano, -CF₃, dialkylamino, hydroxy, oxo, phénoxy, -OCF₃, hétérocycloalkyle, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ et -COOR¹⁰ ;
le groupe aralkyle désigne un groupe alkyle, tel que défini plus haut, dont un ou plusieurs atomes d'hydrogène ont été remplacés par un ou plusieurs groupes aryle, ledit groupe aralkyle pouvant éventuellement et indépendamment porter 1, 2, 3 substituants ou plus parmi les suivants : halogéno, alkyle, aryle, alcoxy, amino, alkylamino, cyano, -CF₃, dialkylamino, hydroxy, oxo, phénoxy, -OCF₃, hétérocycloalkyle, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ et -COOR¹⁰ (les groupes benzyle et diphénylméthyle sont des exemples représentatifs des groupes aralkyle) ;
le groupe cycloalkyle désigne un groupe carbocyclique saturé, ramifié ou non ramifié et comportant de 3 à 20 atomes de carbone et de préférence de 3 à 7 atomes de carbone, ledit groupe cycloalkyle pouvant éventuellement et indépendamment porter 1, 2, 3 substituants ou plus parmi les suivants : halogéno, alkyle, aryle, alcoxy, amino, alkylamino, cyano, -CF₃, dialkylamino, hydroxy, oxo, phénoxy, -OCF₃, hétérocycloalkyle, -SO₂NH₂, -NHSO₂R¹⁰, -SO₂NHR¹⁰, -SO₂R¹⁰, -SOR¹⁰, -SR¹⁰, -NRSO₂, -NO₂, -CONR¹⁰, -NCOR¹⁰ et -COOR¹⁰ ;
le groupe cycloalkyl-alkyle désigne un groupe alkyle, tel que défini plus haut, dont un ou plusieurs des atomes d'hydrogène ont été remplacés par un ou plusieurs groupes cycloalkyle, ledit groupe cycloalkyl-alkyle pouvant éventuellement et indépendamment porter 1, 2, 3 substituants ou plus parmi les suivants : halogéno, alkyle, aryle, alcoxy, amino, alkylamino, cyano, -CF₃, dialkylamino, hydroxy, oxo, phénoxy, -OCF₃, hétérocycloalkyle, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ et -COOR¹⁰;
halogéno désigne fluoro, chloro, bromo ou iodo ;
le groupe hétéroalkyle désigne une chaîne d'atomes de carbone, linéaire ou ramifiée, comportant de 1 à 20 atomes de carbone et de préférence de 1 à 6 atomes de carbone, interrompue par 1 à 3 hétéroatomes choisis parmi -O-, -S- et -N-, n'importe lequel des atomes de carbone et d'azote en état de porter un substituant, dans cette chaîne hétéroalkyle, pouvant éventuellement et indépendamment porter 1, 2, 3 substituants ou plus parmi les suivants : halogéno, alkyle en C₁₋₆, aryle, cyano, hydroxy, alcoxy, oxo, phénoxy, -CF₃, -OCF₃, amino, alkylamino, dialkylamino, hétérocycloalkyle, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ et -COOR¹⁰ ;
le groupe hétéroaryle désigne des groupes cycliques comportant au moins un hétéroatome choisi parmi O, S et N, ce ou ces hétéroatomes s'insérant dans une structure carbocyclique et apportant des électrons π délocalisés en nombre suffisant pour conférer le caractère aromatique, et les groupes hétérocycliques aromatiques comportant de 2 à 14 atomes de carbone, ledit groupe hétéroaryle pouvant éventuellement être condensé avec un ou des cycles aryle, cycloalkyle, hétéroaryle ou hétérocycloalkyle, et n'importe lesquels des atomes de carbone et d'azote en état de porter un substituant, dans ledit groupe hétéroaryle et/ou ledit ou lesdits cycles condensés, pouvant éventuellement et indépendamment porter 1, 2, 3 substituants ou plus parmi les suivants : : halogéno, alkyle en C₁₋₆, aryle, cyano, hydroxy, alcoxy, oxo, phénoxy, -CF₃, -OCF₃, amino, alkylamino, dialkylamino, hétérocycloalkyle, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ et -COOR¹⁰ (parmi les groupes hétéroaryle représentatifs, il y a par exemple les groupes furanyle, imidazolyle, pyrimidinyle, triazolyle, 2-, 3- et 4-pyridyle, et 2-, 3- et 4-pyridyle-N-oxyde, ces groupes pyridyle-N-oxyde pouvant être représentés ainsi : 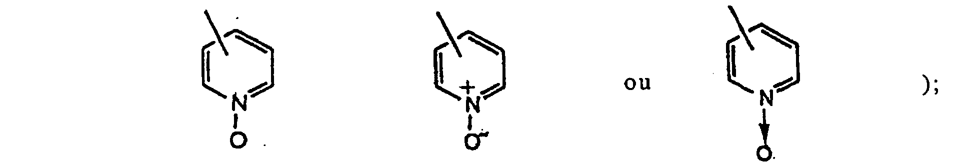
le groupe hétéroaryl-alkyle désigne un groupe alkyle, tel que défini plus haut, dont un ou plusieurs des atomes d'hydrogène ont été remplacés par un ou plusieurs groupes hétéroaryle, ledit groupe hétéroaryl-alkyle pouvant éventuellement et indépendamment porter 1, 2, 3 substituants ou plus parmi les suivants : halogéno, alkyle, aryle, alcoxy, amino, alkylamino, cyano, -CF₃, dialkylamino, hydroxy, oxo, phénoxy, -OCF₃, hétérocycloalkyle, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ et -COOR¹⁰ (des exemples en sont les groupes (2-, 3-ou 4-pyridyl)méthyle et (2-, 3- ou 4-pyridyle-N-oxyde)méthyle ;
le groupe hétérocycloalkyle désigne un carbocycle saturé, ramifié ou non ramifié, comportant de 3 à 15 atomes de carbone, de préférence de 4 à 6 atomes de carbone, et 1 à 3 hétéroatomes, choisis parmi -O-, -S- et -N-, insérés dans le carbocycle, et ce cycle pouvant éventuellement comporter une ou deux liaisons insaturées qui ne lui confèrent pas un caractère aromatique, et n'importe lesquels des atomes de carbone et d'azote du cycle en état de porter un substituant pouvant éventuellement et indépendamment porter 1, 2, 3 substituants ou plus parmi les suivants : halogéno, alkyle, aryle, alcoxy, amino, alkylamino, cyano, -CF₃, dialkylamino, hydroxy, oxo, phénoxy, -OCF₃, hétérocycloalkyle, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ et -COOR¹⁰ (parmi les groupes hétérocycloalkyle représentatifs, il y a les groupes 2- et 3-tétrahydrofuranyle, 2- et 3-tétrahydrothiényle, 1-, 2-, 3- et 4-pipéridinyle, 2- et 3-pyrrolidinyle, 1-, 2- et 3-pipérazinyle, 2- et 4-dioxanyle, morpholinyle, 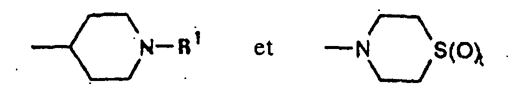 , où R¹ a la signification indiquée plus haut, et t vaut 0, 1 ou 2 ;
et le groupe hétérocycloalkyl-alkyle désigne un groupe alkyle, tel que défini plus haut, dont un ou plusieurs atomes d'hydrogène ont été remplacés par un ou plusieurs groupes hétérocycloalkyle, ce cycle pouvant éventuellement comporter une ou deux liaisons insaturées qui ne lui confèrent pas un caractère aromatique, et ledit groupe hétérocycloalkyl-alkyle pouvant éventuellement et indépendamment porter 1, 2, 3 substituants ou plus parmi les suivants : halogéno, alkyle, aryle, alcoxy, amino, alkylamino, cyano, -CF₃, dialkylamino, hydroxy, oxo, phénoxy, -OCF₃, hétérocycloalkyle, -SO₂NH₂, -NHSO₂R¹⁰, -SO₂NHR¹⁰, -SO₂R¹⁰, -SOR¹⁰, -SR¹⁰, -NHSO₂, -NO₂, -CONR₁₀, -NCOR¹⁰ et -COOR¹⁰.

2. Composé conforme à la revendication 1, dans lequel il y a une liaison simple au niveau de l'atome de carbone 11, X représente un atome de carbone, il y a des substituants, de préférence des atomes d'halogène, en les positions 3, 8 et 10 des cycles, et Z représente -NHR⁴⁰ où R⁴⁰ représente de préférence un groupe hétéroaryl-alkyle, et mieux encore un groupe (3- ou 4-pyridyl-N-oxyde)-méthyle.

3. Composé conforme à la revendication 1, dans lequel a représente N, R¹ représente un atome d'hydrogène, R², R³ et R⁴ représentent des atomes d'halogène, X représente CH, et R⁵, R⁶, R⁷ et R⁸ représentent des atomes d'hydrogène.

4. Composé conforme à la revendication 3, dans lequel Z représente -NR⁴⁰R⁴².

5. Composé conforme à la revendication 4, dans lequel R⁴⁰ représente un atome d'hydrogène et R⁴² représente un groupe hétéroaryl-alkyle.

6. Composé conforme à la revendication 5, dans lequel R⁴² représente un groupe (2-, 3- ou 4-pyridyl-N-oxyde)-méthyle.

7. Composé conforme à la revendication 1, choisi parmi tous les composés des exemples 1 à 83.

8. Composé conforme à la revendication 1, de structure :

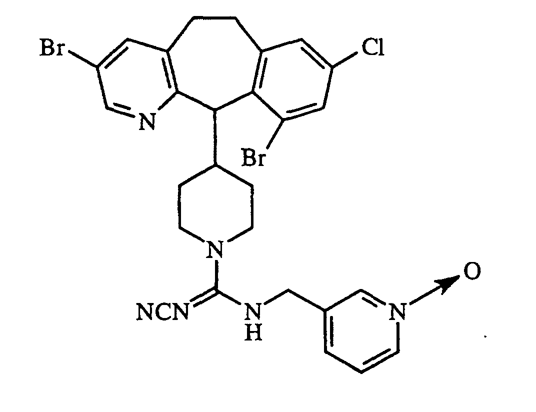

9. Composition pharmaceutique destinée à inhiber la prolifération anormale de cellules, qui contient, en une quantité efficace, un composé conforme à l'une des revendications 1 à 8, combiné avec un véhicule admissible en pharmacie.

10. Emploi d'un composé conforme à l'une des revendications 1 à 8 pour la fabrication d'un médicament destiné à inhiber la prolifération anormale de cellules.

11. Emploi conforme à la revendication 10, les cellules inhibées étant des cellules tumorales qui expriment un oncogène *ras* activé.

12. Emploi conforme à la revendication 11, les cellules inhibées étant des cellules de tumeur du pancréas, des cellules de cancer du poumon, des cellules tumorales de leucémie myéloïde, des cellules de tumeur des vésicules thyroïdiennes, des cellules de tumeur myélodysplasique, des cellules tumorales d'épithéliome épidermoïde, des cellules tumorales de cancer de la vessie ou des cellules de tumeur du côlon.

13. Emploi conforme à la revendication 10, l'inhibition de la prolifération anormale de cellules intervenant grâce à l'inhibition de la farnésyl-protéine Ras transférase.

14. Emploi conforme à la revendication 10, l'inhibition concernant des cellules tumorales dans lesquelles la protéine Ras est activée en résultat d'une mutation tumorigène dans des gènes autres que le gène *ras.*
